(19) Europäisches Patentamt
European Patent Office
Office européen des brevets

(11) Publication number: **0 327 365 B1**

(12) # EUROPEAN PATENT SPECIFICATION

(45) Date of publication of patent specification: **15.06.94**

(51) Int. Cl.[5]: **C07C 335/04**, A61K 31/17, A61K 49/02, A61K 43/00

(21) Application number: **89301012.4**

(22) Date of filing: **02.02.89**

(54) **Improvements in or relating to modified haptens useful as imaging and therapeutic agents.**

(30) Priority: **03.02.88 US 151855**

(43) Date of publication of application:
**09.08.89 Bulletin 89/32**

(45) Publication of the grant of the patent:
**15.06.94 Bulletin 94/24**

(84) Designated Contracting States:
**AT BE CH DE ES FR GB GR IT LI LU NL SE**

(56) References cited:
**EP-A- 0 217 577**
**EP-A- 0 279 307**
**US-A- 4 339 426**
**US-A- 4 622 420**
**US-A- 4 678 667**

(73) Proprietor: **HYBRITECH INCORPORATED**
**11095 Torreyana Road**
**San Diego California 92126(US)**

(72) Inventor: **Anderson, Leslie DeRiemer**
**634 Hollyridge Drive**
**Encinitas California 92024(US)**
Inventor: **Frincke, James Martin**
**2146 Woodwind Drive**
**Olivenhain California 92024(US)**
Inventor: **Meyer, Damon Lawrence**
**7957 Calle Cozumel**
**Carlsbad California 92009(US)**
Inventor: **Sullivan, Brian William**
**1216 Huntington Road**
**San Marcos California 92069(US)**
Inventor: **Battersby, Thomas Robert**
**2409 Newcastle Avenue**
**Cardiff California 92007(US)**
Inventor: **Kusnierz, Edward**
**9710 Mesa Springs Way, Nr. 5**
**San Diego California 92126(US)**

(74) Representative: **Hudson, Christopher Mark et al**
**Erl Wood Manor**
**Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 327 365 B1

## Description

Monoclonal antibodies are becoming increasingly important for in vivo use. Of substantial interest are their potential applications for the imaging and treatment of disease, particularly cancer. Monoclonal antibodies which are labeled, for example, with a chelate complex of a metal ion and a chelating agent are particularly useful for the imaging and treatment of certain disease states. Generally, such monoclonal antibodies are labeled by chemical conjugation with the hapten or, in the case of anti-hapten antibodies, by their ability to specifically bind with the metal chelate to form non-covalent hapten-antibody complexes.

Recent studies relating to the use of monoclonal antibodies for tumor imaging and therapy have shown that bifunctional antibodies having specificity for a hapten and, for example, a tumor target, provide a mechanism for delivery of the hapten to the tumor target. Delivery of a hapten to a disease site with a bifunctional antibody system involves time dependent binding and release of the hapten from the antibody. Consequently, the usefulness of a particular hapten as an imaging or therapeutic agent is dependent upon the interaction of the hapten with the antibody utilized as well as with serum components, e.g., proteins, lipids, and other tissue compartments. For example, the inherent pharmacokinetic properties of a particular hapten and the affinity of an antibody for the hapten may result in the hapten binding so tightly to the anti-hapten antibody so as to be indistinguishable from covalently bound antibody conjugates, or in the hapten binding so loosely to the anti-hapten antibody so as to be incapable of localization with the antibody at the disease site. Additionally, the inherent pharmacokinetic properties of a particular hapten may result in undesirable serum and tissue interactions such that the accumulation of the hapten in the liver, kidneys or intestines reaches intolerable levels. In such cases, the biodistribution and localization of the hapten substantially limits the potential clinical value of the bifunctional antibody delivery system.

Accordingly, there exists a need to modify pharmacokinetic properties of various haptens for in vivo imaging and therapy. Further, there exists a need for novel hapten derivatives that impart desired properties to antibody delivery systems for particular applications. The present invention meets these needs by providing novel hapten derivatives with modified pharmacokinetics, as well as methods for modifying the pharmacokinetics of a given hapten.

Surprisingly, haptens which are useful as in vivo imaging and therapeutic agents can be derivatized to modify the inherent pharmacokinetic properties of the hapten. Accordingly, besides providing novel hapten derivatives, the present invention provides a method for modifying the pharmacokinetics of a hapten as a radioimaging or radiotherapeutic agent which comprises selecting a suitable hapten and an antibody capable of complexing with the hapten, and derivatizing the hapten to modify the dissociation rate of the hapten-antibody complex and the interaction of the hapten with serum components and tissue compartments, thereby increasing or decreasing serum half-life and radiation doses to tissues. A predetermined effective dosage of the derivatized hapten and the antibody are thereafter administered to a patient for purposes of imaging or therapy.

European Patent Number 0 217 577 discloses certain aminobenzyl EDTA hapten derivatives.

Haptens which can be derivatized, in accordance with the present invention, are generally selected from complexes of a chelating agent and a metal ion. Preferably, the hapten selected for use in the invention is a metal ion complex of ethylenediaminetetracetic acid ("EDTA"), diethylenetriaminepentaacetic acid ("DTPA"), 1,4,7,10-tetraazacyclododecane-N,N',N'',N'''-tetraacetic acid ("DOTA"), 1,5,9,13-tetraazacyclohexadecane-N,N',N'',N'''-tetraacetic acid ("HETA"), 1,4,7,10,-tetraazacyclotridecane-N,N',N'',N'''-tetraacetic acid ("TRITA") or 1,4,8,11-tetraazacyclotetradecane-N,N',N'',N'''-tetraacetic acid ("TETA") with the derivatized hapten being represented generally by Formula (I):

$p$-$R_1$-$C_6H_4$-$CH_2$-EDTA-M (I)

wherein

$R_1$ is

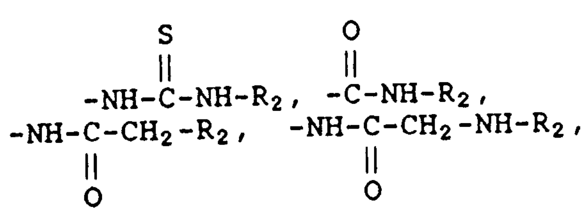

-NH-$R_2$,

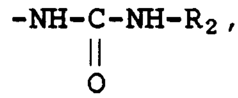

or

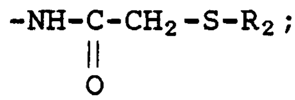

$R_2$ is hydrogen, an amino group, OC{Y), -p-phenyl-$CH_2$-Y, an unsubstituted $C_1$ to $C_{30}$ branched or straight chain alkyl group; a substituted $C_1$ to $C_{30}$ branched or straight chain alkyl, cycloalkyl, aryl or arylalkyl group, in which the substituents are one or more of any of:

hydroxy, carboxy, =O, =S,

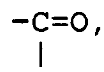

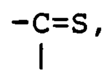

-S-, -$SR_4$, fluoro, chloro, bromo, iodo, amino, nitro, -$SO_3H$, -$NHR_3$, -$NHR_4$, -$N(R_3)_2$, -$CONHR_3$, -$COOR_3$, -$SO_4$, -$PO_4$, phenyl, benzyl, imidazolo, or a group of the formulae:

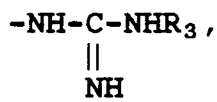 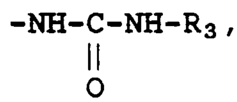 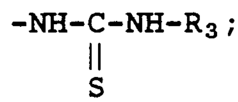

wherein

$R_3$ is hydrogen, a $C_1$ to $C_{30}$ straight or branched chain alkyl group, -p-phenyl-Y,

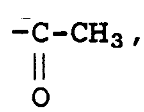

or a non-reactive functional group;

Y is EDTA, DTPA, DOTA, HETA, TRITA or TETA;

$R_4$ is H or

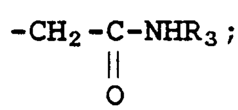

and, M is a metal ion.

In a preferred embodiment, compounds of Formula (Ia):

p-$R_1$-$C_6H_4CH_2$EDTA-M (Ia)

wherein $R_1$ is

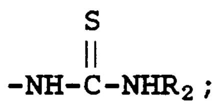 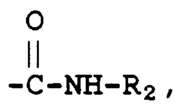

or

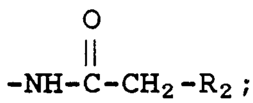

in which $R_2$ is hydrogen; $NH_2$; an unsubstituted $C_1$-$C_{30}$ branched or straight chain alkyl group; or a $C_1$-$C_{30}$ branched or straight chain alkyl, cycloalkyl, aryl or arylalkyl group substituted by one or more substituents independently selected from the group consisting of:

-OH; -COOH; =0; =S; -S-; -Cl; -F; -Br; -I; $-NH_2$; $-N0_2$; $-S0_3H$; $-NH-R_3$; $-CONHR_3$; $-COOR_3$; $-S0_4$; $-P0_4$; phenyl; and benzyl;

$R_3$ is H; an alkyl group; or a non-reactive functional group; and M is a metal ion, are useful as imaging or therapeutic agents.

Especially preferred compounds of the invention are those represented by Formula (Ib):

<u>p</u>-$R_1$-$C_6H_4$-$CH_2$-EDTA-M (Ib)

wherein

$R_1$ is

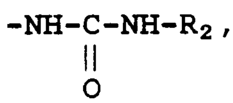 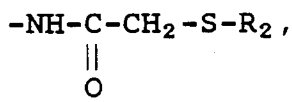 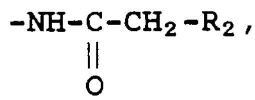

$-NH-R_2$, 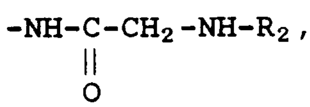 or 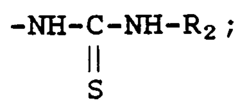

$R_2$ is -<u>p</u>-phenyl-$CH_2$-Y, -OC(Y), a substituted $C_1$ to $C_{30}$ branched or straight chain alkyl, cycloalkyl, aryl or arylalkyl group, in which the substituents are one or more of any of:

hydroxy,

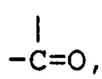 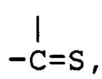

$-SR_4$, $-NHR_3$, $-NHR_4$, $-N(R_3)_2$, imidazolo, or a group of the formulae:

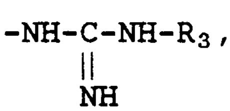 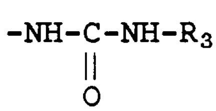 or 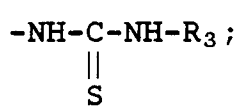

wherein

$R_3$ is -<u>p</u>-phenyl-$CH_2$-Y,

or

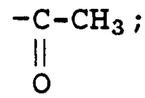

Y is EDTA, DTPA, DOTA, HETA, TRITA or TETA;
$R_4$ is H or

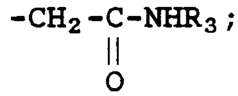

and, M is a metal ion.

Additionally, pharmaceutical formulations are provided which comprise a compound of Formula (I), Formula (Ia), or Formula (Ib) associated with one or more pharmaceutically-acceptable carriers, diluents or excipients therefor. The formulations may further comprise, separate from or in combination with a hapten of the invention, an antibody capable of complexing with the hapten.

Further, the present invention provides methods for in vivo imaging and therapy which comprises administering to a patient, sequentially, simultaneously, or in combination, a predetermined effective dosage of a hapten of the invention, and a suitable anti-hapten antibody.

The invention has been summarized in order that the detailed description that follows may be better understood and the contribution to the art may be better appreciated.

As indicated above, the present invention, in one aspect, provides a method for modifying the pharmacokinetics of a hapten as an in vivo radioimaging or radiotherapeutic agent. The method comprises the selection of a hapten suitable for imaging or therapy and an antibody capable of complexing with the hapten, and the derivatization of the hapten to modify the dissociation rate of the hapten-antibody complex and the interaction of the hapten with serum and tissue components, thereby increasing or decreasing serum half-life, as desired. The derivatized hapten and the antibody are administered to a patient in a predetermined effective dosage for imaging or therapy. Preferably, the biodistribution and localization of the derivatized hapten, and complexes of the derivatized hapten and the antibody, are enhanced depending upon the particular diagnostic or therapeutic purpose.

In the context of the present invention, the term "hapten" refers to a molecule capable of specific reactivity with an antibody, but incapable of stimulating an immune response by antibody production, except in combination with or as part of a carrier protein molecule. As used herein, the term "pharmacokinetics" refers to the change in the biodistribution of a molecule over a period of time. The terms "biodistribution" and "localization," as used herein, refer to the distribution of a molecule within individual organs of an animal at given time points, and the amount or concentration of a molecule present in an organ, respectively. Further, the term "localization" implies the presence, in excess of the concentration due to blood levels, of a molecule at a particular organ for a period of time as a result of the interaction between the molecule and tissue compartments.

For purposes of the present invention, the hapten selected for use in the invention will generally depend upon the chemical, biological and physiological properties of the hapten and the desired application of the invention. For example, the selection of a particular hapten will depend upon whether the hapten will be used as an imaging or therapeutic agent, and generally involves consideration of chemical properties, such as water solubility, biological properties such as the rate of clearance from particular tissues, and physiological properties, such as the capability of binding to a receptor at a disease site.

Haptens comprising chelate complexes of a metal ion and a chelating agent are generally selected for use in the invention. Preferred chelating agents of the invention are ethylenediaminetetracetic acid ("EDTA"), diethylenetriaminepentaacetic acid ("DTPA"), DOTA, HETA, TRITA, TETA, and analogs thereof. EDTA, DTPA, and methods for preparing analogs and the starting materials for the compounds of the present invention are described in U.S. Patent No. 4,622,420. The methods for preparing DOTA, HETA, TRITA, TETA, and starting materials useful in the present invention are described in detail in U.S. Patent No. 4,678,667 (Meares, et al.) and in Moi, et al., J. Am. Chem. Soc., 110, 6266 (1988). The EDTA and DTPA analogs disclosed in U.S. Pat. No. 4,622,420 are capable of forming physiologically stable chelates with a variety of metal ions. Likewise, the DOTA, HETA, TRITA, and TETA analogs described in U.S. Pat. No.

4,678,667 and Moi, et al. are capable of forming physiologically stable chelates with various metal ions.

As used in Formulae (I), (Ia), and (Ib), "M", when referring to a metal ion means a metal atom in ionic form without particular reference to possible valences. The particular valence of the ion, as one skilled in the are will recognize, depends on the particular metal in question. Thus, the term "M" is not meant to limit the metal ion to any particular state of valency. The preferred metal ions, as indicated below, however, are $^{90}Y^{3+}$ and $^{111}In^{3+}$.

Preferred for use in the present invention are haptens that form chelate complexes with metal ions generally selected from indium-111 ("$^{111}In$"), technetium-99m ("$^{99m}Tc$"), copper-67 ("$^{67}Cu$") gallium-67 ("$^{67}Ga$") and yttrium-90 ("$^{90}Y$"). The metal ion $^{111}In$ is generally preferred for diagnostic application, i.e. imaging, while $^{90}Y$ is generally preferred for therapeutic application. Additionally, the selection of a suitable metal ion may involve consideration of the affinity the metal complex has for the anti-hapten antibody utilized in the invention as different metal complexes exhibit different affinities for certain anti-hapten antibodies.

The compounds of the invention having designations DB-I to DB-VIII are particularly useful in overcoming these latter difficulties associated with an anti-hapten antibody showing different affinities for different metal complexes. These particular haptens of the invention possess a unique "dumbbell" shape (thus, the designation "DB") with two separate moieties capable of chelating metal ions used in the invention. For example, the DTPA moiety could complex, for example, $^{111}In$, the preferred metal ion for diagnostic purposes. Once the diagnostic procedure is complete, the same hapten can be used to complex, at the DTPA moiety, a metal ion, for example, $^{90}Y$, useful for therapeutic applications. Thus, the problem of different anti-hapten antibody affinities for different hapten-metal ion complexes could be alleviated or overcome. A single hapten could be used for both diagnostic and therapeutic applications.

The preferred haptens of the invention comprise benzyl EDTA derivatives. Such haptens contain structural moieties that can conveniently complex a variety of metal ions, including $^{111}In$ and $^{90}Y$ for the desired diagnostic or therapeutic application. Benzyl EDTA, analogs thereof, and methods for their preparation are described in U.S. Pat. No. 4,622,420. In accordance with the invention, such haptens are derivatized to have a structure represented by Formula (I):

p-$R_1$-$C_6H_4$-$CH_2$-EDTA-M (I)

wherein

$R_1$ is

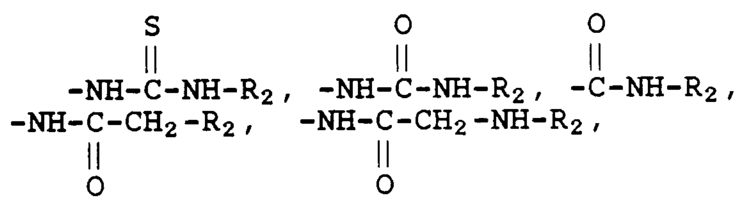

$-NH-R_2$ or 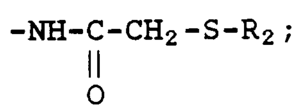

$R_2$ is hydrogen, an amino group, -OC(Y), -p-phenyl-$CH_2$-Y, an unsubstituted $C_1$ to $C_{30}$ branched or straight chain alkyl group; a substituted $C_1$ to $C_{30}$ branched or straight chain alkyl, cycloalkyl, aryl or arylalkyl group, in which the substituents are one or more of any of:

hydroxy, carboxy, =O, =S,

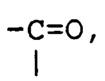

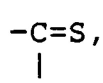

-S-, $-SR_4$, fluoro, chloro, bromo, iodo, amino, nitro, $-SO_3H$, $-NHR_3$, $-NHR_4$, $-N(R_3)_2$, $-CONHR_3$, $-COOR_3$, $-SO_4$, $-PO_4$, phenyl, benzyl, imidazolo, or a group of the formulae:

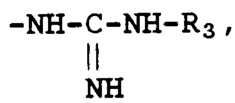, 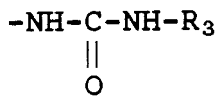, 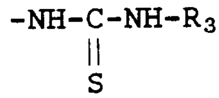;

wherein

$R_3$ is hydrogen, a $C_1$ to $C_{30}$ straight or branched chain alkyl group, -p-phenyl-$CH_2$-Y,

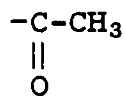,

or a non-reactive functional group;

Y is EDTA, DTPA, DOTA, HETA, TRITA or TETA;

$R_4$ is H or

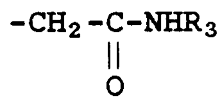;

and, M is a metal ion.

Preferred compounds of the invention are represented by Formula (Ia):

p-$R_1$-$C_6H_4CH_2$EDTA-M (Ia)

wherein $R_1$ is

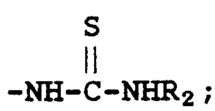; 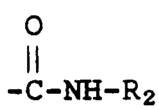, or 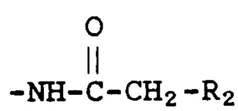;

in which $R_2$ is hydrogen; $NH_2$; an unsubstituted $C_1$-$C_{30}$ branched or straight chain alkyl group; or a $C_1$-$C_{30}$ branched or straight chain alkyl, cycloalkyl, aryl or arylalkyl group substituted by one or more substituents independently selected from the group consisting of:

-OH; -COOH; =0; =S; -S-; -Cl; -F; -Br; -I; $-NH_2$; $-N0_2$; $-S0_3H$; $-NH-R_3$; $-CONHR_3$; $-COOR_3$; $-S0_4$; $-P0_4$; phenyl; and benzyl;

$R_3$ is H; an alkyl group; or a non-reactive functional group; and M is a metal ion.

Additionally, $R_2$ may further comprise non-reactive functional groups which may include substituents that exhibit specific tissue interactions, e.g., amino acids, polypeptides, nucleotides, polynucleotides, carbohydrates or lipids. As used for $R_2$ and $R_3$, the term "non-reactive functional group" refers to a functional group incapable of forming a covalent bond with tissue or serum components, and the anti-hapten antibody utilized, under physiological conditions.

Especially preferred compounds of the invention are those represented by Formula (Ib):

p-$R_1$-$C_6H_4$-$CH_2$-EDTA-M (Ib)

wherein

$R_1$ is

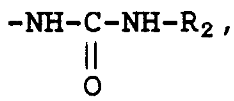, 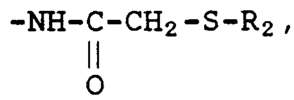, 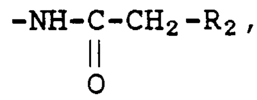,

$-NH-R_2$, 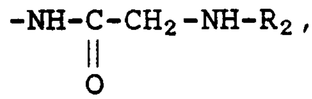, or 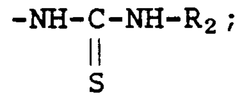;

$R_2$ is -p-phenyl-$CH_2$-Y, -OC{(Y), a substituted $C_1$ to $C_{30}$ branched or straight chain alkyl, cycloalkyl, aryl or arylalkyl group, in which the substituents are one or more of any of:

hydroxy,

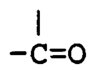, 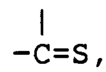,

$-SR_4$, $-NHR_3$, $-NHR_4$, $-N(R_3)_2$, or

a group of the formulae:

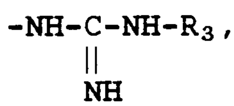, 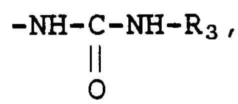, 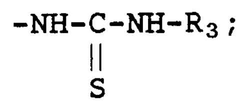;

wherein

$R_3$ is -p-phenyl-$CH_2$-Y, or

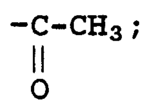;

Y is EDTA, DTPA, DOTA, HETA, TRITA or TETA;

$R_4$ is H or

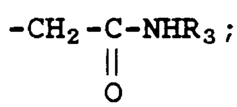;

and, M is a metal ion.

In one embodiment, preferred compounds of the invention are p-thioureidobenzyl EDTA derivatives or analogs thereof, which are derived from isothiocyanatobenzyl EDTA. Isothiocyanatobenzyl EDTA and isothiocyanatobenzyl DTPA, analogs thereof, and methods for their preparation are described in U.S. Patent No. 4,622,420. Such preferred haptens of the invention, have a structure represented by the following formula:

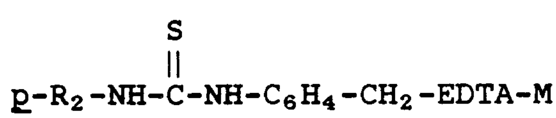

wherein $R_2$ is hydrogen; $NH_2$; an unsubstituted $C_1$-$C_{18}$ branched or straight chain alkyl group; or a $C_1$-$C_{18}$ branched or straight chain alkyl, cycloalkyl, aryl or arylalkyl group, substituted by one or more substituents independently selected from the group consisting of:

-OH; $-NH_2$; -COOH; $-CONH_2$; phenyl; benzyl; $-SO_3H$; and $-NO_2$; and

M is a metal ion.

In addition, $R_2$ may further comprise non-reactive functional groups including substituents that may exhibit specific tissue interactions, e.g., amino acids, polypeptides, nucleotides, polynucleotides, carbohydrates, or lipids.

In Formulae (I) and (Ib), the designation -OC(Y) is meant to relate to one skilled in the art that the moiety "Y", as defined in the formulae, is linked to the adjacent portion of the molecule through one of the carboxy groups of the Y moiety. On the other hand, the designation -Y, alone, is meant to indicate that the Y moiety is linked to the adjacent portion of the molecule through one of the methylene ($-CH_2$) groups present on Y moiety.

The structures of a series of novel derivatized haptens which are preferred for use in the invention are shown in Table I. The abbreviations set forth in Table I will be used later to refer to these novel haptens.

## TABLE I

## DERIVATIZED HAPTENS OF THE INVENTION

$$\underline{p}\text{-}R_2\text{-}NH\text{-}\underset{\underset{S}{\|}}{C}\text{-}NH\text{-}C_6H_4\text{-}CH_2\text{-}EDTA\text{-}M$$

| $R_2$ | HAPTEN ABBREVIATION |
|---|---|
| $-H$ | TUBE |
| $-CH_2CH_3$ | ETUBE |
| $-(CH_2)_3CH_3$ | BUTUBE |
| $-(CH_2)_7CH_3$ | OTUBE |
| $-CH_2CH_2OH$ | EOTUBE |
| $-CH_2CH_2NH_2$ | NUBE |
| $-C_6H_5$ | PHTUBE |
| $-CH_2C_6H_5$ | BETUBE |
| $-4-(C_6H_4)CH_2CO_2H$ | PHATUBE |
| $-(CH_2)_2CO_2H$ | PATUBE |
| $-(CH_2)_3CO_2H$ | BATUBE |
| $-(CH_2)_4CH(NHCOCH_3)CONH_2$ | BALTUBE |
| $-(CH_2)_2NHCO(CH_2)_3CO_2H$ | GNUBE |
| $-C_6H_4-CH_2-DTPA$ | DB-III |
| $-CH_2CH_2-NHC(=S)NH-C_6H_4-CH_2DTPA$ | DB-IV |
| $-(CH_2)_4-NHC(=S)NH-C_6H_4-CH_2DTPA$ | DB-V |

Additionally preferred compounds of the invention are those provided in Table II:

Table II

| X-$CH_2$-(C = O)-NH-$C_6H_4$-$CH_2$-EDTA-M | |
|---|---|
| X | ABBREVIATION |
| -$S(CH_2)_4SCH_2$(C = O)$NHC_6H_4CH_2$DTPA | DB-I |
| -$SCH_2$CH(OH)CH(OH)$CH_2SCH_2$(C = O)$NHC_6H_4CH_2$DTPA | DB-II |
| -NH-$C_6H_4$-$CH_2$-DTPA | DB-VI |
| -$NHCH_2$CH(OH)$CH_2NHCH_2$(C = O)$NHC_6H_4CH_2$DTPA | DB-VIII |

Those skilled in the art will recognize that because the pharmacokinetic criteria for imaging and therapeutic agents may differ, the series of novel derivatized haptens provided by the invention permits the selection of haptens having desirable pharmacokinetics for a particular application of the invention. For example, a particular derivatized hapten may be desirable as a therapeutic agent because of its ability to accumulate at a disease site but may be undesirable as an imaging agent because of its inability to clear rapidly from circulation. Additionally, those skilled in the art will appreciate that the present invention is not limited to the specific derivatized haptens set forth in Tables I or II. The present invention contemplates, therefore, modifications to the structures of the novel derivatized haptens specifically disclosed, provided such structural modifications are intended to modify the inherent pharmacokinetic properties of these haptens for purposes of imaging or therapy.

In accordance with the invention, the derivatization of a hapten to modify the dissociation rate of the hapten-antibody complex, and the interaction of the hapten with serum and tissue components, can be accomplished by standard procedures well-known in the art. For example, hapten structure can be modified by derivatization of the aromatic amine in (S)-4-aminobenzyl EDTA by acylation, alkylation, Schiff's base formation (which may or may not be followed by reduction to the amine), conversion to a reactive isocyanate and subsequent conversion to a substituted urea conversion to a sulfonamide or diazotization and subsequent reaction with a nucleophile. These reactions can be accomplished with compounds containing simple aliphatic chains, or branched chains, or aromatic or heterocyclic compounds which may or may not contain functional groups. (See, e.g., March, J. Advanced Organic Chemistry, John Wiley & Sons, New York, (1985)). Additionally, in accordance with the invention, the structure of a hapten is preferably modified to enhance the biodistribution and localization of the hapten for a particular application of the invention. Further, it will be understood that haptens derivatized in accordance with the present invention retain the ability to bind the anti-hapten antibody selected for use.

The preferred haptens of the invention represented by Formulae (I), (Ia), and (Ib) are prepared using standard synthetic methods familiar to one skilled in the art. For example, the preferred thiourea-type compounds of the invention are prepared by reacting a precursor isothiocyanate compound with an amine. The most useful isothiocyanates for purposes of the present invention are isothiocyanatobenzyl EDTA ("ITCBE") and isothiocyanatobenzyl DTPA ("ITCBD"), both of which are prepared according to the teachings of U.S. Patent No. 4,622,420, incorporated herein by reference. Thus, one skilled in the art determines which compound of the invention to prepare and then reacts one, or possibly both, of ITCBE or ITCBD with the necessary amine compound(s) to obtain the desired structure. Likewise, to prepare the corresponding macrocyclic polyamine derivatives of the invention, the corresponding isothiocyanate derivatives of DOTA, HETA, TRITA, and TETA are described in U.S. Pat. NO. 4,678,667, the teachings of which are incorporated herein by reference. The preparation of HETA, DOTA, TRITA and TETA also are disclosed in U.S. Pat. No. 4,678,667. An improved synthesis of DOTA is described in Moi, et al., J. Am. Chem. Soc., 110, 6266 (1988). The corresponding isothiocyanate compounds useful as starting materials in the present invention are prepared in a manner completely analogous to the preparation of ITCBD and ITCBE.

To prepare the amide-type compounds of the invention, two starting materials are particularly useful: p-bromoacetamidobenzyl EDTA ("BABE") and p-bromoacetamidobenzyl DTPA ("BABD"). These compounds are prepared according to the teachings of DeReimer, et al., J. Lab. Comp. and Radiopharm., 18, 1517 (1981) and U.S. Patent No. 4,622,420. The corresponding bromoacetamido-derivatives for HETA, DOTA, TRITA, and TETA are prepared in a manner completely analogous to the preparation of BABE and BABD. The preparation of these starting materials is described in U.S. Pat. No. 4,678,667, incorporated herein by reference. The alpha-bromo moiety of these compounds renders the adjacent methylene moiety particularly susceptible to nucleophilic attack from groups such as free sulfhydryl or amino groups. Thus, to prepare the desired compound of the invention, one needs only to determine the appropriate intermediate $R_2$ moiety of Formulae (I), (Ia), or (Ib) and react it with the required starting material(s).

Typically, the desired compounds of the invention are prepared in aqueous buffer solutions at pH levels from about 6 to about 12. Preferred pH ranges are from about 8 to about 11. The progress of the reaction can be monitored easily using HPLC. The temperature is not crucial but preferably the reaction is run at temperatures from about 0° C. to about 60° C, preferably from about 20° C. to about 40° C.

The product obtained from the reaction can be purified using standard chromatography techniques. The preferred separation method involves anion-exchange chromatography using, for example, a DEAE Sephadex A-25 resin (Pharmacia Fine Chemicals) or an AG-1X8, formate column (BioRad Laboratories, Richmond California). Of course, those skilled in the art will recognize other equally useful means for purification of the final products.

In addition, it will be recognized that many compounds of the present invention contain basic and acidic groups which are capable of forming pharmaceutically-acceptable acid or base addition salts. For example, those compounds which contain a free carboxyl group are able to react with alkaline earth metal bases, for example, sodium bicarbonate, sodium hydroxide, potassium carbonate, potassium bicarbonate, potassium hydroxide, calcium hydroxide, etc., to form pharmaceutically-acceptable salts which also are useful in the invention. Likewise, free amino groups present in the molecule are capable of reacting with acidic reagents to form the corresponding acid addition salts. For example, an amino-containing compound of the invention could be reacted with acids such as hydrochloric, sulfuric, tartaric, lactic, nitric, etc., to prepare the corresponding salt. "Pharmaceutically-acceptable" in this context refers to those salt forms which are useful in the treatment or diagnosis of a warm-blooded animal. Consequently, pharmaceutically-acceptable salt forms of appropriate compounds are also encompassed by the invention.

Although the methods described here and further elaborated in the accompanying non-limiting examples are the presently preferred method for preparing the compounds of the invention, one skilled in the art will recognize other means which may work equally as well. Thus, the methods provided for preparing compounds of Formulae (I), (Ia), or (Ib) are not to be construed as limiting on the means for preparing the desired compound. Other equally-acceptable means for preparing the compounds of may be available and are meant to be encompassed by the present invention.

Thus, in another aspect of the invention there is provided a process for preparing a compound of Formula (I):

$\underline{p}$-$R_1$-$C_6H_4$-$CH_2$-EDTA-M (I)

wherein

$R_1$ is

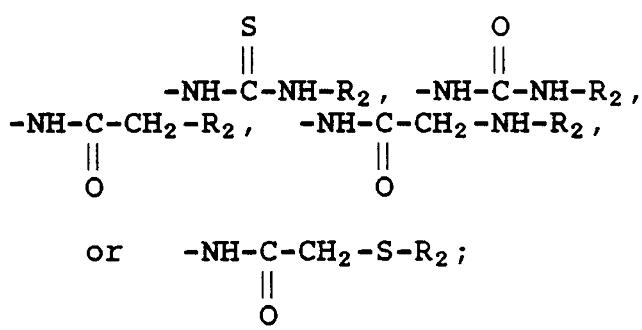

$R_2$ is hydrogen, an amino group, -$\underline{p}$-phenyl-$CH_2$-Y, an unsubstituted $C_1$ to $C_{30}$ branched or straight chain alkyl group; a substituted $C_1$ to $C_{30}$ branched or straight chain alkyl, cycloalkyl, aryl or arylalkyl group, in which the substituents are one or more of any of:

hydroxy, carboxy, =O, =S,

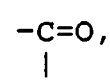

$$\begin{array}{l}-C=S, \\ \;\;| \end{array}$$

-S-, $-SR_4$, fluoro, chloro, bromo, iodo, amino, nitro, $-SO_3H$, $-NHR_3$, $-NHR_4$, $-N(R_3)_2$, $-CONHR_3$, $-COOR_3$, $-SO_4$, $-PO_4$, phenyl, benzyl, imidazolo, or agroup of the formulae:

$$\begin{array}{c}-NH-\underset{\underset{NH}{\|}}{C}-NH-R_3,\quad -NH-\underset{\underset{O}{\|}}{C}-NH-R_3,\quad \text{or}\quad -NH-\underset{\underset{S}{\|}}{C}-NH-R_3;\end{array}$$

wherein

$R_3$ is hydrogen, a $C_1$ to $C_{30}$ straight or branched chain alkyl group, -p-phenyl-$CH_2$-Y,

$$-\underset{\underset{O}{\|}}{C}-CH_3,$$

or a non-reactive functional group;

Y is EDTA, DTPA, DOTA, HETA, TRITA or TETA;

$R_4$ is H or

$$-CH_2-\underset{\underset{O}{\|}}{C}-NHR_3;$$

and, M is a metal ion,

which comprises,

(A) in the case in which $R_1$ is $-NH(C=S)NH-R_2$, $-NH(C=O)NH-R_2$,

(1) reacting isothiocyanatobenzyl EDTA, or isocyanatobenzyl EDTA, as appropriate, or a metal chelate thereof, with a compound of the formula $H_2N-R_2$, in which $R_2$ is as described above; or,

(2) reacting a compound of the formula $R_2-N=C=S$, $R_2-N=C=S$, as appropriate, or a metal chelate thereof, with a compound of the formula:

EDTA-$CH_2$-$C_6H_4$-$(NH(C=S)NH)_m$-$(X)_n$-$NH_2$, or
EDTA-$CH_2$-$C_6H_4$-$(NH(C=O)NH)_m$-$(X)_n$-$NH_2$

or a metal chelate thereof, in which X is a $C_1$ to $C_{30}$ branched or straight chain alkyl, aryl or arylalkyl group optionally substituted with one or more of the substituents allowed in the definition of $R_2$ above, and n and m, independently, are 0 or 1;

(B) if $R_1$ is $-NH(C=O)CH_2-S-R_2$, reacting p-bromoacetamidobenzyl-EDTA ("BABE"), or a metal chelate thereof, with a compound of the formula $HS-R_2$, in which $R_2$ is as defined above;

(C) if $R_1$ is $-NH(C=O)CH_2-NH-R_2$, reacting p-bromoacetamidobenzyl EDTA ("BABE") with a compound of the formula $H_2N-R_2$, in which $R_2$ is as defined above; or, if $R_1$ is $-NH(C=O)CH_2-NH-R_2$ in which a substituent of $R_2$ is $NHR_4$, reacting p-bromoacetamidobenzyl-Y, in which Y is EDTA, DTPA, DOTA, HETA, TRITA or TETA; or a metal chelate thereof, with a compound of the formula:

EDTA-$CH_2$-$C_6H_4$-$NH(C=O)CH_2$-NH-X-$NH_2$,

or a metal chelate thereof, in which X is a $C_1$ to $C_{30}$ branched or straight chain alkyl, aryl, or arylalkyl group optionally substituted with one or more of the substituents allowed in the definition of $R_2$, above; and,

(D) if desired, salifying or preparing the metal chelate of the product obtained.

For purposes of the present invention, the antibodies selected for use are anti-hapten antibodies capable of specifically binding and complexing with the hapten selected for use. It will be appreciated by those skilled in the art that the same antibody can be utilized to complex with a hapten of the invention which may contain a broad range of functional groups.

Preferred for use in the invention are monoclonal antibodies, particularly monoclonal antibodies exhibiting the ability to bind preferentially to chelate complexes, said complexes comprising a specific metal ion and a chelating agent, e.g., complexes of $^{111}In$ and benzyl EDTA derivatives of the invention. Such monoclonal antibodies and methods for the preparation are described in U.S. Pat. No. 4,722,892, the disclosure of which is incorporated by reference. However, those skilled in the art will appreciate that monoclonal or polyclonal antibodies may be utilized in the invention provided the antibodies selected exhibit the requisite specificity for the hapten selected for use. Methods for the preparation of monoclonal and polyclonal antibodies are now quite well known in the art. See, e.g., Kohler, G. and Milstein, C., Nature 256, 495 (1975).

Particularly preferred for use in the invention are bifunctional monoclonal antibodies having a dual specificity, with one or more binding sites for a hapten of the invention and one or more binding sites for a disease site, e.g. a tumor target. Because such bifunctional antibodies have specificity for the hapten as well as for a disease site, they are capable of functioning both as carriers for the hapten and as disease site-associated receptors. Such bifunctional antibodies, therefore, provide an effective mechanism for the site-directed delivery and localization of the derivatized hapten to the disease site. The disease sites which such bifunctional antibodies recognize may be, for example, tumor-associated antigens such as alpha-fetoprotein ("AFP"), carcinoembryonic antigen ("CEA"), human choriogonadotropin ("HCG"), prostatic acid phosphatase ("PAP"), prostate specific antigen ("PSA"), ferritin, bombesin, melanoma-associated antigens p97 and gp240, or milk fat globulins. Alternatively, the disease-sites for which such bifunctional antibodies are specific may be, for example, sites of infection or thrombosis. Bifunctional antibodies and methods for their preparation are described in European Patent Publication 105360, the disclosure of which is incorporated by reference. The preparation of bifunctional antibodies may be accomplished biologically, for example, by cell fusion techniques to produce polydomas, or chemically, by synthetic techniques, all of which now are well-known in the art. See, for example, U.S. Pat. Nos. 4,470,925 and U.S. 4,444,878.

In certain applications of the invention, human monoclonal antibodies, particularly human bifunctional antibodies, having the requisite hapten-binding specificity are preferred. Human monoclonal antibodies can be prepared by techniques well known in the art and are produced by hybridomas which are, for example, the fusion product of a human B-lymphocyte with a human or or mouse myeloma cell line. Human bifunctional antibodies can also be prepared by conventional techniques known in the art. See, Human Hybridomas and Monoclonal Antibodies, edited by E.G. Engleman, S.K. Foung, J. Larrick and A. Raubitscheck, Plenum Press, New York (1985).

Also preferred for use in certain applications of the invention are chimeric monoclonal antibodies capable of complexing with the hapten selected for use. Chimeric antibodies are generally comprised of a variable region, i.e. binding region, derived from one mammalian species, e.g., mouse, and a constant region derived from a second and different mammalian species, e.g. human. Murine/human chimeric antibodies will be beneficial in certain applications of the invention because such antibodies are expected to retain the specificity and affinity of murine antibodies while being substantially less immunogenic. Such murine/human chimeric antibodies, which may be the product of fused immunoglobulin genes, can be prepared by recombinant DNA and gene transfection techniques well-known in the art. See, for example, European Patent Publication 173494 and WO 86/01533. Chimeric monoclonal antibodies which are bifunctional are particularly preferred for use in certain applications of the invention. The chimeric bifunctional antibodies useful in the invention may be the product of fused immunoglobulin genes encoding for variable region specificity both for the hapten selected for use and a disease site, e.g., tumor target. Such antibodies may be able to be prepared by conventional recombinant DNA and gene transfection techniques. See, for example, European Patent Publication 012507. In addition, synthetic chimeric, bifunctional, or chimeric-bifunctional antibodies can be prepared from antibody half-molecules, or fragments thereof, cross-linked with a bifunctional cross-linking agent such as bis-(maleimido)-methyl ether ("BMME"), or other such crosslinking agents familiar to those skilled in the art.

Also, encompassed by the term "chimeric antibody", whether in reference to chimeric antibodies per se, cross-linked chimeric antibodies, or chimeric-bifunctional antibodies, is the concept of "humanized antibody", that is those antibodies in which the framework or complementarity determining regions ("CDR") have been modified to comprise the CDR of an immunoglobulin of different specificity as compared to that of the parent immunoglobulin. In a preferred embodiment, a murine CDR is grafted into the framework region of a human antibody to prepare the "humanized antibody". Particularly preferred CDR's correspond

to those representing sequences recognizing the antigens noted above for the chimeric and bifunctional antibodies. The reader is referred to the teaching of EP A 0 239 400 (Sept. 30, 1987), incorporated herein by reference, for its teaching of CDR modified antibodies.

In addition, the invention encompasses within its scope immunoglobulins or immunoglobulin fragments to which are fused active proteins, for example, an enzyme of the type disclosed in Neuberger, et al., WO 86/01533, published March 13, 1986. The disclosure of such products is incorporated herein by reference.

Additionally, those skilled in the art will recognize that the present invention encompasses in any of the previously described antibody formats the use of antibody fragments including Fab, Fab′ and F(ab)′$_2$ fragments, or any other antibody fragment retaining the essential binding function of the antibody selected for use. However, it will be recognized that in those utilities of the invention requiring bifunctional antibodies, Fab or Fab′ fragments may only be suitably utilized if the haptens of the invention have some specificity of their own. Antibody fragments can be prepared by proteolytic enzyme digestion, for example, by pepsin or papain digestion, reductive alkylation, or recombinant techniques, all of which are well-known in the art. See, Nisonoff, A., Molecular Immunology. Sinauer Associates, Massachusetts (1982). Those skilled in the art will also appreciate that in certain applications of the invention it may be beneficial to use a mixture of two or more antibodies or fragments thereof, or mixtures of antibody fragments with whole antibody.

Further, it will be understood by those skilled in the art that for certain applications of the invention it may be beneficial to select a high affinity antibody, while in other applications of the invention the selection of a low affinity antibody will be advantageous. Accordingly, the present invention includes the selection of antibodies which may be characterized as having high or low affinity for the hapten selected for use, and in the case of bifunctional antibodies, high or low affinity for a disease site.

In accordance with the invention, the derivatized hapten and anti-hapten antibody can be administered to the patient sequentially, simultaneously, or in combination as a complex for purposes of in vivo imaging or therapy.

In preferred methods of the invention, in which the anti-hapten antibody selected for use is a bifunctional antibody, the hapten of the invention and the antibody are administered separately. Preferably, the antibody is administered and permitted to distribute itself throughout the patient prior to administration of the derivatized hapten. After sufficient time has elapsed to permit localization of the bifunctional antibody at the disease site, the derivatized hapten is administered to the patient. The derivatized hapten can be administered in multiple doses over a period of time or by slow infusion. The derivatized hapten antibody complex is thereafter formed in vivo and the diagnostic or therapeutic effect can be observed. Such methods are desirable for applications of the invention in which the bifunctional antibody selected for use requires a relatively long period of time for localization at disease sites, or the derivatized hapten has a relatively short serum half-life or a relatively short isotope half-life.

In an additionally preferred method of the invention, a first bifunctional anti-hapten antibody is selected for use and administered to the patient followed, after a period of time, by administration of a mixture of the derivatized hapten and a second carrier anti-hapten antibody. The derivatized hapten and second carrier antibody are administered to the patient after sufficient time has elapsed to permit localization of the first bifunctional antibody at the disease site. The second carrier antibody permits, in effect, transfer of the derivatized hapten to the prelocalized bifunctional antibody at the disease site. The administration of the derivatized hapten with a second carrier antibody may be required in certain applications of the invention in which the concentration of circulating bifunctional antibody is insufficient to bind the derivatized hapten for delivery to the disease site prior to clearance of the hapten from circulation.

Alternatively, the derivatized hapten and the antibody can be administered to the patient as a mixture. Accordingly, the derivatized hapten and the antibody can be combined in vitro to permit the formation of derivatized hapten-antibody complexes prior to administration to the patient. After administration of the complexes to the patient, the desired diagnostic or therapeutic effect can be observed.

Administration of the derivatized hapten and the antibody, whether administered separately or as a mixture, can be accomplished by intravenous, intraperitoneal, intra-lymphatic, subcutaneous or intramuscular injection.

As indicated above, the novel derivatized haptens provided by the invention are useful in compositions for in vivo imaging and therapy. Thus, in an additional embodiment of the invention, compositions are provided for in vivo imaging and therapy comprising a hapten wherein the hapten is derivatized to modify the dissociation rate of a hapten-antibody complex and the interaction of the hapten with serum components and tissue compartments, thereby increasing or decreasing serum half-life and radiation doses to tissues. Accordingly, the pharmacokinetics of the compositions of the invention are modified for purposes of imaging and therapy. Preferably, the biodistribution and localization of the compositions of the invention are enhanced as compared to compositions comprising unmodified haptens. More specifically, the enhanced

biodistribution and localization of the compositions of the invention result in an increased and more rapid accumulation of the derivatized hapten at the disease site, and improved clearance of the derivatized hapten from normal tissues.

Preferred for use in the compositions of the present invention are derivatized haptens having structures as previously described. Especially preferred are the series of novel hapten derivatives set forth in Tables I and II above. As previously indicated, the series of novel hapten derivatives provided by the invention permits the selection of haptens most desirable for particular applications of the invention.

The compositions of the present invention may further comprise an antibody capable of complexing with the hapten, preferably a monoclonal antibody. Particularly preferred for use in the compositions of the invention are bifunctional antibodies having hapten-binding specificity as well as specificity for a disease site. Additionally, compositions comprising human antibodies or chimeric antibodies are particularly preferred in certain applications of the invention. Further, those skilled in the art will appreciate that the compositions of the invention may be comprised of antibody fragments or mixtures of antibodies or fragments thereof, as described earlier.

In further embodiments of the invention, methods are provided for in vivo imaging and therapy which comprise administering to a patient a predetermined effective dosage of a hapten, derivatized in accordance with the invention, and an antibody, or antibody fragment, capable of complexing with the hapten. The derivatized hapten and anti-hapten antibody, which may be administered sequentially, simultaneously, or as a complex, are prepared preferably for use in formulations suitable for injection.

Thus, the invention also provides pharmaceutical formulations, comprising a compound of Formula (I), (Ia), or (Ib), associated with one or more pharmaceutically-acceptable carriers, diluents, or excipients therefor. As those skilled in the art will recognize, "pharmaceutically-acceptable" refers to those carriers, diluents, or excipients recognized as useful in the diagnosis or therapy of a warm-blooded animal.

Such formulations are preferably in unit dosage form, each dosage containing, for example, from about 10 picomols to about 10 millimols of the desired hapten. Preferred formulations contain from about 100 picomols to about 1 micromol of the derivatized hapten. The novel derivatized haptens and anti-hapten antibodies are effective over a wide dosage range depending upon factors such as the disease state involved, the antigen density at the disease site, the hapten-antibody affinity, the extent of non-specific binding interactions, the manner of administration and the condition of the patient.

Figure 1: represents the tumor uptake at 24 and 48 hours of the derivatized haptens shown in Table I in a pre-mix protocol.

Figure 2: respesents the tumor to blood ratios at 24 and 48 hours of the derivatized haptens shown in Table I in a pre-mix protocol.

Figure 3: represents the biodistributions (percent dose/gram organ) at 4 hours for the derivatized haptens EOTUBE, TUBE, NUBE and PHATUBE in a prelocalization protocol.

Figure 4: represents the biodistributions (percent dose/gram organ) at 24 hours for the derivatized haptens EOTUBE, TUBE, NUBE, and PHATUBE in a prelocalization protocol.

Figure 5: represents the biodistributions (percent dose/gram organ) at 48 hours for the derivatized haptens EOTUBE, TUBE, NUBE and PHATUBE in a prelocalization protocol.

Figure 6: represents the tumor to organ ratios at 4 hours for the derivatized haptens EOTUBE, TUBE, NUBE and PHATUBE in a prelocalizatlon protocol.

Figure 7: represents the tumor to organ ratios at 24 hours for the derivatized haptens EOTUBE, TUBE, NUBE and PHATUBE in a prelocalization protocol.

Figure 8: represents the tumor to organ ratios at 48 hours for the derivatized haptens EOTUBE, TUBE, NUBE and PHATUBE in a prelocalization protocol.

The following non-limiting examples are provided to further illustrate the invention.

## EXAMPLES

The molecule used in some of the examples to prepare the novel hapten derivatives of the invention was isothiocyanatobenzyl EDTA ("ITCBE"), the structure of which is shown below:

p-S = C = N-$C_6H_4$-$CH_2$EDTA

Also used as a starting material in the examples is "ITCBD", isothiocyanatobenzyl DTPA, the structure of which is shown below:

p-S = C = N-$C_6H_4$-$CH_2$-DTPA.

Unless stated otherwise, for in vitro and in vivo characterization, indium complexes of the novel hapten derivatives of the invention were used. For in vitro characterization, the monoclonal antibody, designated as CHA255 and having specificity for the indium complex of benzyl EDTA, was used. However, as one skilled in the art would appreciate, alternative antibodies having specificity for indium benzyl EDTA complexes may be prepared and used interchangeably with CHA255.

For in vivo characterization, a bifunctional antibody was used in conjunction with the novel hapten derivatives of the invention. The bifunctional antibody used, designated as ECH037.2, has a dual specificity for carcinoembryonic antigen (CEA) and the indium complex of benzyl EDTA. Those skilled in the art will appreciate, however, that alternative antibodies having specificity for CEA and indium benzyl EDTA complexes may be utilized and interchanged with ECH037.2.

## EXAMPLE 1

## HAPTEN DERIVATIZATION

### A. Syntheses of Hapten Derivatives of the Invention

All glassware and plasticware used in the syntheses was acid washed to remove metal ions. Glassware was washed with mixed acids (equal volumes of concentrated sulfuric and nitric acids) and plasticware was washed in 6M HCl. Both were rinsed exhaustively with deionized water before use. Metal-free plastic pipet tips from BioRad (Richmond, CA) were used to transfer solutions. Deionized water with a resistivity of at least 15 Mohm-cm was used throughout. This was obtained by passing water through a Milli Q Milli-RO 4 apparatus (Millipore Corporation, Bedford, MA). "Milli-Q water" refers to this deionized water. All reagents used were the purest commercially available. The different amines were all purchased from Aldrich Laboratories (Milwaukee, Wisconsin) and were used without further purification.

(S)-p-nitrobenzyl EDTA was prepared and converted to (S)-4 isothiocyanatobenzyl EDTA ("ITCBE") as described in U.S. Pat. No. 4,622,420, and Meares, C.F., Anal. Biochem. 142, 68-75 (1984). Analogously, p-isothiocyanatobenzyl DTPA ("ITCBD") was prepared. Similarly, for the preparation of the corresponding DOTA, HETA, TETA and TRITA haptens of the invention, one would use the corresponding isothiocyanato-derivative described in U.S. Pat. No. 4,678,667, incorporated herein by reference. Using the HPLC gradient system described below, ITCBD had a retention time of 5.0 minutes. The lyophilized ITCBE was resuspended in 0.3 M HC1 (Ultrex, J.T., Baker Chemical, Phillipsburg, New Jersey) to a final concentration of roughly 50 mM. This solution was stored at -70° C. Unless indicated otherwise, all reactions were performed in aqueous solution at 40° C.

Products were purified by anion exchange chromatography on either AG-1x8, formate form (BioRad Laboratories, Richmond, CA) or DEAE Sephadex A-25 ("A-25 column") (Pharmacia Fine Chemicals, Uppsala, Sweden). Columns were monitored at 280 nm on a LKB UVicord S detector. Fluorescamine (Fluram™, Hoffman-LaRoche, Nutley, N.J.) was used to test for the presence of amines. The sample to be tested was spotted on Whatman 4 filter paper and then sequentially sprayed with 0.2 M sodium borate, pH 9.5 and 0.25 mg/ml fluorescamine in acetonitrile. Fluorescence, indicating the presence of an amine, was detected by illuminating the sample with a short wave-length UV lamp. HPLC analyses were done on a Hewlett-Packard 1090 liquid chromatograph with a microbore $C_{18}$ column (Hewlett Packard #79916 OD opt 552). Starting buffer was aqueous 50 mM triethylammonium acetate, 10 mM EDTA, pH 6.0. A 10 minute linear gradient from starting buffer to neat methanol was used throughout. The flow rate was kept constant at 0.4 ml/minute and column effluent was monitored at 280 nm. With this system, the ITCBE starting material had a retention time of 5.3 minutes.

NMR spectra were obtained on a Varian model XL-300 300 MHz instrument with multinuclear capabilities. Samples were dissolved in $D_2O$ and the pH adjusted with NaOD or DCl.

Chelate concentrations were determined by titration with indium spiked with radioactive $^{111}In$, in a modification of the procedure described by Meares, et al., Anal. Biochem. 142, 68-78 (1984). Standard indium solutions were prepared by dissolution of a precisely known mass of indium foil (Puratonic, Alfa Products, Danvers, Massachusetts) in Ultrex HCl (J.T. Baker, Chemical Co., Phillipsburg, New Jersey). A 0.13 M ammonium citrate buffer, pH 6.0, was used for the titrations, and the TLC system used was that described by Meares, et al.

Unless indicated otherwise, labeling of the haptens with designations starting with "DB" with $^{111}In^{3+}$ and $^{90}Y^{3+}$ were performed in the following manner: The hapten to be labeled is prepared as a 2 micromolar solution in 25 mg/ml glycine, pH 4.5-6.5. $^{111}In^{3+}$ labeling is done using a 2.5 mCi/ml solution of $^{111}In^{3+}$ in

0.088 M glycine. Equal volumes of the hapten solution and $^{111}In^{3+}$ solutions are combined, vortexed and allowd to stand at room temperature for at least 10 minutes. The labeling mixture then is neutralized by adding 0.22 M glycine, 0.2 M DTPA, 4% (w/v) $NH_4Cl$, pH 8.2, in a volume equal to three times the volume of $^{111}In^{3+}$ added. $^{90}Y^{3+}$ labeling is done by combining one volume of 200 mCi/ml $^{90}Y^{3+}$ in 70 mM HCl with 10 volumes of the DB hapten chelating agent. The mixture is vortexed and allowed to stand at room temperature for at least 5 minutes, but no longer than 6 minutes. The mixture is quickly neutralized by adding it to a volume equal to 14 times the volume of hapten chelating agent of 150 mM ascorbic acid, 50 mM Tris, 0.1 mM DTPA, pH 7.4.

Radio-HPLC of the $^{111}In^{3+}$ and $^{90}Y^{3+}$ labeled DB hapten chelates was done on an IBM model 9533 chromatograph using a $C_{18}$ reversed phase column (IBM No. 8635308). Elution was with a 20 minute linear gradient from neat buffer A (50 mM aqueous triethylammonium acetate, 10mM EDTA, pH 6.0) to neat methanol at a flow rate of 1 ml/min. Two different systems were used for radiodetection. $^{90}Y$ detection was done with a Canberra 2007p/802-3 sodium iodide crystal. $^{111}In$ detection was done with a Ludlem model 177 Alarm ratemeter coupled with a model 44-6 side window G-M detector. Both systems were in-line and coupled to an integrator. Prior to injection, samples were diluted with HPLC buffer A, defined above. In this system, all $^{111}In^{3+}$ labeled hapten chelates appeared as a single, well-defined peak. $^{90}Y^{3+}$ labeled DTPA species appeared as a doublet, with two peaks within 0.5 minutes of one another, with relative areas of 3:2 for the leading and trailing peak, respectively.

Absorbance measurements were made on a Hewlett Packard Model 8451A Diode Array spectrophotometer. 1 cm path length cuvettes were used throughout, and samples were diluted in, and measured against PBS (10 mM sodium phosphate, 150 mM NaCl, pH 7.2). Molar extinction coefficients were calculated for the metal-free chelating agents based on concentrations determined by indium titrations.

B. Synthesis of Hapten ETUBE

2.5 ml of 20 mM ITCBE was added to 1.25 ml of 200 mM ethylamine and the pH adjusted to 11.8 with 10 N NaOH. The volume was adjusted to 5 ml with $H_20$ and the mixture allowed to react for 15 minutes, at which time it was checked by HPLC analysis. All of the ITCBE was converted to ETUBE, with a retention time of 3.6 minutes.

The product was purified by anion exchange on a 5 ml AG1x8 (formate form) column, equilibrated with 0.5 M formic acid. The column was washed with 0.5 M formic acid until the effluent was fluorescamine negative, indicating that all of the excess amine was removed. The product, ETUBE, was eluted by washing the column with 8 M formic acid. Excess formic acid was removed by lyophilization. The product was characterized by indium titration, HPLC and UV/visible absorbance. The material had an absorbance maximum at 244 nm, with $\epsilon$ = 2.0 x $10^4$ $M^{-1}$ $cm^{-1}$

The structure of ETUBE is shown below

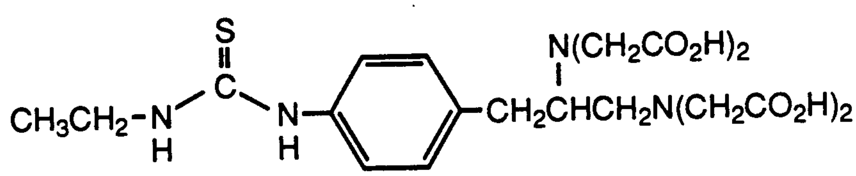

C. Synthesis of Hapten BUTUBE

BUTUBE was prepared exactly as described in Example 1B, except that 4-aminobutane was used in place of ethylamine. The product had an HPLC retention time of 4.8 minutes and an absorbance maximum of 244 nm with $\epsilon$ = 1.9 x $10^4$ $M^{-1}$ $cm^{-1}$.

The structure of BUTUBE is shown below:

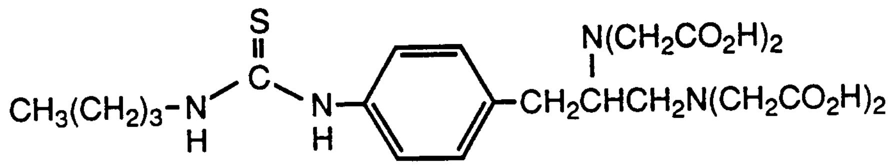

D. Synthesis of Hapten BATUBE

BATUBE was prepared exactly as described in Example 1B, except that 4-aminobutyric acid was used in place of ethylamine. The product had an HPLC retention time of 3.1 minutes and an absorbance maximum of 244 nm with $\epsilon$ = 1.8 x $10^4$ $M^{-1}$ $cm^{-1}$.

The structure of BATUBE is shown below

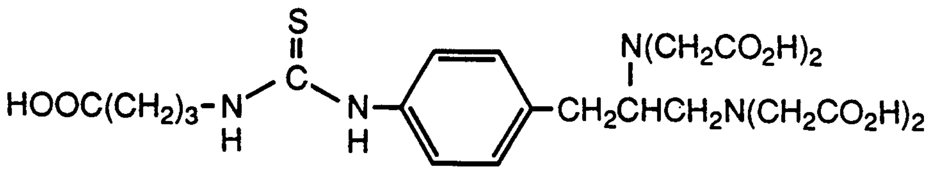

E. Synthesis of Hapten PATUBE

PATUBE was prepared exactly as described in Example 1B, except that $\beta$-alanine was used in place of ethylamine. The product had an HPLC retention time of 3.0 minutes and an absorbance maximum at 244 nm with $\epsilon$ = 1.8 x $10^4$ $M^{-1}$ $cm^{-1}$.

The structure of PATUBE is shown below:

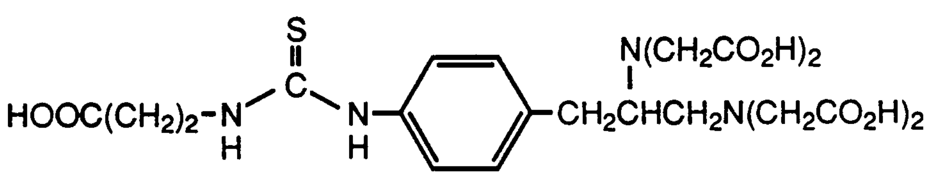

F. Synthesis of Hapten BETUBE

BETUBE was prepared exactly as described in Example 1B, except that benzylamine was used in place of ethylamine. The product had an HPLC retention time of 5.1 minutes and an absorbance maximum at 244 nm with $\epsilon$ = 2.4 x $10^4$ $M^{-1}$ $cm^{-1}$.

The structure of BETUBE is shown below:

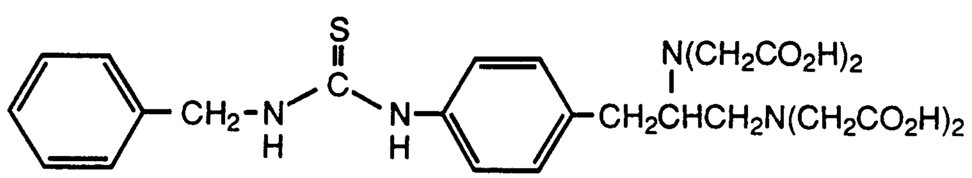

G. Synthesis of Hapten TUBE

2 ml of 42.7 mM ITCBE in 0.3 M HCl was lyophilized to dryness in an acid-washed flask. To this was added 1.2 ml of 5 M ammonium formate pH 10-10.5. After 15 minutes at room temperature, the reaction was checked by HPLC; only about half of the starting chelating agent had converted to product (retention time 3.14 minutes). The pH of the mixture was then adjusted to 10.5 by the addition of 25% (w/v) $NH_4OH$. The reaction was mixed and checked by HPLC. All of the ITCBE had converted to the desired product, TUBE. The reaction mixture was taken to dryness by lyophilization.

The structure of TUBE is shown below:

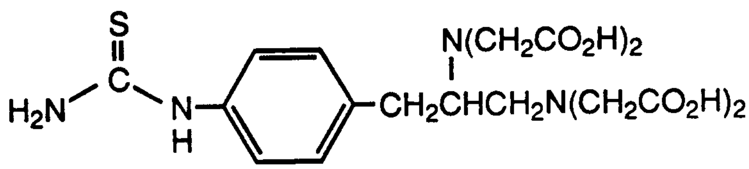

H. Synthesis of Hapten OTUBE

2.5 ml of 20 mM ITCBE was added to 1.25 ml of 20 mM octylamine in 40% (v/v) aqueous ethanol. The pH was adjusted to 11.8 with 1O N NaOH, and the volume increased to 5 ml by the addition of 40% (v/v) aqueous ethanol. The HPLC retention time of product was 7.4 minutes, and the absorbance maximum was 244 nm. Low aqueous solubility precluded an accurate determination of the molar extinction coefficient.

The structure of OTUBE is shown below:

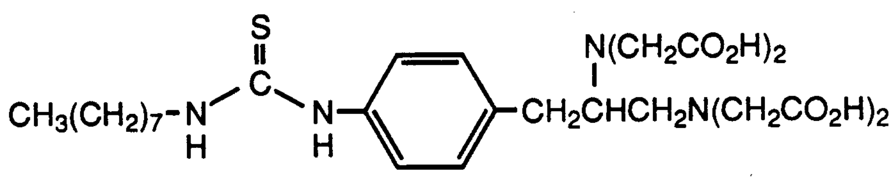

I. Synthesis of Hapten PHTUBE

2.5 ml of 20 mM ITCBE was added to 1.25 ml of 200 mM aniline and the pH was adjusted to 7.6 with 10 N NaOH. Addition of water brought the volume up to 5 ml, and the reaction was allowed to proceed for 75 minutes. The product was purified as described in Example 1B, and had an HPLC retention time of 4.4 minutes. The product has an absorbance maximum at 258 nm with $\epsilon$ = 2.3 x $10^4$ $M^{-1}cm^{-1}$.

The structure of PHTUBE is shown below:

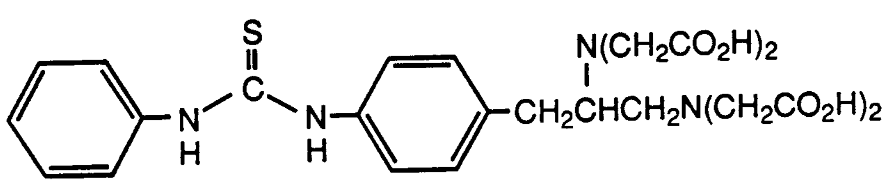

J. Synthesis of Hapten PHATUBE

PHATUBE was prepared exactly as described in Example 1H, except that p-amino phenylacetic acid was used as the amine. The product had an HPLC retention time of 3.5 minutes, with an absorbance maximum at 256 nm with $\epsilon$ = 2.4 x $10^4$ $M^{-1}$ $cm^{-1}$.

The structure of PHATUBE is shown below:

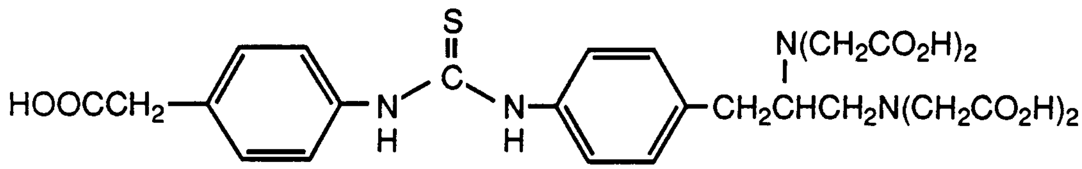

K. Synthesis of Hapten EOTUBE

EOTUBE was prepared exactly as described in Example 1B, except that ethanolamine was used in place of ethylamine, and the final pH of the reaction was 11.0. The product had an HPLC retention time of 3.1 minutes.

The product was purified by anion exchange chromatography on an 11 ml column of DEAE Sephadex A-25, eluted with a 110 ml linear gradient of 0.1 to 1 M ammonium formate, pH 6. Fractions containing the product were pooled and lyophilized. The product had an absorbance maximum at 246 nM with $\epsilon$ = 1.8 x $10^4$ $M^{-1}$ $cm^{-1}$.

The structure was verified by $^{13}C$ NMR spectroscopy. The peak corresponding to the carbon in the isothiocyanate moiety in ITCBE was at 139 ppm, and was replaced by a peak at 182 ppm in EOTUBE. This corresponds to the carbon in the thiourea linkage. The aromatic region (128-138 ppm) of the spectrum of ITCBE shows four peaks, while that of EOTUBE shows 3. In the aliphatic region, there are 5 peaks in common for ITCBE and EOTUBE, and an additional two peaks at 64 and 49 ppm in the EOTUBE spectrum. The latter correspond to the carbons adjacent to the hydroxyl and thiourea moieties, respectively.

The structure of EOTUBE is shown below:

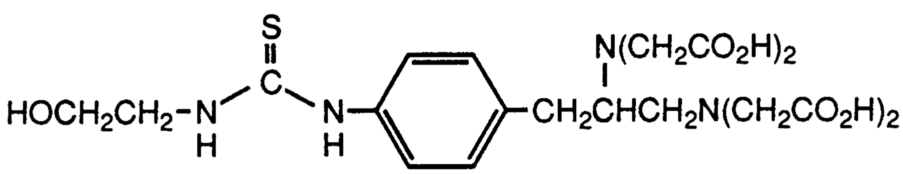

L. Synthesis of Hapten BALTUBE

BALTUBE was prepared as described in Example 1B, except that N-α-acetyl-L-lysine amide was used as the amine, and the reaction was done at pH 11.5.

Purification of BALTUBE was done as described in Example 1B. The product had an absorbance maximum of 244 nm with $\epsilon$ = 2.3 x $10^4$ $M^{-1}$ $cm^{-1}$. The structure was verified by $^{13}C$ and NMR spectroscopy and is shown below:

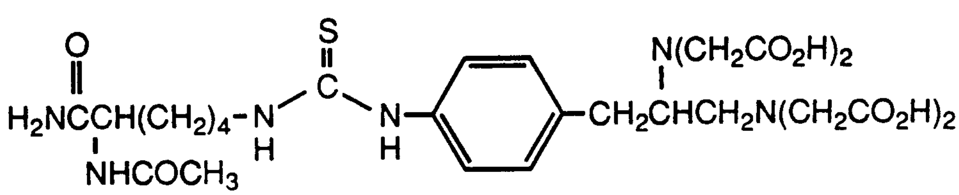

The amide of N-α-acetyl-L-lysine was prepared via the methyl ester using a procedure described by Yeh, S.M. et al. Anal. Biochem. 100, 152-159 (1979). 1 g of N-α-acetyl-L-lysine methyl ester was dissolved in 45 ml of anhydrous methanol and the solution saturated with gaseous $NH_3$. The reaction was kept under $N_2$ and saturated with ammonia twice a day for 2-3 days. The reaction was followed by TLC on silica plates (PolyGram Sil G/UV, Brinkmann Instruments, Inc., Westbury, New York) with a solvent comprised of

ethanol: 25% ammonium hydroxide (4:1, v/v). Spots were visualized by a ninhydrin spray reagent. The methyl ester had an $R_f$ of 0.3. NMR indicated the presence of an additional material which could not be visualized by ninhydrin. Analysis of the NMR indicated that the side product was a seven-membered cyclic amide formed by an intramolecular reaction. Attempts to eliminate the side product by presaturating the methanol prior to the addition of the methyl ester were unsuccessful.

When the reaction was complete it was taken to dryness by rotary evaporation and the solid redissolved in sufficient water to make a 10 mM solution. The amide was purified by chromatography on a 90 ml column of Sephadex CM-25 (Pharmacia Fine Chemicals, Uppsala, Sweden), previously equilibrated with 10 mM trimethylammonium formate, pH 5. Before application, the pH of the sample was adjusted to less than 8 with 10 mM trimethylammonium formate. The cyclic amide side product flowed through the column as the sample was applied and during a wash with 2 column volumes of 10 mM trimethylammonium formate. The desired product then was eluted with a 900 ml linear gradient of trimethylammonium formate, pH 5, from 10 to 500 mM. Fluorescamine was used to test the individual fractions for the presence of amine. Those fractions which were flourescamine positive were pooled and lyophilized. The NMR spectra were consistent with the expected structure for N-acetyl-L-lysine amide. The concentration of amine was determined by assay with trinitrobenzenesulfonic acid (TNBS) (Glazer, A.N. et al., Chemical Modification of Proteins, pp 76-77, American Elsevier, New York, (1975)).

M. Synthesis of Hapten NUBE

Ethylenediamine dihydrochloride (115 mg, 875 µmole) was added to 108 µl (1.624 µmole) ethylenediamine. To the resulting solution was added 2.5 ml of 42.2 mM ITCBE (105 µmole) in 0.3 M HCl with stirring. The pH was adjusted to 9-10 with triethylamine or hydrochloric acid. HPLC analysis showed that the reaction occurred instantaneously. The HPLC retention time of NUBE was 2.67 minutes under the HPLC conditions in Example 1A.

The reaction mixture was diluted to 500 ml with water and applied to a 15 ml DEAE Sephadex A25 chromatography column equilibrated in 20 mM triethylammonium formate, pH 6-7. The column then was eluted with a 500 ml linear gradient from 20 to 250 mM triethylammonium formate. Peak fractions (as determined by absorbance at 250 nm) were pooled and lyophilized. When compared with the $^{13}C$ NMR spectrum of ITCBE, new resonances appeared at 183.0, 44.7, and 41.8 ppm, corresponding to the thiourea, and ethylenediamine methylene carbons, respectively. In addition, one signal near the 140 ppm region of the ITCBE spectrum (assigned to the isothiocyanate carbon) did not appear in the NUBE spectrum.

The structure of NUBE is shown below:

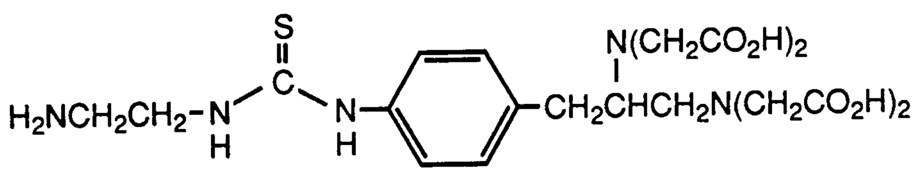

N. Synthesis of Hapten GNUBE

A solution of 16 mg (140 µmole) glutaric anhydride in 100 µl dimethylacetamide was added to a stirred 35 mM solution (800 µl, 28 µmole, pH 8) of NUBE, prepared as described in Example 1M. The reaction was allowed to proceed 20 hours at room temperature, then stopped by dilution into 250 ml 20 mM triethylammonium formate, pH 6.5. The HPLC retention time, under the condition described in Example IA, was 3.19 minutes.

The solution was then applied to a 15 ml DEAE Sephadex A-25 column equilibrated in 20 mM triethylammonium formate, pH 6.5 and eluted with a 500 ml gradient from 200 to 800 mM of the same buffer. The peak fractions, as determined by absorbance at 250 nm, were pooled and lyophilized. When compared with the $^{13}C$ spectrum of NUBE, new resonances appeared at 180.6, 178.7, 37.7, 35.9, and 23.6 ppm, corresponding to the acid, amide, and three methylene carbons from the glutaramate substituent, respectively. In addition, the NUBE resonance appearing at 44.7 ppm shifted to 46.8 ppm in GNUBE, presumably due to acylation of the adjacent amine.

The structure of GNUBE is shown below:

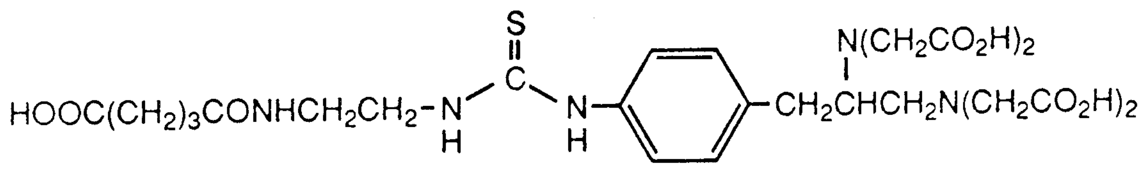

O. Synthesis of Hapten DB-I

p-Bromoacetamidobenyl-EDTA ("BABE") is prepared according to the procedure outlined in U.S. Patent No. 4,622,420, which is incorporated herein by reference. The BABE then was labeled with 10% molar excess of cold indium in 1 M NaOAc, pH 8.0, such that the final pH is about 4. A trace amount of indium-111 was added to follow the labeling procedure. The conditions for the labeling were as follows:

2000 µl 1 M NaOAc, pH 8.0
1089 µl 100 mM $InCl_3$ in 50 mM HCl
120 µl In-111
900 µl BABE (110 mM)

The above reagents were added in the order listed to an acid-washed container containing an acid-washed stirring bar. The pH is adjusted to 4.0 with either 10 N NaOH or 1 N NaOH and the reaction is stirred for 30-60 minutes. An ED3A (ethylenediamine triacetate) column was equilibrated in 50 mM NaOAc, pH 4.0 buffer. A 6ml column was packed and loaded to about 34.4% of the resin capacity with the reaction mixture and the product was eluted. BABE-In with a purity of about 65% was obtained.

p-Bromoacetamidobenzyl-DTPA ("BABD") is prepared analogously to the procedures taught for BABE, described earlier, in U.S. Pat. No. 4,622,420. BABD (47.1 µmoles, 145 mM) was reacted in 23.55 ml of 5 mM 1,4-butanedithiol (plus a 40-fold excess of 1,4-butanedithiol) in 0.1 M $NaHCO_3$, pH 8.2. The reaction was followed by HPLC. After the reaction was complete, the reaction mixture was centrifuged at 3200 RPM for ten minutes and the supernatent was acidified with 23 M formic acid to a final concentration of 0.5 M formic acid. The resulting precipitate was stored at -70° C. The reaction was then extracted with 40 ml portions of diethyl ether until the extracts were DTNB negative. The aqueous layer was lyophilized overnight and stored at -70° C. The yield of the "MBD" obtained was 73.5%

MBD (31.5 µmoles; 19.1 mM in 0.1 M $NaHCO_3$, pH 8.2) was reacted with 63.0 µmoles BABE-In (100 mM in 0.3 M HCl), prepared as indicated above. The final volume was 6.3 ml. The reagents were added as indicated below:

4.025 ml 0.1 M $NaHCO_3$, pH 8.2
1.645 ml MBD with the pH being adjusted as necessary to 8.2 with saturated $Na_2CO_3$.
0.630 ml BABE-In again adjusting the pH to 8.2 with saturated $Na_2CO_3$, as necessary.

The reaction time was about 1 to 2 hours as determined by HPLC. The reaction mixture is applied to a 2 ml chromatography column (BioRad Affi-gel-501 column equilibrated with 0.1 M $NaHCO_3$, pH 8.2). About 30 ml of eluent were collected and the pH of the eluent was adjusted to 6.0 with 23 M formic acid. The eluent then immediately was applied to a 5 ml A-25 column equilibrated in 10 mM ammonium formate, pH 6.0 buffer. The column was washed with 10 mM buffer to make certain that the sample did not elute. A 50 ml gradient from 10 mM to 1.0 M ammonium formate, pH 6.0 was run. The HPLC retention time of the product was 5.08 minutes.

P. Synthesis of Hapten DB-II

DB-II was prepared by reacting DTT (dithiothreitol) with BABD, followed by reaction with BABE-In.

BABD (833 µl, 100 mM, in 0.2 M HCl) was added to 832 µl of DTT (0.5 M in $NaHCO_3$, pH 8). The pH was adjusted to 8.2 and the reaction was complete within 2 hours as determined by HPLC. The reaction was acidified with 23 M formic acid and then was extracted with diethyl ether until DTNB negative. The product was lyophilized overnight then dissolved in 1.0 ml water. The reaction product, designated as MBD-2, then was reacted with BABE-In, prepared in Example 1, Section O. The reaction conditions were as follows:

4440 µl 0.1 M $NaHCO_3$, pH 8.2

900 $\mu$l MBD-2 (33mM)

520 $\mu$l BABE-In (113 mM)

The reaction was complete within 2 hours as determined by HPLC. The reaction mixture was purified on a 5.5 ml BioRad Affi-gel column eluted with .1 M $NaHCO_3$, pH 8.2. The pH of the eluted material was adjusted to pH 6 and further purified on a 5 ml A-25 column with a 100 ml gradient from 10 mM to 1M ammonium formate, pH 6 buffer. The product was obtained in about 78% purity as determined on HPLC and had a retention time of 4.1 minutes.

Q. Synthesis of Hapten DB-III

DB-III is prepared by reacting p-aminobenzyl EDTA ("ABE") with p-isothiocyanatobenyl-DTPA ("ITCBD"), both starting materials prepared according to the teaching of U.S. Patent No. 4,622,420.

ABE was labeled with 20% molar excess indium in 0.1 M NaOAc, pH 6, solution. The reagents were added as follows:

2310 $\mu$l 0.1 M NaOAc, pH 6

1410 $\mu$l 100 mM $InCl_3$

900 $\mu$l 129.6 mM ABE

The pH was adjusted to 6 with 10 N NaOH and the reaction continued for 1 hour. The indium-labeled ABE then was passed through a 28 ml ED3A (ethylenediaminetriacetate) column and eluted with 10 mM NaOAc, pH 6 buffer. The mixture was lyophilized overnight and re-dissolved in 1 ml of milli-Q water. The pH must be greater than or equal to 6 throughout the procedure to prevent scrambling of the indium species.

ITCBD (1.2 ml, 51 mM) was lyophilized prior to the addition of indium-labeled ABE. The addition was made as follows: $NaHCO_3$ (1.5 ml, 0.1 M, pH 8.2) was added to 1 ml of 70.3 mM ABE-In prepared above. The pH was adjusted to 8 with saturated $Na_2CO_3$ and then the solution was added to the lyophilized ITCBD. The mixture was reacted at 40° C. and the reaction was complete after 2 hours as determined by HPLC. The product was obtained at 78.4% purity and had an HPLC retention time of about 3.04 minutes. The pH of the sample was adjusted to 6 with formic acid, diluted to 20 mls with milli-Q water and purified through a 20 ml A-25 column equilibrated with 10 mM ammonium formate, pH 6, buffer. A 200 ml gradient of 10 mM to 1 M ammonium formate, pH 6, buffer was used to elute the product. The extinction coefficient at 260 nm in water, pH 7 was about 20,380. Radiolabeling the product, using In-111 and Y-90, followed by radio-HPLC, showed 100% and 98% of the activity associated with the DB-III for the In-111 and Y-90 labeled compounds, respectively. The $^{90}Y^{3+}$ labeled compound showed the characteristic doublet associated with $^{90}Y^{3+}$ labeled DTPA chelates in this HPLC system. The labeled DB-III, 96% and 97%, respectively, for the $^{111}In^{3+}$ and $^{90}Y^{3+}$ labeled species, bound to antibody CHA255.

The $^{1}H$ and $^{13}C$ NMR of DB-III were determined as follows: Samples were freshly prepared using distilled $D_2O$ as the solvent in acid-washed NMR tubes. The pH was adjusted to 6.5 as necessary with NaOD or DC1. The pH reported is the actual pH meter reading without correction for the activity of $D_2O$. p-Dioxane was used as an internal standard for the $^{13}C$ NMR spectrum (downfield shift of 66.5 ppm) and the HDO peak was used as a reference for the proton spectrum (4.70 ppm). All spectra were recorded on a Varian Model XL-300 NMR spectrometer.

The following show the results of the $^{13}C$ spectrum in ppm ($\delta$)(not all peaks are given): 179.5, 177.6, 176.0, 175.8, 175.5, 170.9, 170.3, 170.2, 135.3, 134.4, 130.2 (CH), 130.1 (CH), 126.8 (CH), 126.7 (CH), 62.5 (CH), 61.0 (CH), 59.7 ($CH_2$), 57.7 ($CH_2$), 57.2 ($CH_2$), 56.7 ($CH_2$), 55.4 ($CH_2$), 53.9 ($CH_2$), 53.5 ($CH_2$), 53.1 ($CH_2$), 51.5 ($CH_2$), 48.5 ($CH_2$), 32.3 ($CH_2$), 31.9 ($CH_2$).

DB-III was labeled with $^{90}Y$ as follows:

10 $\mu$l .22 M glycine, pH 8.2

1 $\mu$l .1 mM DB-III

5 $\mu$l $^{90}Y$ (180 $\mu$Ci/$\mu$l)

The noted mixture was incubated for 30 minutes at room temperature.

R. Synthesis of Hapten DB-IV

DB-IV is prepared by reacting NUBE with ITCBD in a manner analogous to that previously described for the preparation of the other DB-haptens.

The volumes of NUBE and ITCBD used were lyophilized prior to mixing. $Na_2CO_3$ (50 $\mu$l), pH 11-12 was added to NUBE followed by addition to ITCBD. The reaction was performed at 40° C. The quantities used were as follows:

25 $\mu$l ITCBD (51 mM)

125 µl NUBE (20 mM) with 100% excess NUBE added

50 µl final volume of $Na_2CO_3$ pH 11-12

The reaction was monitored by HPLC and was complete within about one-half hour. The product had a retention time of about 3.42 minutes.

S. Synthesis of Hapten DB-V

ITCBE was reacted with butyldiamine to prepare BUBE. The BUBE then was loaded with cold indium and subsequently reacted with ITCBD to prepare DB-V.

In particular, 80 µl butyldiamine (BUDA, 800 µmoles) was mixed with 300 µl of 3 N HCl so that the pH was about 10.5. ITCBE (1 ml, 39 mM) was added dropwise with stirring to the mixture. An additional 10 µl of BUDA was added to keep the pH at 10.5. When the reaction was complete as indicated by HPLC, the reaction mixture was diluted with milli-Q water and the pH was adjusted to 3 with .3 N HCl. The product was purified on column loaded with AG-1X8 resin. BUDA was eluted with 50 mM formic acid until the eluent tested negative with fluorescamine. The product then was eluted with 1 N formic acid. Those fractions containing the product were combined and lyophilized.

The BUBE prepared above then was labeled with cold indium. Excess indium was removed using an ethylenediaminetriacetate columm. ITCBD (0.5 ml, 39 mM), prepared as indicated earlier, was lyophilized and then re-dissolved in 1 ml of 0.1 M $NaHCO_3$. The pH was adjusted to 7 with 1 N NaOH. The In-BUBE (28 µmoles) prepared above was dissolved in 0.5 ml of 0.5 ml of 0.1 M $NaHCO_3$, pH 8. The two solutions were mixed and the pH was adjusted to 10.5 with 1 N NaOH. The reaction was complete in about 3 hours as indicated by HPLC.

The reaction mixture was purified on an A-25 column using a 200 ml gradient running from 10 mM to 1 M ammonium formate, pH 6 buffer. Fractions containing the desired product were combined, lyophilized and then dissolved in 1 ml 100 mM ammonium formate, pH 6, and characterized. The product obtained had an HPLC retention time of 3.83 minutes with a purity of about 88.6%. This product showed an absorbance maximum at 246 nm with an extinction coefficient of about 26,700.

T. Synthesis of Hapten DB-VI

p-aminobenzyl-DTPA ("ABD") was prepared in a manner analogous to the preparation of ABE described above, using the teaching of U.S. Patent No. 4,622,420. BABE, prepared as indicated earlier, was indium-labeled as follows: Indium chloride (30.6 mM in 1.2 N HCl, 2.0 ml) was added to a solution of BABE (144 mM, 400 µl) and the pH was adjusted to about 6.0 with 10 N and 1 N NaOH. The mixture was incubated for 30 minutes and then purified over an ethylenediaminetriacetate column. The product was eluted with 10 mM sodium acetate. Column fractions containing the desired product were combined and lyophilized.

The indium-loaded BABE (20 µmoles in 1 ml $H_2O$) was added to a solution of ABD (20 µmoles) in $H_2O$ (0.5 ml). The pH was adjusted to about 5.6 and the reaction was incubated at 37° C. overnight. When HPLC analysis indicated that the reaction was complete, the reaction mixture was purified on an A-25 column using a gradient running from 0.1 to 1.0 M triethylammonium formate, pH 8.7, to elute the material. Fractions containing the desired product were combined and lyophilized. The DB-VI obtained had an HPLC retention time of about 3.3 minutes. The $^{13}C$ NMR spectrum showed the following chemical shifts in ppm (δ) (dioxane as the internal standard): 178.8, 178.6, 178.3, 177.7, 175.6, 173.1, 173.0, 172.1, 149.4, 138.1, 136.5, 133.1 (CH), 132.7 (CH), 127.0, 125.6 (CH), 116.8 (CH), 65.5 (CH), 64.0 (CH), 62.5 ($CH_2$), 60.6 ($CH_2$), 60.2 ($CH_2$), 59.6 ($CH_2$), 56.8 ($CH_2$), 56.5 ($CH_2$), 56.0 ($CH_2$), 55.7 ($CH_2$), 54.4 ($CH_2$), 51.3 ($CH_2$), 50.7 ($CH_2$), 35.0 ($CH_2$), 34.2 ($CH_2$). The proton NMR (HOD as an internal standard) showed resonances (δ) at 7.31 (d, 1H, J= 8.4 Hz), 7.19 (d, 1H, J=8.4 Hz), 7.08 (d, 1H, J=8.4 Hz), 6.7 (d, 1H, J=8.4 Hz), 3.94 (s, 2H), 2.70 (m, 2H), 2.55 (m, 1H).

U. Synthesis of Hapten DB-VIII

DB-VIII is prepared by reacting p-bromoacetamidobenzyl DTPA ("BABD") with indium-loaded DHPBE which is prepared from BABE and 1,3-diamino-2-hydroxypropane. The BABD is prepared in a manner analogous to that previously taught for the preparation of BABE and generally follows the teaching of DeReimer, L. H., et al., J. Lab. Comp. and Radiopharm, 18, 1517 (1981) and U.S. Patent No. 4,622,420.

In particular, 1,3-diamino-2-hydroxypropane (28 mg) was dissolved in 50 mM HEPES buffer, pH 10 (1 ml) and BABE (24 mM, 1 ml), prepared as noted above, was added dropwise. The reaction was stirred

overnight at room temperature until HPLC indicated that the reaction was complete. The reaction mixture was diluted to 60 ml and applied to an A-25 column (10 ml) which was equilibrated with 100 mM triethylammonium formate, pH 8.7. The column was eluted with a 400 ml gradient running from 100 mM to 1.0 M triethylammonium formate, pH 8.7. The fractions were analyzed with HPLC and those fractions containing the product were combined and lyophilized. The product was dissolved in water (1 ml, 24 µmoles) and indium chloride (30.6 mM, 400 µl) was added. The pH was raised to about 5.8 and the solution was incubated for 30 minutes. Because the mixture showed that the product and starting material had the same retention times, excess indium chloride (600 µl) was added. The pH was adjusted to about 6.0 and the material was applied to an ethylenediaminetriacetate column. The column was eluted with 10 mM sodium acetate and the fractions containing the product were combined and lyophilized.

The indium loaded DHPBE (20 µmoles) obtained was dissolved in water (1 ml) and added dropwise to BABD (40 µmoles). The pH was adjusted to about 9.0 and the reaction was stirred at 37° C. for about six days. HPLC indicated that several products were formed. After repeated purifications over A-25 and BioRad Affi-gel-102 columns, a single product, designated DB-VIII was obtained. This product had an HPLC retention time of 3.2 minutes.

## EXAMPLE 2

## MONOCLONAL ANTIBODY PREPARATION

### A. Preparation of Monoclonal Antibody CHA255

Antibody producing hybridoma cell lines were prepared as follows. Spleen cells from BALB/c mice multiply immunized with the antigen were fused with a variant of the P3.653 myeloma cell line. See Gerhard, Monoclonal Antibodies, edited by Kenneth et al., Plenum Press, New York (1980). The resulting hybridomas were screened by a solid phase second antibody radioimmunoassay for their ability to bind indium-aminobenzyl-EDTA (Wang et al., Journal of Immunological Methods, 18, 157, (1977)). Based on their high titers and relatively high affinity as determined by inhibition of binding by unlabeled antigen, a monoclonal antibody designated as CHA255 was chosen for further study and injected intra-peritoneally into BALB/c mice for ascites production. The monoclonal antibodies were purified from mouse ascites by ion-exchange chromatography on DEAE-cellulose as described by Parham et al., J. Immunol. Meth., 53, 133 (1982). Monoclonal antibody CHA255 is further described by Reardon, D.T. et al., Nature 316, 265-268 (1985).

### B. Preparation of Bifunctional Antibody ECH037.2

Bifunctional antibody ECH037.2 was prepared by the formation of a tetradoma by the biological fusion of the hybridoma cell line expressing monoclonal antibody CHA255, prepared as described in Example 2A, and a hybridoma cell line, designated CEM231.6.7, expressing antibody having specificity for carcinoembryonic antigen (CEA). Hybridoma cell line 231.6.7 was deposited with the American Type Culture Collection under the Budapest Treaty on January 7, 1988 (ATCC Accession No. HB 9620). Thus, the bifunctional antibody produced has specificity for both indium-benzyl-EDTA and CEA. The tetradoma which expresses the bifunctional antibody designated as ECH037.2 was prepared by biological fusion of the hybridoma cell lines CHA255 and CEM231.6.7 as described by Burnett, K.G. et al. in Biotechnology: Applications and Research, pp. 401-409, edited by Cheremisinoff and Ouellette, Technomic Publishing Company, Pennsylvania (1985). ECH037.2 was purified from culture fluid in a two step process. IgG molecules were first isolated from other components of the culture medium by chromatography on DEAE-cellulose, as described by Parham et al. J. Immunol. Meth., 53, 133 (1982), followed by further purification by HPLC on hydroxylapatite (BioRad Laboratories, Richmond, Ca). The ECH037.2 bifunctional antibody was eluted using a gradient from 10 mM to 160 mM sodium phosphate, pH 6.8. Both buffers also contained 10 mM $Ca^{2+}$ to prevent column degradation. The bifunctional antibody ECH037.2 was identified by the pattern of protein bands generated with electrophoresis in 7.5% acrylamide gels (SDS), and the ability of the antibody to bind both indium-benzyl EDTA chelates and CEA.

### Hapten capacity assay:

To determine the ability of the antibody to bind indium-benzyl-EDTA, the antibody and the $In^{3+}$ chelate of (S)-4-p-nitrobenzyl EDTA were combined in PBS (1O mM sodium phosphate, 150 mM NaCl, pH 7.5)

containing 1.5 mg/ml normal serum albumin. Antibody concentration was 0.15 mg/ml and the Indium-(S)-4-p-nitrobenzyl EDTA concentration was 3 $\mu$M. The chelate was radiolabeled at a specific activity of 0.5-3.0 $\mu$Ci/nmol with $^{111}$In. After a 5 minute incubation at room temperature, duplicate 400 $\mu$l aliquots of the mixture were transferred to separate Centrifree™ ultrafiltration devices (NMW cutoff = 30,000; available from Amicon, Danvers, Massachusetts). These were centrifuged at 1500 g for 10 minutes. Duplicate 50 $\mu$l pre-Centrifree samples were counted and averaged. This value was called "TC". Duplicate 50 $\mu$l aliquots of the Centrifree filtrates were counted and averaged. This value was called "F". The percent of theoretical capacity was calculated from the following equation:

$$(1 - F/TC) \times M$$

M was assigned a value of 150, 100, 200 or 300 for intact antibody (CHA255), Fab fragment, F(ab)'$_2$ bifunctional antibody and intact bifunctional antibody (ECH037.2), respectively.

To test the ability of the antibody to bind CEA, an immunoreactivity assay was designed to determine the percentage of the radiolabeled anti-CEA antibody preparation which bound to CEA. This value was determined by a two site immunoassay employing solid phase CEA bound to a polystyrene bead by a second antibody reactive with CEA, essentially as described in U.S. 4,376,110. Antigen positive and negative beads were prepared by incubating CEA beads, obtained from the commercially available TANDEM-R CEA test kit (Hybritech Incorporated, San Diego, California) in high CEA and zero calibrators. After unbound CEA antigen was washed off the beads, approximately 1 ng of the antibody radiolabeled with $^{125}$I to a specific activity of 10 $\mu$Ci/$\mu$g was added. The percent immunoreactivity was determined by calculating the percent of counts bound to the bead in the presence of antigen. A non-specific binding control was determined by the percent of counts bound to beads that have no CEA antigen present.

Antibody CHA255 Binding Assay

As indicated, the monoclonal antibody CHA255 is specific for the $In^{3+}$-benzyl EDTA complex. The affinity and characterization have been described previously in Nature, 316, 265 (1985). Binding assays were performed to verify the identity of the haptens designated as DB-I to DB-VIII. Under the conditions of the assay, neither $^{90}Y^{3+}$ or $^{111}In^{3+}$ DTPA, alone, bound to CHA255. For the $^{90}Y^{3+}$ hapten chelate, in particular, CHA255 binding required the presence of the In-benzyl-EDTA moiety as provided in the compounds of the invention. The labeled DB hapten chelates were combined with antibody CHA255 in PBS (10 mM sodium phosphate, 150 mM sodium chloride, pH 7.4) containing 44 $\mu$g/ml of human serum albumin and 0.05% Triton-X-100 detergent. Concentrations of the radiolabeled hapten chelating agent and antibody are 5 nM and 133 nM, respectively. The mixture is vortexed and allowed to stand at room temperature for 15 minutes, after which it is transferred to an Amicon Centrifree™ device, with a NMW limit of 30,000, and centrifuged to separate antibody-bound chelate from free hapten chelate. The species bound to the antibody is retained by the membrane and the free chelate flows through the membrane into the filtrate. The extent of antibody-hapten chelate binding is then determined.

EXAMPLE 3

## IN VITRO CHARACTERIZATION OF DERIVATIZED HAPTENS

A. Determination of Hapten-Antibody Dissociation Rates

Hapten-antibody dissociation rates were determined using monoclonal antibody CHA255, prepared as described in Example 2A, by measuring the quantity of $^{111}In^{3+}$ chelate released from the antibody hapten complex as a function of time after addition of a 100 fold excess (over antibody binding sites) of indium-115 loaded hapten. Thus, for each hapten, a 700 $\mu$l solution was prepared containing: antibody at a concentration of 160 nM; indium loaded hapten, trace labeled with $^{111}$In (total concentration = 160 nM); normal human serum albumin at a total concentration of 1.5 mg/mL; and isotonic phosphate buffered saline, (10 mM $PO_4^{2-}$, 150 mM NaCl, pH 7.2). Indium-115 loaded hapten was added to a final Concentration of 32 $\mu$M. Reactions were stirred by placing the sample containers ir a rack mounted on a vortexer head.

At various times after addition of the excess hapten, 400 µL portions of the reaction solutions were transferred to Centrifree tubes (Amicon Corperation, Danvers, Massachusetts) and centrifuged at 1500xg for eight to ten minutes. The reaction time was taken to be the time between addition of the excess hapten and the time the centrifuge reached the desired velocity. Six timepoints, each in duplicate, were obtained over a range of about two reaction half-lives for each rate constant determination. A 200 µl portion from each centrifuged and uncentrifuged solution was then counted for $^{111}In$. The relative concentration of free hapten was taken to be the ratio of counts in the centrifuged solution to the uncentrifuged solution at each timepoint. Times were recorded to the nearest tenth of a minute.

Room temperature rate constants were measured without the aid of a constant temperature bath. The ambient temperature remained at 22°±1 C.

Rate constants and standard deviations were obtained from a plot of the logarithm of relative complex concentration vs. time. Data were analyzed using the linear regression module of the RS/1 Program (BBN Software Products Corporation, Cambridge, Massachusetts).

Control samples were included in each experiment to determine: A) that the initial condition of 100% of labeled hapten bound to antibody was met, and B) that in the absence of antibody, 100% of counts were able to penetrate the Centrifree membrane. If control A showed that a significant portion of counts did not bind antibody initially, the average number of counts in the control duplicates was subtracted from all centrifuged and uncentrifuged sample solutions. If control B showed that not all counts penetrated the membrane, each count ratio (i.e. the relative concentration of free hapten) was divided by the average of the fraction penetrating the membrane in the control duplicates. The antibody-hapten dissociation rates, expressed as reaction half-lives ($t_{1/2}$ minutes) are shown in Table III.

TABLE III

| 22°C Dissociation Half-Lives of Selected Haptens | | | |
|---|---|---|---|
| Hapten Abbreviation | $^{t}1/2$(min) | S.D.* | -r** |
| GNUBE | 98.8 | 1.2 | 0.999 |
| BETUBE | 94.2 | 3.0 | 0.995 |
| BALTUBE | 90.9 | 1.2 | 0.999 |
| BUTUBE | 90.6 | 2.0 | 0.998 |
| PATUBE | 87.8 | 2.6 | 0.996 |
| TUBE | 84.2 | 2.5 | 0.996 |
| PHTUBE | 75.0 | 0.8 | 0 999 |
| BATUBE | 74.3 | 1.8 | 0.997 |
| ETUBE | 72.6 | 1.3 | 0 998 |
| PHATUBE | 71.5 | 0.8 | 0.999 |
| EOTUBE | 59 7 | 1.5 | 0.997 |
| NUBE | 45.6 | 0.8 | 0.998 |

* standard deviation
** correlation coefficient

As shown in Table III, haptens of the invention dissociate from the antibody at rates which vary over a wide range. Thus, a hapten may be selected or designed to dissociate slowly from the antibody for applications such as therapy in which total dose to the target is the most important consideration. Conversely, haptens with shorter half-lives may be more advantageous for applications such as imaging, in which signal (at target) to noise (for example, blood pool) is the most important factor, or in protocols which require transfer of haptens between antibodies.

EXAMPLE 4

# IN VIVO CHARACTERIZATION OF DERIVATIZED HAPTENS

A. Determination of Hapten Biodistributions

Two protocols were used for examining the biodistribution of the derivatized haptens in the presence of the bifunctional antibody ECH037.2. In a premix protocol, ECH037.2 and the derivatized hapten were combined prior to injection in the animals. In the prelocalization protocol, ECH037.2 was injected into the animals, and the antibody was allowed to prelocalize for 24 hours prior to injection of the radiolabeled hapten.

Biodistribution studies were performed in nude mice carrying subcutaneous implants of the human colon carcinoma cell line T-380 which expresses the carcinoembryonic antigen (CEA) recognized by ECH037.2. Briefly, Balb/C Nu/Nu mice were injected subcutaneously with a minced preparation of T-380 tumor implants and maintained for approximately two weeks, or until the tumor implant had grown to a size of approximately 0.5 grams as described by Hagan, P.L., et al. J. Nuc. Med., 26 1418-1423 (1985). Mice were kept in a sterile environment to prevent complications due to bacterial or viral infections. Biodistributions were performed as described by Halpern, S., et al., Cancer Res. 43, 5347-5355 (1983). Briefly, the radiolabeled haptens were injected intravenously in normal mice, and the pharmacokinetics were evaluated at several time points post-injection. At these times, mice were sacrificed and weighed. Organs were removed, their weight recorded, and each organ was then washed in sterile PBS, pH 7.0, and fixed in formalin solution. Samples of blood, urine and feces also were collected and their weight similarly recorded. The organs, or a portion thereof, were transferred to clean tubes and their radioactive content was determined in a well-type gamma-counter.

Relative radioactive content of each organ was expressed as a percentage of injected dose per organ.

B. Preparation of Samples for Biodistribution--Premix Protocol

To indium-label the haptens of the invention 10 $\mu$l of 0.1 mM ligand was combined with X $\mu$l of $^{111}InCl_3$ (in 50 mM HCl) and (x+10) $\mu$l of 0.26 M ammonium citrate, pH 6.0. X is calculated by the following equation:

$$X = (200\ \mu Ci)/(A\ \mu Ci/\mu l)$$

where A is the specific activity of the $^{111}In$.

The labeling was done in an acid-washed microfuge tube. After combining all of the components above, the solution was vortexed and allowed to stand at room temperature for 15 minutes.

Incorporation of the $^{111}In$ into the chelating agent was determined by TLC on silica in a methanol/aqueous ammonium acetate solvent system as described by Meares, et al. (Anal. Biochem., 142, 68-75 (1984)). Greater than 95% incorporation of the radioactivity into the chelate was required for the sample to be submitted for a biodistribution assay.

Prior to injection the $^{111}In$-labeled hapten was diluted into freshly prepared isotonic sodium bicarbonate (1.39% w/v) containing 1 mg/ml normal human serum albumin (Buminate, 25% solution, Travenol Laboratories, Glendale, California) to which the bifunctional antibody had been added. The final composition of the solution was 0.2 mg/ml ECH037.2, 0.5 $\mu$M chelate, 0.1 $\mu$Ci/$\mu$l $^{111}In$, all in a volume of 2.0 ml. For the biodistribution studies, 100 $\mu$l of the solution was injected into the tail vein of each animal.

Tables IV and V below summarize the results of the biodistribution (expressed as percentage of injected dose/gram of organ) of the different haptens as determined using the premix protocol described above.

TABLE IV

| COMPARISON OF VARIATIONS OF HAPTENS USED IN A PREMIX PROTOCOL WITH 20 μg ECH037.2 IN NUDE MICE BEARING T-380 TUMORS | | | | | | | | |
|---|---|---|---|---|---|---|---|---|
| HAPTEN | % DOSE PER GRAM AT 48 HOURS | | | | | | | |
| | BLOOD | KIDNEY | LIVER | TUMOR | URINE* | FECES* | TOTAL RECOV | TUMOR/BLOOD |
| ETUBE | 5.9 | 1.2 | 2.4 | 10.4 | 36.0 | 15.2 | 66.6 | 1.76 |
| BETUBE | 5.5 | 1.5 | 1.9 | 7.5 | 12.0 | 29.0 | 54.2 | 1.36 |
| PATUBE | 7.6 | 1.7 | 2.4 | 9.8 | 36.0 | 8.9 | 61.7 | 1.29 |
| BATUBE | 7.9 | 1.5 | 2.0 | 12.1 | 30.4 | 21.1 | 68.3 | 1.53 |
| BUTUBE | 5.8 | 1.2 | 3.8 | 5.7 | 27.0 | 26.5 | 69.3 | 0.98 |
| OTUBE | 0.5 | 0.2 | 0.6 | 0.7 | 10.9 | 68.4 | 81.4 | 1.40 |
| TUBE | 3.8 | 1.1 | 1.6 | 6.5 | 48.8 | 9.8 | 69.5 | 1.71 |
| EOTUBE | 4.7 | 1.4 | 3.1 | 14.5 | 49.3 | 7.9 | 72.3 | 3.09 |
| BALTUBE | 5.2 | 1.9 | 2.2 | 12.9 | 26.5 | 13.8 | 57.8 | 2.48 |
| PHTUBE | 5.4 | 1.7 | 2.3 | 10.6 | 24.5 | 21.7 | 62.4 | 1.96 |
| PHATUBE | 3.7 | 0.8 | 1.1 | 6.9 | 22.4 | 34.9 | 66.4 | 1.86 |
| NUBE | 2.0 | 2.5 | 1.9 | 8.1 | 52.6 | 12.9 | 74.1 | 4.0 |
| GNUBE | 8.0 | 1.5 | 2.4 | 12.0 | 26.9 | 17.8 | 63.1 | 1.49 |

* Urine and Feces are percent of total injected dose

TABLE V

| COMPARISON OF VARIATIONS OF HAPTENS USED IN A PREMIX PROTOCOL WITH 20 μg ECH037.2 IN NUDE MICE BEARING T-380 TUMORS | | | | | | | | |
|---|---|---|---|---|---|---|---|---|
| HAPTEN | % DOSE PER GRAM AT 24 HOURS | | | | | | | |
| | BLOOD | KIDNEY | LIVER | TUMOR | URINE* | FECES* | TOTAL RECOV | TUMOR/BLOOD |
| ETUBE | 10.4 | 2.4 | 3.5 | 8.0 | 25.8 | 5.9 | 52.2 | 0.77 |
| BETUBE | 9.0 | 2.5 | 2.7 | 8.0 | 8.4 | 17.4 | 44.6 | 0.89 |
| PATUBE | 12.2 | 2.2 | 3.3 | 10.6 | 23.6 | 5.0 | 53.7 | 0.87 |
| BATUBE | 11.8 | 2.5 | 3.3 | 11.5 | 20.0 | 6.9 | 50.8 | 0.97 |
| BUTUBE | 9.0 | 1.7 | 3.4 | 5.3 | 22.0 | 16.8 | 63.8 | 0.59 |
| OTUBE | 1.1 | 0.5 | 0.8 | 1.1 | 8.9 | 56.5 | 68.9 | 1.00 |
| TUBE | 8.0 | 2.4 | 4.0 | 8.5 | 23.7 | 4.1 | 48.2 | 1.06 |
| EOTUBE | 9.3 | 2.8 | 3.8 | 12.5 | 25.9 | 6.2 | 55.6 | 1.34 |
| BALTUBE | 11.0 | 3.6 | 4.0 | 13.0 | 16.3 | 6.8 | 52.0 | 1.18 |
| PHTUBE | 8.9 | 2.9 | 3.6 | 12.4 | 20.3 | 13.9 | 57.2 | 1.39 |
| PHATUBE | 6.2 | 1.2 | 1.7 | 8.4 | 21.7 | 22.0 | 59.7 | 1.35 |
| NUBE | 5.9 | 4.0 | 3.1 | 8.5 | 42.5 | 5.9 | 66.3 | 1.44 |
| GNUBE | 13.0 | 2.7 | 3.4 | 9.0 | 14.2 | 10.6 | 52.0 | 0.70 |

* Urine and Feces are percent of total injected dose

Figure 1 shows the absolute uptake in the tumor and Figure 2 shows the variation in the tumor/blood ratio for the different haptens. In the figures, the designations "e", "be", "pa", "ba", "bu", "o", "eo", "bal", "ph", and "pha" refer to ETUBE, BETUBE, PATUBE, BATUBE, BUTUBE, OTUBE, EOTUBE, BALTUBE, PHTUBE, and PHATUBE, respectively. As shown in Tables IV and V and Figures 1 and 2, the biodistribution obtained is dependent upon the identity of the hapten. For example, if the simplest hapten TUBE is compared to those derivatives with increasing aliphatic chain length, (ETUBE, BUTUBE and OTUBE), some trends in physiologic behavior may be observed. The biodistribution of ETUBE is very similar to that of

TUBE, but as chain length increases (and hydrophobicity increases as shown by the HPLC data determined in Example 1 and shown in Table VI below), the amount of hapten in the tumor diminishes (e.g., 8.0, 5.3, and 1.1 percent injected dose/gram at 24 hrs. and 10.4, 5.7 and 0.7 percent injected dose/gram at 48 hrs. for ETUBE, BUTUBE and OTUBE, respectively). For the same series of compounds a dramatic increase is seen in the amount of material excreted through the gut and into the feces. For example, at 48 hrs. post injection, 15.2, 26.5 and 68.4 percent of the injected material was excreted in the feces for ETUBE, BUTUBE and OTUBE, respectively. In general, as the lipophilicity (as indicated by HPLC retention time) of the compounds increased, so did the amount of material excreted through the intestines and out as feces in the premix protocol. This is shown in Table VI, below.

TABLE VI

| Compound | HPLC Retention Time | Percent Injected Dose in Feces | |
|---|---|---|---|
| | | 24 hrs. | 48 hrs. |
| NUBE | 2.6 min | 5.9 | 12.9 |
| TUBE | 2.8 | 4.1 | 9.9 |
| PATUBE | 3.0 | 5.0 | 8.9 |
| BATUBE | 3.1 | 6.9 | 21.1 |
| EOTUBE | 3.1 | 6.2 | 7.9 |
| GNUBE | 3.2 | 10.6 | 17.8 |
| BALTUBE | 3.5 | 8.5 | 13.8 |
| PHATUBE | 3.5 | 22.0 | 34.9 |
| ETUBE | 3.6 | 6.7 | 15.2 |
| PHTUBE | 4.5 | 13.9 | 27.1 |
| BUTUBE | 4.8 | 16.8 | 26.5 |
| BETUBE | 5.1 | 17.4 | 29.0 |
| OTUBE | 7.4 | 56.5 | 68.4 |

As can be seen from Table VI, those compounds with HPLC retention times greater than 4 minutes were judged to be undesirable as potential imaging agents because of the difficulty which may be encountered in trying to image haptens in the abdominal cavity due to the high levels observed in the intestines. It is also interesting to compare the compounds ETUBE, PATUBE, EOTUBE and NUBE in which the terminal carbon has a carboxyl, a hydroxyl or an amino group within this series. The compound EOTUBE stands out with its outstanding tumor uptake (Figure 1) and good tumor/blood ratios (Figure 2).

C. Preparation of Samples for Biodistribution Prelocalization Protocol

To indium-label the chelating agents for the prelocalization protocol, 5 µl of 0.1 mM chelating agent was combined with X µl of $^{111}InCl_3$ (in 50 mM HCl) and (x + 5) µl of 0.26 M ammonium citrate, pH 6.0. X was calculated by the following equation:

X = 200 µCi/A µCi/µl

where A is the specific activity of the $^{111}InCl_3$ solution. The solution was vortexed and allowed to stand at room temperature for 15 minutes, after which incorporation of $^{111}In$ label into the chelating agent was determined as described for the premix protocol. If at least 95 percent of the $^{111}In$ was bound by the chelating agent, the material was then diluted to a final volume of 2 ml with isotonic (1.39 percent, w/v) sodium bicarbonate containing 1 mg/ml normal human serum albumin. This material (100 µl) then was injected into each tail vein of mice which had each been injected 24 hours earlier with 5 µg of ECH037.2 in 100 µl isotonic sodium bicarbonate containing 1 mg/ml normal human serum albumin.

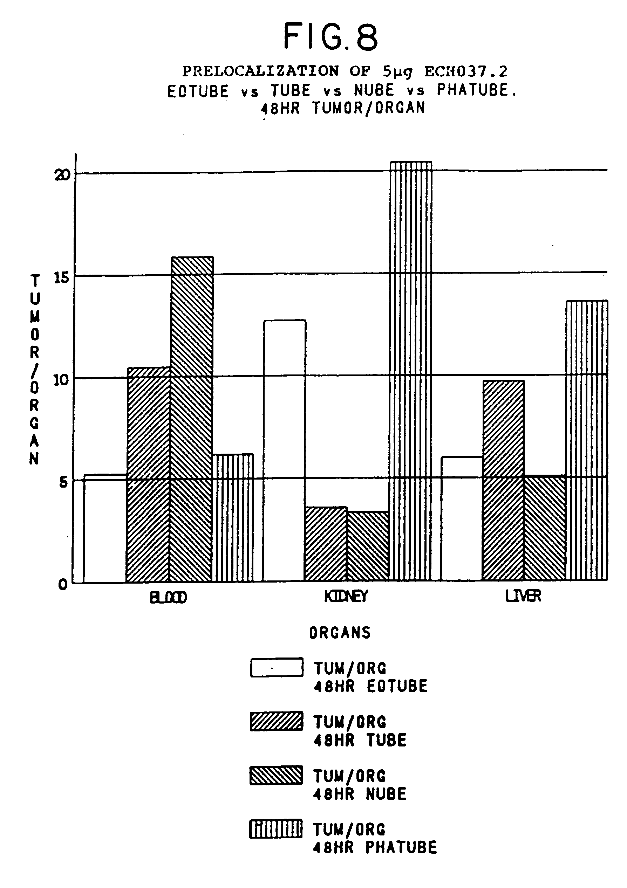

Four of the haptens, TUBE, EOTUBE, NUBE and PHATUBE were further subjected to the prelocalization biodistribution protocol as described above. These results are summarized in Figures 3, 4 and 5, and the tumor/organ ratios are shown in Figures 6-8.

As can be seen in Figures 3-5, PHATUBE is characterized by the high degree of fecal excretion, when compared to the other compounds, which agrees with the results seen with the premix protocol. Thus, although the tumor/organ values found for this hapten are exceptional (Figures 6-8), the high degree of fecal

excretion for all time points suggests that this hapten would not be ideal as an imaging agent.

The biodistribution of EOTUBE is characterized by high tumor uptake at all time points (Figures 3-5) and high tumor/organ ratios at 24 hours (Figure 7). For this reason, this hapten was selected as the optimal imaging agent from the series of four compounds examined using this protocol.

Table VII shows the average tissue localization properties of $^{111}$-labeled DB-I, DB-II, and DB-III when combined with 20 µg of ECH037.2 and injected into nude mice carrying T-380 tumor implants. Each entry (%Dose per gram) for 24 hours represents the average of 5 replicates for DB-I and DB-II and the data for DB-I, DB-II, and DB-III at 48 hours is the average of 6 replicates . The standard deviations are shown for each value.

Table VII
Tissue Localization of DB-I and DB-II
%Dose per Gram

24 Hours Post-Injection

| Hapten | Blood | Kidney | Liver | Tumor | Urine* | Feces* |
|---|---|---|---|---|---|---|
| DB-I | 10.1±1.6 | 2.4±.57 | 3.6±1.3 | 7.7±.8 | 25.4 | 4.1 |
| DB-II | 8.03±2.7 | 2.6±.70 | 3.6±1.3 | 5.6±1.3 | 40.9 | 2.9 |
| DB-III | 12.2±3.0 | 3.4±.94 | 3.3±.74 | 15.6±2.4 | 19.0 | 4.0 |

48 Hours Post-Injection

| Hapten | Blood | Kidney | Liver | Tumor | Urine* | Feces* |
|---|---|---|---|---|---|---|
| DB-I | 5.9±1.6 | 2.8±1.4 | 3.9±1.5 | 8.2±1.85 | 30.0 | 7.9 |
| DB-II | 4.4±1.9 | 1.6±.61 | 2.58±1.7 | 6.9±2.9 | 50.1 | 4.89 |
| DB-III | 8.2±1.1 | 2.7±.36 | 3.2±.92 | 17.5±3.1 | 32.6 | 5.2 |

* Urine/Feces Data is %dose/organ

Table VIII shows summaries of tissue localization properties of $^{111}$In labeled-DB-III, NUBE and TUBE. The data is obtained by injecting nude mice bearing T-380 tumor implants with 20 µg of ECH037.2 and the noted hapten, using a premix protocol.

Table VIII

| Tissue Localization of DB-III, NUBE and TUBE 24, 48 and 72 Hours Post-injection | | | | | | |
|---|---|---|---|---|---|---|
| | %Dose per Gram | | | | | |
| | DB-III | | NUBE | | TUBE | |
| | Blood | Tumor | Blood | Tumor | Blood | Tumor |
| 24 hrs. | 12.2 | 15.6 | 5.8 | 11.7 | 8.0 | 8.5 |
| 48 hrs. | 8.2 | 17.5 | 3.3 | 12.2 | 3.8 | 6.5 |
| 72 hrs. | 6.6 | 18.1 | 0.7 | 10.5 | 1.7 | 5.3 |

## Claims

**Claims for the following Contracting States : AT, BE, CH, DE, FR, GB, IT, LI, LU, NL, SE**

**1.** A compound of Formula (I):

$p$-$R_1$-$C_6H_4$-$CH_2$-EDTA-M (I)

wherein

$R_1$ is

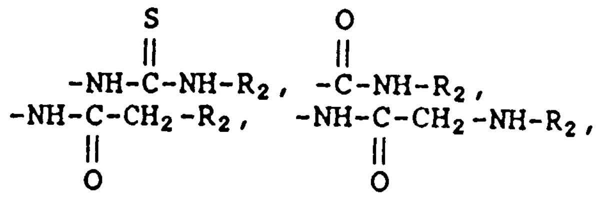

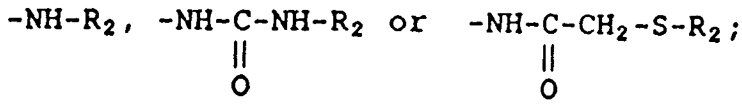

$R_2$ is hydrogen, an amino group, -OC(Y), -$p$-phenyl-$CH_2$-Y, an unsubstituted $C_1$ to $C_{30}$ branched or straight chain alkyl group; a substituted $C_1$ to $C_{30}$ branched or straight chain alkyl, cycloalkyl, aryl or arylalkyl group, in which the substituents are one or more of any of:

hydroxy, carboxy, =O, =S,

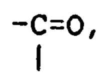

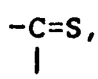

-S-, -$SR_4$, fluoro, chloro, bromo, iodo, amino, nitro, -$SO_3H$, -$NHR_3$, -$NHR_4$, -$N(R_3)_2$, -$CONHR_3$, -$COOR_3$, -$SO_4$, -$PO_4$, phenyl, benzyl, imidazolo, or a group of the formulae:

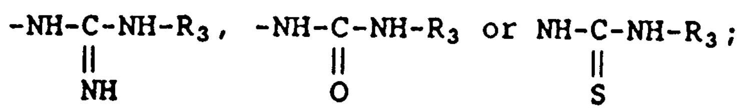

wherein

$R_3$ is hydrogen, a $C_1$ to $C_{30}$ straight or branched chain alkyl group, -$p$-phenyl-$CH_2$-Y,

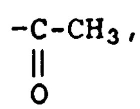

or a non-reactive functional group;

Y is EDTA, DTPA, DOTA, HETA, TRITA or TETA;

$R_4$ is H or

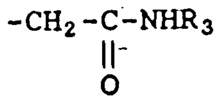

and, M is a metal ion,
or a pharmaceutically-acceptable salt thereof.

**2.** A compound according to Claim 1 of Formula (Ia):

p-$R_1$-$C_6H_4CH_2$EDTA-M (Ia)

wherein $R_1$ is

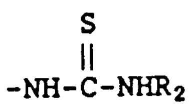 ; 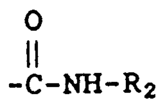 , or 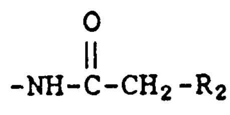 ;

in which $R_2$ is hydrogen; $NH_2$; an unsubstituted $C_1$-$C_{30}$ branched or straight chain alkyl group; or a $C_1$-$C_{30}$ branched or straight chain alkyl, cycloalkyl, aryl or arylalkyl group substituted by one or more substituents independently selected from the group consisting of:
-OH; -COOH; =0; =S; -S-; -Cl; -F; -Br; -I; $-NH_2$; $-N0_2$; $-S0_3H$; $-NH-R_3$; $-CONHR_3$; $-COOR_3$; $-S0_4$; $-P0_4$; phenyl; and benzyl;
$R_3$ is H; an alkyl group; or a non-reactive functional group; and M is a metal ion, or a pharmaceutically-acceptable salt thereof.

**3.** A compound according to Claim 1 of Formula (Ib):

p-$R_1$,-$C_6H_4$-$CH_2$-EDTA-M (Ib)

wherein
$R_1$ is

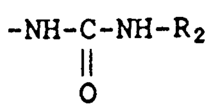 , 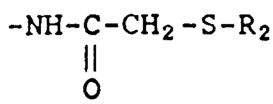 , 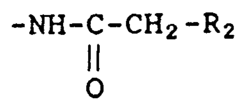 ,

$-NH-R_2$, 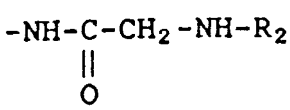 , or 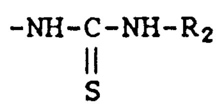 ;

$R_2$ is -p-phenyl-$CH_2$-Y, -OC(Y), a substituted $C_1$ to $C_{30}$ branched or straight chain alkyl, cycloalkyl, aryl or arylalkyl group, in which the substituents are one or more of any of:
hydroxy,

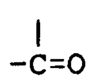 , 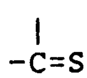 ,

$-SR_4$, $-NHR_3$, $NHR_4$, $-N(R_3)_2$, imidazolo, or a group of the formulae:

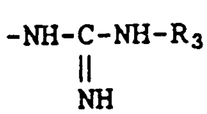 , 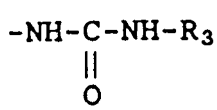 or 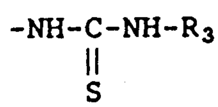 ;

wherein

$R_3$ is -p-phenyl-$CH_2$-Y, or

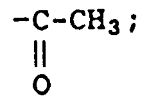

Y is EDTA, DTPA, DOTA, HETA, TRITA, or TETA;

$R_4$ is H or

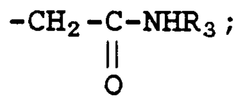

and, M is a metal ion,

or a pharmaceutically-acceptable salt thereof.

**4.** A compound of Claim 2 in which $R_1$ is -NH(C=S)NH-$R_2$ in which $R_2$ is selected from -H, -$CH_2CH_3$, -$(CH_2)_3CH_3$, -$(CH_2)_7CH_3$, -$CH_2CH_2OH$, -$CH_2CH_2NH_2$, -$C_6H_5$, -$CH_2C_6H_5$, -4-($C_6H_4$)$CH_2CO_2H$, -$(CH_2)_2CO_2H$, -$(CH_2)_3CO_2H$, -$(CH_2)_4CH(NHCOCH_3)CONH_2$, -$(CH_2)_2NHCO(CH_2)_3CO_2H$, or a pharmaceutically-acceptable salt thereof.

**5.** A compound of Claim 4 in which $R_2$ is -$CH_2CH_2OH$.

**6.** A compound of Claim 3 in which $R_1$ is -NH(C=S)NH-$R_2$ in which $R_2$ is -$C_6H_4$-$CH_2$-DTPA, -$CH_2CH_2$-NH(C=S)NH-$C_6H_4$-$CH_2$DTPA, or -$(CH_2)_4$-NH(C=S)NH-$C_6H_4$-$CH_2$DTPA.

**7.** A compound of Claim 6 in which $R_2$ is -$C_6H_4$-$CH_2$-DTPA.

**8.** A compound of Claim 3 of the Formula:

X-$CH_2$-CO-NH-$C_6H_4$-$CH_2$-EDTA-M,

in which X is -NH-$C_6H_4$-$CH_2$-DTPA,
-S$(CH_2)_4SCH_2$(C=O)NH$C_6H_4CH_2$DTPA,
-S$CH_2$CH(OH)CH(OH)$CH_2SCH_2$(C=O)NH$C_6H_4CH_2$DTPA,
or -NH$CH_2$CH(OH)$CH_2$NH$CH_2$(C=O)NH$C_6H_4CH_2$DTPA.

**9.** A compound of Claim 8 in which X is -NH-$C_6H_4$-$CH_2$-DTPA.

**10.** A compound, as claimed in any one of Claims 1 to 9, in which the metal ion is indium or yttrium.

**11.** A compound, as claimed in Claim 10, in which the metal ion is $^{111}$In or $^{90}$Y.

**12.** A diagnostic or therapeutic formulation which comprises a compound of Formulae (I), (Ia), or (Ib), or a pharmaceutically acceptable salt thereof, as defined in any one of Claims 1 to 11, associated with a pharmaceutically acceptable carrier, diluent or excipient therefor.

**13.** A kit adapted for the administration, simultaneously, sequentially, or as a complex, of an immunodiagnostic or immunotherapeutic agent, which comprises a compound of any one of Claims 1 to 11 and an antibody, or fragment thereof, said antibody or fragment capable of complexing with said compound.

**14.** A kit as claimed in Claim 13 in which the antibody is a monoclonal antibody, or fragment thereof.

**15.** A kit as claimed in Claim 13 in which the antibody is a bifunctional antibody, or fragment thereof.

**16.** A kit as claimed in Claim 13 in which the antibody is a chimeric antibody, or fragment thereof.

**17.** A kit as claimed in Claim 16 in which the antibody is a humanized antibody, or fragment thereof.

**18.** A kit as claimed in Claim in Claim 16 or 17 in which the antibody is a chimeric-bifunctional antibody, or fragment thereof.

**19.** A kit as claimed in any one of Claims 15 to 18 in which the antibody is a synthetic cross-linked antibody, or a fragment thereof.

**20.** A compound as claimed in any one of Claims 1 to 11 for use in therapy or diagnosis.

**Claims for the following Contracting States : ES, GR**

**1.** A diagnostic or therapeutic formulation which comprises as an active ingredient a compound of Formula (I):

$\underline{p}$-$R_1$-$C_6H_4$-$CH_2$-EDTA-M (I)

wherein

$R_1$ is

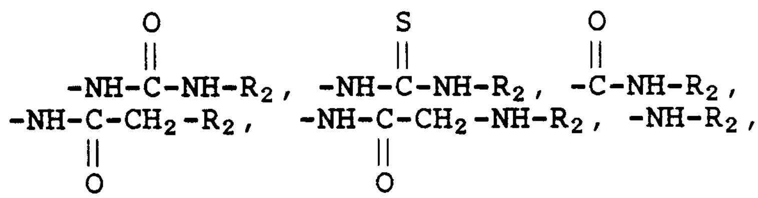

or 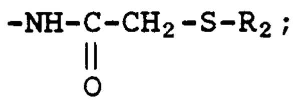

$R_2$ is hydrogen, an amino group, -OC(Y), -$\underline{p}$-phenyl-$CH_2$-Y, an unsubstituted $C_1$ to $C_{30}$ branched or straight chain alkyl group; a substituted $C_1$ to $C_{30}$ branched or straight chain alkyl, cycloalkyl, aryl or arylalkyl group, in which the substituents are one or more of any of: hydroxy, carboxy, =O, =S,

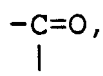

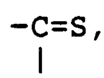

-S-, -$SR_4$, fluoro, chloro, bromo, iodo, amino, nitro, -$SO_3H$, -$NHR_3$, $NHR_4$, -$N(R_3)_2$, -$CONHR_3$, -$COOR_3$, -$SO_4$, -$PO_4$, phenyl, benzyl, imidazolo, or a group of the formulae:

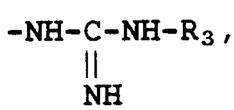, 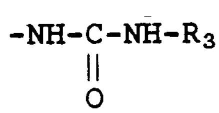 or 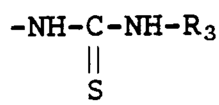;

wherein

$R_3$ is hydrogen, a $C_1$ to $C_{30}$ straight or branched chain alkyl group, -$\underline{p}$-phenyl-$CH_2$-Y,

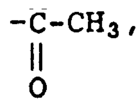

or a non-reactive functional group;

Y is EDTA, DTPA, DOTA, HETA, TRITA or TETA;

$R_4$ is H or

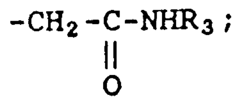

and, M is a metal ion,

or a pharmaceutically-acceptable salt thereof, associated with one or more pharmaceutically acceptable carriers, diluents or excipients therefor.

**2.** A diagnostic or therapeutic formulation which comprises as an active ingredient a compound as defined in Claim 1, of Formula (Ia):

p-$R_1$-$C_6H_4CH_2$EDTA-M (Ia)

wherein $R_1$ is

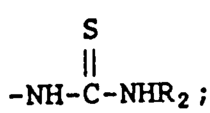 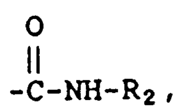 or 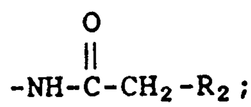

in which $R_2$ is hydrogen; $NH_2$; an unsubstituted $C_1$-$C_{30}$ branched or straight chain alkyl group; or a $C_1$-$C_{30}$ branched or straight chain alkyl, cycloalkyl, aryl or arylalkyl group substituted by one or more substituents independently selected from the group consisting of:

-OH; -COOH; =0; =S; -S-; -Cl; -F; -Br; -I; $-NH_2$; $-N0_2$; $-S0_3H$; $-NH-R_3$; $-CONHR_3$; $-COOR_3$; $-S0_4$; $-P0_4$; phenyl; and benzyl;

$R_3$ is H; an alkyl group; or a non-reactive functional group; and M is a metal ion, or a pharmaceutically-acceptable salt thereof, associated with one or more pharmaceutically acceptable carriers, diluents or excipients therefor.

**3.** A diagnostic or therapeutic formulation which comprises as an active ingredient a compound as defined in Claim 1, of Formula (Ib):

p-$R_1$-$C_6H_4$-$CH_2$-EDTA-M (Ib)

wherein

$R_1$ is

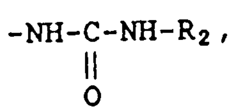 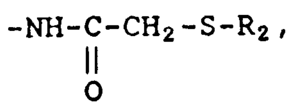 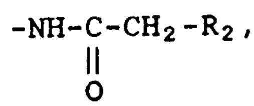

$-NH-R_2$, 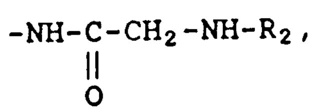 or 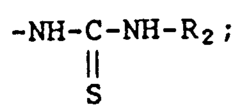

$R_2$ is -p-phenyl-$CH_2$-Y, -OC(Y), a substituted $C_1$ to $C_{30}$ branched or straight chain alkyl, cycloalkyl, aryl or arylalkyl group, in which the substituents are one or more of any of:

hydroxy,

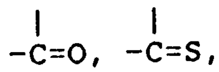

$-SR_4$, $-NHR_3$, $-NHR_4$, $-N(R_3)_2$, imidazolo, or a group of the formulae:

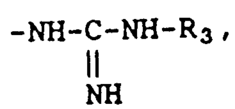 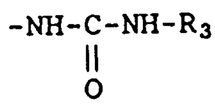 or 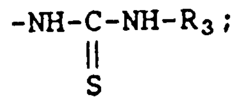

wherein

$R_3$ is -p-phenyl-$CH_2$-Y, or

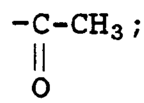

Y is EDTA, DTPA, DOTA, HETA, TRITA or TETA;

$R_4$ is H or

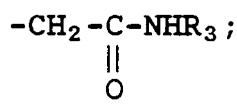

and, M is a metal ion,

or a pharmaceutically-acceptable salt thereof, associated with one or more pharmaceutically acceptable carriers, diluents or excipients therefor.

**4.** A formulation as claimed in Claim 2 in which $R_1$ of the active ingredient is -NH(C=S)NH-$R_2$ in which $R_2$ is selected from -H, $-CH_2CH_3$, $-(CH_2)_3CH_3$, $-(CH_2)_7CH_3$, $-CH_2CH_2OH$, $-CH_2CH,NH_2$, $-C_6H_5$, $-CH_2C_6H_5$, $-4-(C_6H_4)CH_2CO_2H$, $-(CH_2)_2CO_2H$, $-(CH_2)_3CO_2H$, $-(CH_2)_4CH(NHCOCH_3)CONH_2$, $-(CH_2)_2NHCO(CH_2)_3CO_2H$, or a pharmaceutically-acceptable salt thereof.

**5.** A formulation as claimed in Claim 4 in which $R_2$ of the active ingredient is $-CH_2CH_2OH$.

**6.** A formulation as claimed in Claim 3 in which $R_1$ of the active ingredient is -NH(C=S)NH-$R_2$ in which $R_2$ is $-C_6H_4-CH_2$-DTPA, $-CH_2CH_2-NH(C=S)NH-C_6H_4-CH_2$DTPA, or $-(CH_2)_4-NH(C=S)NH-C_6H_4-CH_2$DTPA.

**7.** A formulation as claimed in Claim 6 in which $R_2$ of the active ingredient is $-C_6H_4-CH_2$-DTPA.

**8.** A formulation as claimed in Claim 3 in which the active ingredient is a compound of the Formula:

X-$CH_2$-CO-NH-$C_6H_4$-$CH_2$-EDTA-M,

in which X is -NH-$C_6H_4$-$CH_2$-DTPA,
$-S(CH_2)_4SCH_2(C=O)NHC_6H_4CH_2$DTPA,
$-SCH_2CH(OH)CH(OH)CH_2SCH_2(C=O)NHC_6H_4CH_2$DTPA,
or $-NHCH_2CH(OH)CH_2NHCH_2(C=O)NHC_6H_4CH_2$DTPA.

**9.** A formulation as claimed in Claim 8 in which X of the active ingredient is -NH-$C_6H_4$-$CH_2$-DTPA.

**10.** A formulation as claimed in any one of Claims 1 to 9, in which the metal ion is indium or yttrium.

**11.** A formulation, as claimed in Claim 10, in which the metal ion is $^{111}In$ or $^{90}Y$.

**12.** A process for preparing a formulation as claimed in any one of Claims 1 to 11 which comprises admixing a compound of Formulae (I), (Ia), or (Ib), or a pharmaceutically acceptable salt thereof, as defined in any one of Claims 1 to 11, with a pharmaceutically acceptable carrier, diluent or excipient therefor.

**13.** A kit adapted for the administration, simultaneously, sequentially, or as a complex, of an immunodiagnostic or immunotherapeutic agent, which comprises a compound of any one of Claims 1 to 11 and an antibody, or fragment thereof, said antibody or fragment capable of complexing with said compound.

**14.** A kit as claimed in Claim 13 in which the antibody is a monoclonal antibody, or fragment thereof.

**15.** A kit as claimed in Claim 13 in which the antibody is a bifunctional antibody, or fragment thereof.

**16.** A kit as claimed in Claim 13 in which the antibody is a chimeric antibody, or fragment thereof.

**17.** A kit as claimed in Claim 16 in which the antibody is a humanized antibody, or fragment thereof.

**18.** A kit as claimed in Claim in Claim 16 or 17 in which the antibody is a chimeric-bifunctional antibody, or fragment thereof.

**19.** A kit as claimed in any one of Claims 15 to 18 in which the antibody is a synthetic cross-linked antibody, or a fragment thereof.

**20.** A process for preparing a compound of Formula (I):

$\underline{p}$-$R_1$-$C_6H_4$-$CH_2$-EDTA-M (I)

wherein

$R_1$ is

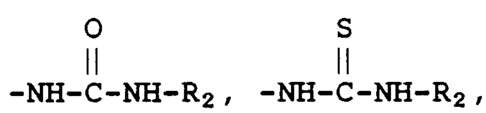

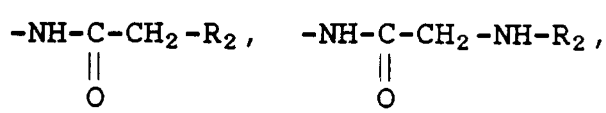

or 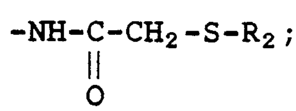

$R_2$ is hydrogen, an amino group, -$\underline{p}$-phenyl-$CH_2$-Y, an unsubstituted $C_1$ to $C_{30}$ branched or straight chain alkyl group; a substituted $C_1$ to $C_{30}$ branched or straight chain alkyl, cycloalkyl, aryl or arylalkyl group, in which the substituents are one or more of any of:

hydroxy, carboxy, =O, =S,

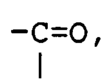

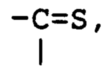

-S-, $-SR_4$, fluoro, chloro, bromo, iodo, amino, nitro, $-SO_3H$, $-NHR_3$, $-NHR_4$, $-N(R_3)_2$, $-CONHR_3$, $-COOR_3$, $-SO_4$, $-PO_4$, phenyl, benzyl, imidazolo, or a group of the formulae:

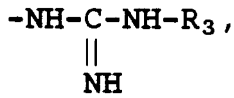 , 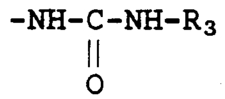 or 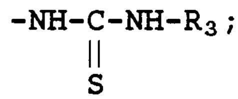 ;

wherein

$R_3$ is hydrogen, a $C_1$ to $C_{30}$ straight or branched chain alkyl group, -p-phenyl-$CH_2$-Y,

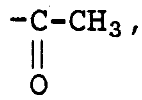

or a non-reactive functional group;

Y is EDTA, DTPA, DOTA, HETA, TRITA or TETA;

$R_4$ is H or

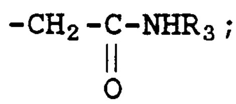

and, M is a metal ion,

or a pharmaceutically acceptable salt thereof, which comprises,

(A) in the case in which $R_1$ is $-NH(C=S)NH-R_2$, or $-NH(C=O)NH-R_2$,

(1) reacting isothiocyanatobenzyl EDTA, isocyanatobenzyl EDTA, as appropriate, or a metal chelate thereof, with a compound of the formula $H_2N-R_2$, in which $R_2$ is as described above; or,

(2) reacting a compound of the formula $R_2-N=C=S$, $R_2-N=C=O$, as appropriate, or a metal chelate thereof, with a compound of the formula:

EDTA-$CH_2$-$C_6H_4$-$(NH(C=S)NH)_m$-$(X)_n$-$NH_2$, or
EDTA-$CH_2$-$C_6H_4$-$(NH(C=O)NH)_m$-$(X)_n$-$NH_2$,

or a metal chelate thereof, in which X is a $C_1$ to $C_{30}$ branched or straight chain alkyl, aryl or arylalkyl group optionally substituted with one or more of the substituents allowed in the definition of $R_2$ above, and n and m, independently, are 0 or 1;

(B) if $R_1$ is $-NH(C=O)CH_2-S-R_2$, reacting p-bromoacetamidobenzyl-EDTA ("BABE"), or a metal chelate thereof, with a compound of the formula $HS-R_2$, in which $R_2$ is as defined above;

(C) if $R_1$ is $-NH(C=O)CH_2-NH-R_2$, reacting p-bromoacetamidobenzyl EDTA ("BABE") with a compound of the formula $H_2N-R_2$, in which $R_2$ is as defined above; or, if $R_1$ is $-NH(C=O)CH_2-NH-R_2$ in which a substituent of $R_2$ is $NHR_4$, reacting p-bromoacetamidobenzyl-Y in which Y is EDTA, DTPA, DOTA, HETA, TRITA or TETA,

or a metal chelate thereof, with a compound of the formula:

EDTA-$CH_2$-$C_6H_4$-$NH(C=O)CH_2$-NH-X-$NH_2$,

or a metal chelate thereof, in which X is a $C_1$ to $C_{30}$ branched or straight chain alkyl, aryl, or arylalkyl group optionally substituted with one or more of the substituents allowed in the definition of $R_2$, above; and,

(D) if desired, salifying or preparing the metal chelate of the product obtained.

**21.** A process as claimed in Claim 20 for preparing a compound of formula (I) in which $R_1$ is -NH(C=S)NH-$R_2$ in which $R_2$ is selected from -H, $-CH_2CH_3$, $-(CH_2)_3CH_3$, $-(CH_2)_7CH_3$, $-CH_2CH_2OH$, $-CH_2CH_2NH_2$, $-C_6H_5$, $-CH_2C_6H_5$, $-4-(C_6H_4)CH_2CO_2H$, $-(CH_2)_2CO_2H$, $-(CH_2)_3CO_2H$, $-(CH_2)_4CH(NHCOCH_3)CONH_2$, $-(CH_2)_2NHCO(CH_2)_3CO_2H$, or a pharmaceutically-acceptable salt thereof.

**22.** A process as claimed in Claim 21 for preparing a compound in which $R_2$ is $-CH_2CH_2OH$.

**23.** A process as claimed in Claim 20 for preparing a compound in which $R_1$ is -NH(C=S)NH-$R_2$ in which $R_2$ is $-C_6H_4-CH_2$-DTPA, $-CH_2CH_2$-NH(C=S)NH-$C_6H_4$-$CH_2$DTPA, or $-(CH_2)_4$-NH(C=S)NH-$C_6H_4$-$CH_2$DTPA.

**24.** A process as claimed in Claim 23 for preparing a compound in which $R_2$ is $-C_6H_4-CH_2$-DTPA.

**25.** A process as claimed in Claim 20 for preparing a compound of the Formula:

X-$CH_2$-CO-NH-$C_6H_4$-$CH_2$-EDTA-M,

in which X is -NH-$C_6H_4$-$CH_2$-DTPA,
-S$(CH_2)_4$S$CH_2$(C=O)NH$C_6H_4CH_2$DTPA,
-S$CH_2$CH(OH)CH(OH)$CH_2$S$CH_2$(C=O)NH$C_6H_4CH_2$DTPA,
or -NH$CH_2$CH(OH)$CH_2$NH$CH_2$(C=O)NH$C_6H_4CH_2$DTPA.

**26.** A process as claimed in Claim 25 in which X is -NH-$C_6H_4$-$CH_2$-DTPA.

**27.** A process as claimed in any one of Claims 20 to 25 in which the metal ion is indium or yttrium.

**28.** A process as claimed in Claim 27, in which the metal ion is $^{111}In$ or $^{90}Y$.

**29.** A process as claimed in any one of Claims 20 to 28 in which the pH of the reaction is between 6 and 12.

**30.** A process as claimed in Claim 29 in which the pH of the reaction is between 8 and 11.

**31.** A process as claimed in Claim 30 in which the temperature of the reaction is between 0° C. and 60° C.

**32.** A process as claimed in Claim 31 in which the temperature of the reaction is between 20° C. and 40° C.

**Patentansprüche**
**Patentansprüche für folgende Vertragsstaaten : AT, BE, CH, DE, FR, GB, IT, LI, LU, NL, SE**

**1.** Verbindung der Formel (I)

p-$R_1$-$C_6H_4$-$CH_2$-EDTA-M (I)

worin $R_1$ für

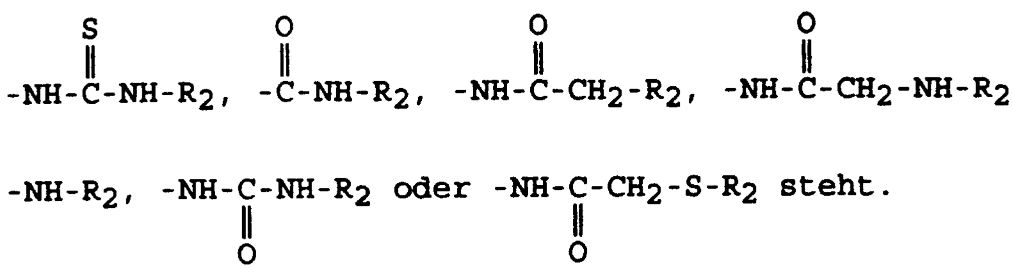

oder

steht.

$R_2$ für Wasserstoff, eine Aminogruppe, -OC-(-Y), p-Phenyl-$CH_2$-Y, eine unsubstituierte verzweigt- oder geradkettige $C_1$ bis $C_{30}$ Alkylgruppe, eine substituierte verzweigt- oder geradkettige $C_1$ bis $C_{30}$ Alkyl-, Cycloalkyl-, Aryl- oder Arylalkylgruppe steht, worin die Substituenten einer oder mehrere der folgenden sind
Hydroxy, Carboxy, =O, =S,

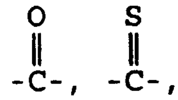

-S-, $-SR_4$, Fluor, Chlor, Brom, Iod, Amino, Nitro, $-SO_3H$, $-NHR_3$, $-NHR_4$, $-N(R_3)_2$, $-CONHR_3$, $-COOR_3$, $-SO_4$, $-PO_4$, Phenyl, Benzyl, Imidazol oder eine Gruppe der Formeln

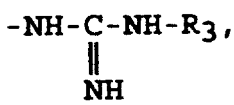 , 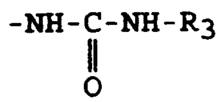 oder 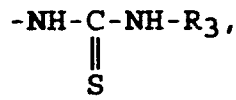 ,

worin
$R_3$ für Wasserstoff, eine geradkettige oder verzweigte $C_1$ bis $C_{30}$ Alkylgruppe, p-Phenyl-$CH_2$-Y,

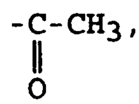

oder eine nicht-reaktive funktionelle Gruppe steht,
Y für EDTA, DTPA, DOTA, HETA, TRITA oder TETA steht,
$R_4$ für H oder

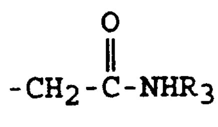

steht und M ein Metallion ist, oder ein pharmazeutisch annehmbares Salz hiervon.

2. Verbindung nach Anspruch 1 der Formel (Ia)

p-$R_1$-$C_6H_4CH_2$EDTA-M (Ia)

worin $R_1$ für

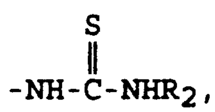 , 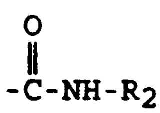 oder 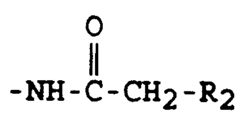

steht,
worin $R_2$ für Wasserstoff, $NH_2$, eine unsubstituierte verzweigt- oder geradkettige $C_1$ bis $C_{30}$ Alkylgruppe oder eine verzweigt- oder geradkettige $C_1$ bis $C_{30}$ Alkyl-, Cycloalkyl-, Aryl- oder Arylalkylgruppe steht, die durch einen oder mehrere aus folgender Gruppe unabhängig ausgewählte Substituenten substituiert ist
-OH, -COOH, =O, =S, -S-, -Cl-, -F-, -Br-, -I-, $-NH_2$, $-NO_2$, $-SO_3H$, $-NH-R_3$, $-CONHR_3$, $-COOR_3$, $-SO_4$, $-PO_4$, Phenyl und Benzyl
$R_3$ für H, eine Alkylgruppe oder eine nicht-reaktive funktionelle Gruppe steht, und M ein Metallion ist,

oder ein pharmazeutisch annehmbares Salz hiervon.

**3.** Verbindung nach Anspruch 1 der Formel (Ib)

p-$R_1$-$C_6H_4$-$CH_2$-EDTA-M (Ib)

worin $R_1$ für

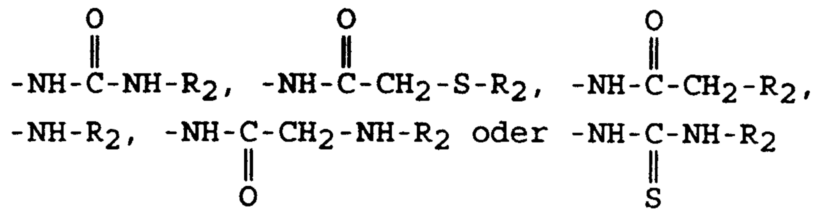

oder

steht.
$R_2$ für p-Phenyl-$CH_2$-Y, -OC-(-Y), eine substituierte verzweigt- oder geradkettige $C_1$ bis $C_{30}$ Alkyl-, Cycloalkyl-, Aryl- oder Arylalkylgruppe steht, worin die Substituenten einer oder mehrere der folgenden sind
Hydroxy,

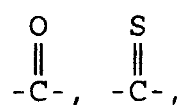

-$SR_4$, -$NHR_3$, -$NHR_4$, -$N(R_3)_2$, Imidazol oder eine Gruppe der Formeln

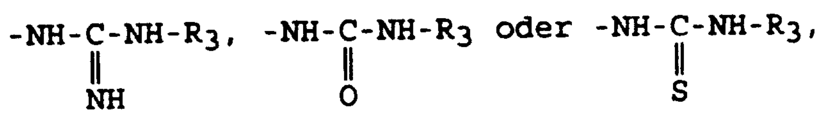

oder

worin
$R_3$ für p-Phenyl-$CH_2$-Y oder

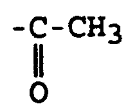

steht,
Y für EDTA, DTPA, DOTA, HETA, TRITA oder TETA steht,
$R_4$ für H oder

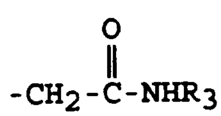

steht und M ein Metallion ist,
oder ein pharmazeutisch annehmbares Salz hiervon.

**4.** Verbindung nach Anspruch 2, worin $R_1$ für $-NH(C=S)NH-R_2$ steht, worin $R_2$ ausgewählt wird aus -H, $-CH_2CH_3$, $-(CH_2)_3CH_3$, $-(CH_2)_7CH_3$, $-CH_2CH_2OH$ $-CH_2CH_2NH_2$, $-C_6H_5$, $-CH_2C_6H_5$, $-4-(C_6H_4)CH_2CO_2H$, $-(CH_2)_2CO_2H$, $-(CH_2)_3CO_2H$, $-(CH_2)_4CH(NHCOCH_3)CONH_2$ oder $-(CH_2)_2NHCO(CH_2)_3CO_2H$, oder ein pharmazeutisch annehmbares Salz hiervon.

**5.** Verbindung nach Anspruch 4, worin $R_2$ für $-CH_2CH_2OH$ steht.

**6.** Verbindung nach Anspruch 3, worin $R_1$ für $-NH(C=S)NH-R_2$ steht, worin $R_2$ für $-C_6H_4-CH_2-DTPA$, $-CH_2CH_2-NH(C=S)NH-C_6H_4-CH_2DTPA$ oder $-(CH_2)_4-NH(C=S)NH-C_6H_4-CH_2DTPA$ steht.

**7.** Verbindung nach Anspruch 6, worin $R_2$ für $-C_6H_4-CH_2-DTPA$ steht.

**8.** Verbindung nach Anspruch 3 der Formel

$X-CH_2-CO-NH-C_6H_4-CH_2-EDTA-M$,

worin X für $-NH-C_6H_4-CH_2-DTPA$, $-S(CH_2)_4SCH_2(C=O)NHC_6H_4CH_2DTPA$, $-SCH_2CH(OH)CH(OH)-CH_2SCH_2(C=O)NHC_6H_4CH_2DTPA$ oder $-NHCH_2CH(OH)CH_2NHCH_2(C=O)NHC_6H_4CH_2DTPA$ steht.

**9.** Verbindung nach Anspruch 8, worin X für $-NH-C_6H_4-CH_2-DTPA$ steht.

**10.** Verbindung nach einem der Ansprüche 1 bis 9, worin das Metallion Indium oder Yttrium ist.

**11.** Verbindung nach Anspruch 10, worin das Metallion $^{111}In$ oder $^{90}Y$ ist.

**12.** Diagnostische oder therapeutische Formulierung, die eine Verbindung der Formel (I), (Ia) oder (Ib) oder ein pharmazeutisch annehmbares Salz hiervon nach einem der Ansprüche 1 bis 11 zusammen mit einem pharmazeutisch annehmbaren Träger, Verdünnungsmittel oder Hilfsstoff hierfür umfaßt.

**13.** Zur gleichzeitigen, sequentiellen oder komplexierten Verabreichung abgestimmter Kit eines immundiagnostischen oder immuntherapeutischen Mittels, der eine Verbindung nach einem der Ansprüche 1 bis 11 und einen Antikörper oder ein Fragment hiervon umfaßt, wobei dieser Antikörper oder dieses Fragment zur Komplexbildung mit dieser Verbindung fähig ist.

**14.** Kit nach Anspruch 13, worin der Antikörper ein monokionaler Antikörper oder ein Fragment hiervon ist.

**15.** Kit nach Anspruch 13, worin der Antikörper ein bifunktioneller Antikörper oder ein Fragment hiervon ist.

**16.** Kit nach Anspruch 13, worin der Antikörper ein chimärer Antikörper oder ein Fragment hiervon ist.

**17.** Kit nach Anspruch 16, worin der Antikörper ein humanisierter Antikörper oder ein Fragment hiervon ist.

**18.** Kit nach Anspruch 16 oder 17, worin der Antikörper ein chimärbifunktioneller Antikörper oder ein Fragment hiervon ist.

**19.** Kit nach einem der Ansprüche 15 bis 18, worin der Antikörper ein synthetisch quervernetzter Antikörper oder ein Fragment hiervon ist.

**20.** Verbindung nach einem der Ansprüche 1 bis 11 zur Verwendung in Therapie und Diagnose.

**Patentansprüche für folgende Vertragsstaaten : ES, GR**

**1.** Diagnostische oder therapeutische Formulierung, die als Wirkstoff eine Verbindung der Formel (I) umfaßt:

$p-R_1-C_6H_4-CH_2-EDTA-M$ (I)

worin $R_1$ für

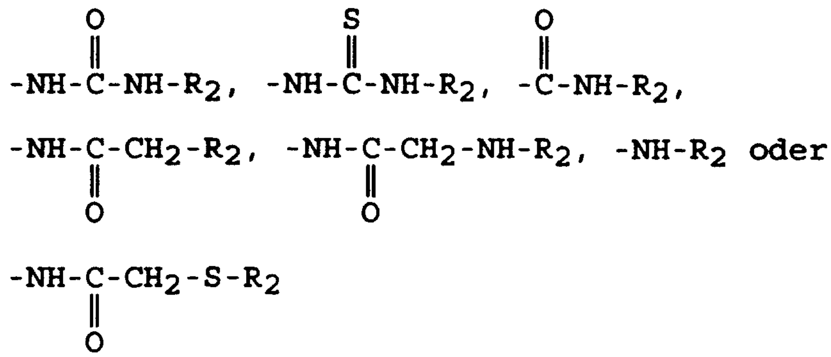

steht.

$R_2$ für Wasserstoff, eine Aminogruppe, -OC-(-Y), p-Phenyl-$CH_2$-Y, eine unsubstituierte verzweigt- oder geradkettige $C_1$ bis $C_{30}$ Alkylgruppe, eine substituierte verzweigt- oder geradkettige $C_1$ bis $C_{30}$ Alkyl-, Cycloalkyl-, Aryl- oder Arylalkylgruppe steht, worin die Substituenten einer oder mehrere der folgenden sind

Hydroxy, Carboxy, =O, =S,

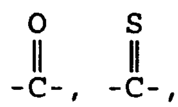

-S-, $-SR_4$, Fluor, Chlor, Brom, Iod, Amino, Nitro, $-SO_3H$, $NHR_3$, $-NHR_4$, $-N(R_3)_2$, $-CONHR_3$, $-COOR_3$, $-SO_4$, $-PO_4$, Phenyl, Benzyl, Imidazol oder eine Gruppe der Formeln

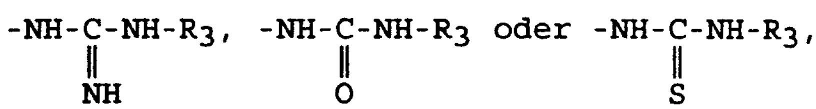

worin

$R_3$ für Wasserstoff, eine geradkettige oder verzweigte $C_1$ bis $C_{30}$ Alkylgruppe, p-Phenyl-$CH_2$-Y,

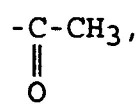

oder eine nicht-reaktive funktionelle Gruppe steht,

Y für EDTA, DTPA, DOTA, HETA, TRITA oder TETA steht,

$R_4$ für H oder

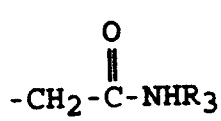

steht und M ein Metallion ist,

oder ein pharmazeutisch annehmbares Salz hiervon, zusammen mit einem oder mehreren pharmazeutisch annehmbaren Trägern, Verdünnungsmitteln oder Hilfsstoffen hierfür.

**2.** Diagnostische oder therapeutische Formulierung, die als Wirkstoff eine Verbindung nach Anspruch 1 der Formel (Ia) umfaßt:

p-$R_1$-$C_6H_4CH_2$EDTA-M (Ia)

worin $R_1$ für

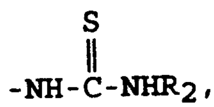, 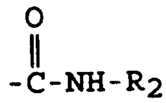 oder 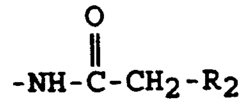

steht,
worin $R_2$ für Wasserstoff, $NH_2$, eine unsubstituierte verzweigt- oder geradkettige $C_1$ bis $C_{30}$ Alkylgruppe oder eine verzweigt- oder geradkettige $C_1$ bis $C_{30}$ Alkyl-, Cycloalkyl-, Aryl oder Arylalkylgruppe steht, die durch einen oder mehrere aus folgender Gruppe unabhängig ausgewählte Substituenten substituiert ist
-OH, -COOH, =O, =S, -S-, -Cl-, -F-, -Br-, -I-, $-NH_2$, $-NO_2$, $-SO_3H$, $-NH-R_3$, $-CONHR_3$, $-COOR_3$, $-SO_4$, $-PO_4$, Phenyl und Benzyl
$R_3$ für H, eine Alkylgruppe oder eine nicht-reaktive funktionelle Gruppe steht, und M ein Metallion ist, oder ein pharmazeutisch annehmbares Salz hiervon,
zusammen mit einem oder mehreren pharmazeutisch annehmbaren Trägern, Verdünnungsmitteln oder Hilfsstoffen hierfür.

3. Diagnostische oder therapeutische Formulierung, die als Wirkstoff eine Verbindung nach Anspruch 1 der Formel (Ib) umfaßt

p-$R_1$-$C_6H_4$-$CH_2$-EDTA-M (Ib)

worin $R_1$ für

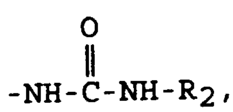, 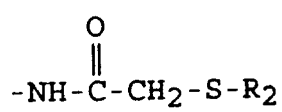, 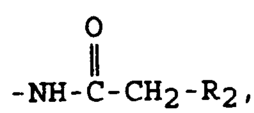,
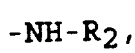, 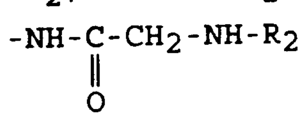 oder 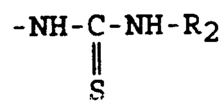

steht.
$R_2$ für p-Phenyl-$CH_2$-Y, -OC-(-Y), eine substituierte verzweigt- oder geradkettige $C_1$ bis $C_{30}$ Alkyl-, Cycloalkyl-, Aryl- oder Aryl-alkylgruppe steht, worin die Substituenten einer oder mehrere der folgenden sind
Hydroxy,

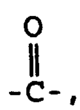, 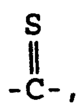,

$-SR_4$, $-NHR_3$, $-NHR_4$, $-N(R_3)_2$, Imidazol oder eine Gruppe der Formeln

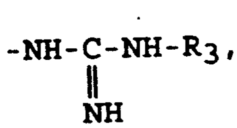, 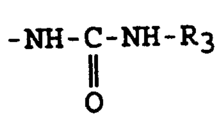 oder 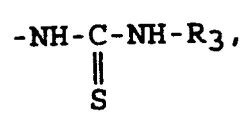,

worin
$R_3$ für p-Phenyl-$CH_2$-Y oder

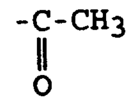

steht,
Y für EDTA, DTPA, DOTA, HETA, TRITA oder TETA steht,
$R_4$ für H oder

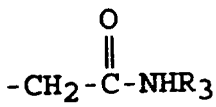

steht und M ein Metallion ist,
oder ein pharmazeutisch annehmbares Salz hiervon,
zusammen mit einem oder mehreren pharmazeutisch annehmbaren Trägern, Verdünnungsmitteln oder Hilfsstoffen hierfür.

**4.** Formulierung nach Anspruch 2, worin $R_1$ des Wirkstoffs für -NH(C = S)NH-$R_2$ steht, worin $R_2$ ausgewählt wird aus -H, -$CH_2CH_3$, -$(CH_2)_3CH_3$, -$(CH_2)_7CH_3$, -$CH_2CH_2OH$ -$CH_2CH_2NH_2$, -$C_6H_5$, -$CH_2C_6H_5$, -4-$(C_6H_4)CH_2CO_2H$, -$(CH_2)_2CO_2H$, -$(CH_2)_3CO_2H$,
-$(CH_2)_4CH(NHCOCH_3)CONH_2$ oder $(CH_2)_2NHCO(CH_2)_3CO_2H$,
oder ein pharmazeutisch annehmbares Salz hiervon.

**5.** Formulierung nach Anspruch 4, worin $R_2$ des Wirkstoffs für -$CH_2CH_2OH$ steht.

**6.** Formulierung nach Anspruch 3, worin $R_1$ des Wirkstoffs für -NH(C = S)NH-$R_2$ steht, worin $R_2$ für -$C_6H_4$-$CH_2$-DTPA für -$CH_2CH_2$-NH(C = S)NH-$C_6H_4$-$CH_2$DTPA oder $(CH_2)_4$-NH(C = S)NH-$C_6H_4$-$CH_2$DTPA steht.

**7.** Formulierung nach Anspruch 6, worin $R_2$ des Wirkstoffs für -$C_6H_4$-$CH_2$-DTPA steht.

**8.** Formulierung nach Anspruch 3, worin der Wirkstoff eine Verbindung folgender Formel ist

X-$CH_2$-CO-NH-$C_6H_4$-$CH_2$-EDTA-M,

worin X für -NH-$C_6H_4$-$CH_2$-DTPA, -$S(CH_2)_4SCH_2$(C = O)$NHC_6H_4CH_2$DTPA, -$SCH_2$CH(OH)CH(OH)-$CH_2SCH_2$(C = O)$NHC_6H_4CH_2$DTPA oder -$NHCH_2CH(OH)CH_2NHCH_2$(C = O)$NHC_6H_4CH_2$DTPA steht.

**9.** Formulierung nach Anspruch 8, worin X des Wirkstoffs für -NH-$C_6H_4$-$CH_2$-DTPA steht.

**10.** Formulierung nach einem der Ansprüche 1 bis 9, worin das Metallion Indium oder Yttrium ist.

**11.** Formulierung nach Anspruch 10, worin das Metallion $^{111}$In oder $^{90}$Y ist.

**12.** Verfahren zur Herstellung einer Formulierung nach einem der Ansprüche 1 bis 11, gekennzeichnet durch Mischen einer Verbindung der Formel (I), (Ia) oder (Ib) oder einem pharmazeutisch annehmbaren Salz hiervon nach einem der Ansprüche 1 bis 11 mit einem pharmazeutisch annehmbaren Träger, Verdünnungsmittel oder Hilfsstoff hierfür.

**13.** Zur gleichzeitigen, sequentiellen oder komplexierten Verabreichung abgestimmter Kit eines immundiagnostischen oder immuntherapeutischen Mittels, der eine Verbindung nach einem der Ansprüche 1 bis 11 und einen Antikörper oder Fragment hiervon umfaßt, wobei dieser Antikörper oder dieses Fragment zur Komplexbildung mit dieser Verbindung fähig ist.

**14.** Kit nach Anspruch 13, worin der Antikörper ein monoklonaler Antikörper oder ein Fragment hiervon ist.

**15.** Kit nach Anspruch 13, worin der Antikörper ein bifunktioneller Antikörper oder ein Fragment hiervon ist.

**16.** Kit nach Anspruch 13, worin der Antikörper ein chimärer Antikörper oder ein Fragment hiervon ist.

**17.** Kit nach Anspruch 16, worin der Antikörper ein humanisierter Antikörper oder ein Fragment hiervon ist.

**18.** Kit nach Anspruch 16 oder 17, worin der Antikörper ein chimärbifunktioneller Antikörper oder ein Fragment hiervon ist.

**19.** Kit nach einem der Ansprüche 15 bis 18, worin der Antikörper ein synthetisch quervernetzter Antikörper oder ein Fragment hiervon ist.

**20.** Verfahren zur Herstellung einer Verbindung der Formel (I)

$p\text{-}R_1\text{-}C_6H_4\text{-}CH_2\text{-EDTA-M}$ (I)

worin $R_1$ für

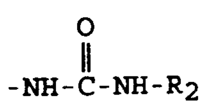, 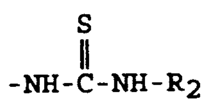, 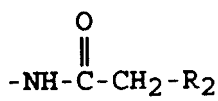, 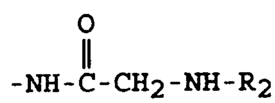,

oder

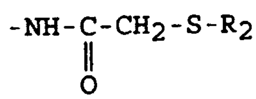

steht.
$R_2$ für Wasserstoff, eine Aminogruppe, p-Phenyl-$CH_2$-Y, eine unsubstituierte verzweigt- oder geradkettige $C_1$ bis $C_{30}$ Alkylgruppe, eine substituierte verzweigt- oder geradkettige $C_1$ bis $C_{30}$ Alkyl-, Cycloalkyl-, Aryl- oder Arylalkylgruppe steht, worin die Substituenten einer oder mehrere der folgenden sind Hydroxy, Carboxy, =O, =S,

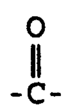, 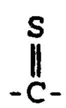,

-S-, $-SR_4$, Fluor, Chlor, Brom, Iod, Amino, Nitro, $-SO_3H$, $-NHR_3$, $-NHR_4$, $-N(R_3)_2$, $-CONHR_3$, $-COOR_3$, $-SO_4$, $-PO_4$, Phenyl, Benzyl, Imidazol oder eine Gruppe der Formeln

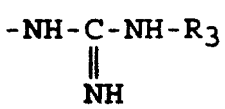, 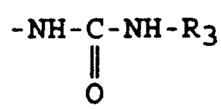 oder 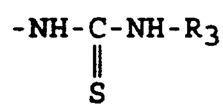,

worin
$R_3$ für Wasserstoff, eine geradkettige oder Verzweigte $C_1$ bis $C_{30}$ Alkylgruppe, p-Phenyl-$CH_2$-Y,

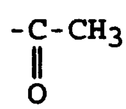,

oder eine nicht-reaktive funktionelle Gruppe steht,

Y für EDTA, DTPA, DOTA, HETA, TRITA oder TETA steht,
$R_4$ für H oder

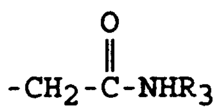

steht und M ein Metallion ist, oder ein pharmazeutisch annehmbares Salz hiervon, gekennzeichnet durch

(A) falls $R_1$ für $-NH(C=S)NH-R_2$ oder $-NH(C=O)NH-R_2$ steht

(1) Umsetzung von Isothiocyanatbenzyl-EDTA oder IsocyanatbenzylEDTA, je nach Bignung, oder eines Metallchelats hiervon mit einer Verbindung der Formel $H_2N-R_2$, worin $R_2$ dasselbe ist, wie vorher beschrieben, oder

(2) Umsetzung einer Verbindung der Formel $R_2-N=C=S$, $R_2-N=C=O$, je nach Eignung, oder eines Metallchelats hiervon, mit einer Verbindung der Formel

$EDTA-CH_2-C_6-H_4-(NH(C=S)NH)_m-(X)_n-NH_2$ oder
$EDTA-CH_2-C_6H_4-(NH(C=O)NH)_m-(X)_n-NH_2$ steht

oder einem Metallchelat hiervon, worin X für eine verzweigt- oder geradkettige $C_1$ bis $C_{30}$ Alkyl-, Aryl- oder Arylalkylgruppe steht, die wahlweise mit einem oder mehreren Substituenten substituiert sind, die in der vorhergehenden Definition für $R_2$ zugelassen sind, und n und m unabhängig für 0 oder 1 stehen,

(B) falls $R_1$ für $-NH(C=O)CH_2-S-R_2$ steht,
Umsetzung von p-Bromacetamidbenzyl-EDTA ("BABE") oder eines Metallchelats hiervon mit einer Verbindung der Formel $HS-R_2$, worin $R_2$ wie vorher definiert ist.

(C) falls $R_1$ für $-NH(C=O)CH_2-NH-R_2$ steht,
Umsetzung von p-Bromacetamidbenzyl-EDTA ("BABE") mit einer Verbindung der Formel $H_2N-R_2$, worin $R_2$ wie vorher definiert ist, oder falls $R_1$ für $-NH(C=O)CH_2-NH-R_2$ steht, worin ein Substituent von $R_2$ $NHR_4$ ist, Umsetzung von p-Bromacetamidbenzyl-Y, worin Y für EDTA, DTPA, DOTA, HETA, TRITA oder TETA steht, oder eines Metallchelats hiervon mit einer Verbindung der Formel

$EDTA-CH_2-C_6H_4-NH(C=O)CH_2-NH-X-NH_2$

oder einem Metallchelat hiervon, worin X für eine verzweigt- oder geradkettige $C_1$ bis $C_{30}$ Alkyl-, Aryl- oder Arylalkylgruppe steht, die wahlweise mit einem oder mehreren Substituenten substituiert sind, die in der vorhergehenden Definition für $R_2$ zugelassen sind, und

(D) falls erforderlich, Salzbildung oder Herstellung des Metallchelats des erhaltenen Produkts.

**21.** Verfahren nach Anspruch 20 zur Herstellung einer Verbindung der Formel (I), worin $R_1$ für $-NH(C=S)-NH-R_2$ steht, worin $R_2$ ausgewählt wird aus -H, $-CH_2CH_3$, $-(CH_2)_3CH_3$, $-(CH_2)_7CH_3$, $-CH_2CH_2OH$ $-CH_2CH_2NH_2$, $-C_6H_5$, $-CH_2C_6H_5$, $-4-(C_6H_4)CH_2CO_2H$, $-(CH_2)_2CO_2H$, $-(CH_2)_3CO_2H$, $-(CH_2)_4CH-(NHCOCH_3)CONH_2$ oder $-(CH_2)_2NHCO(CH_2)_3CO_2H$, oder eines pharmazeutischen annehmbaren Salzes hiervon.

**22.** Verfahren nach Anspruch 21 zur Herstellung einer Verbindung, worin $R_2$ für $-CH_2CH_2OH$ steht.

**23.** Verfahren nach Anspruch 20 zur Herstellung einer Verbindung, worin $R_1$ für $-NH(C=S)NH-R_2$ steht, worin $R_2$ für $-C_6H_4-CH_2-DTPA$,$-CH_2CH_2-NH(C=S)NH-C_6H_4-CH_2DTPA$ oder $-(CH_2)_4-NH(C=S)NH-C_6H_4-CH_2DTPA$ steht.

**24.** Verfahren nach Anspruch 23 zur Herstellung einer Verbindung, worin $R_2$ für $-C_6H_4-CH_2-DTPA$ steht.

**25.** Verfahren nach Anspruch 20 zur Herstellung einer Verbindung der Formel

$X-CH_2-CO-NH-C_6H_4-CH_2-EDTA-M$,

worin X für $-NH-C_6H_4-CH_2-DTPA$, $-S(CH_2)_4SCH_2(C=O)NHC_6H_4CH_2DTPA$, $-SCH_2CH(OH)CH(OH)CH_2SCH_2(C=O)NHC_6H_4CH_2DTPA$ oder $-NHCH_2CH(OH)CH_2NHCH_2(C=O)NHC_6H_4CH_2DTPA$ steht.

**26.** Verfahren nach Anspruch 25, worin X für $-NH-C_6H_4-CH_2-DTPA$ steht.

**27.** Verfahren nach einem der Ansprüche 20 bis 25, worin das Metallion Indium oder Yttrium ist.

**28.** Verfahren nach Anspruch 27, worin das Metallion $^{111}In$ oder $^{90}Y$ ist.

**29.** Verfahren nach einem der Ansprüche 20 bis 28, worin der pH der Reaktion zwischen 6 und 12 liegt.

**30.** Verfahren nach Anspruch 29, worin der pH der Reaktion zwischen 8 und 11 liegt.

**31.** Verfahren nach Anspruch 30, worin die Reaktionstemperatur zwischen 0 °C und 60 °C liegt.

**32.** Verfahren nach Anspruch 31, worin die Reaktionstemperatur zwischen 20 °C und 40 °C liegt.

**Revendications**
**Revendications pour les Etats contractants suivants : AT, BE, CH, DE, FR, GB, IT, LI, LU, NL, SE**

**1.** Composé de formule (I) :

$p$-$R_1$-$C_6H_4$-$CH_2$-EDTA-M (I)

dans laquelle
$R_1$ représente

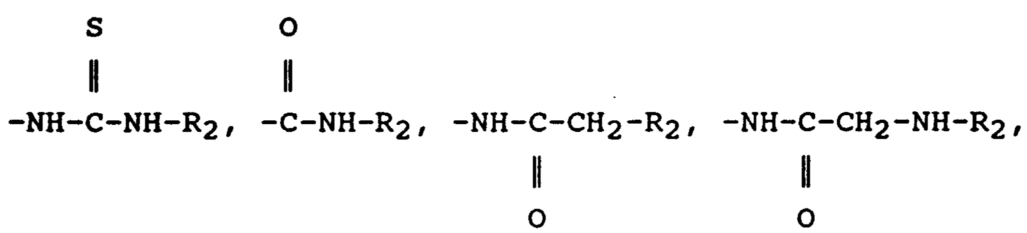

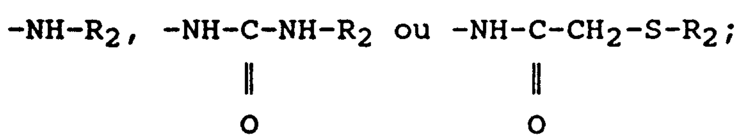

$R_2$ représente un atome d'hydrogène, un groupe amino, -OC-(-Y), un groupe $p$-phényle-$CH_2$-Y, un groupe alkyle en $C_1$-$C_{30}$ à chaîne droite ou ramifiée non substitué; un groupe alkyle en $C_1$-$C_{30}$ à chaîne droite ou ramifiée, un groupe cycloalkyle, un groupe aryle ou un groupe arylalkyle, dans lesquels les substituants sont un ou plusieurs groupes de n'importe quels groupes ci-après :
un groupe hydroxyle, un groupe carboxyle, =O, =S,

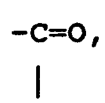

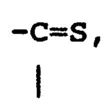

-S-, $-SR_4$, un atome de fluor, un atome de chlore, un atome de brome, un atome d'iode, un groupe amino, un groupe nitro, $-SO_3H$, $-NHR_3$, $-NHR_4$, $-N(R_3)_2$, $-CONHR_3$, $-COOR_3$, $-SO_4$, $-PO_4$, un groupe phényle, un groupe benzyle, un groupe imidazolo ou encore un groupe répondant aux formules :

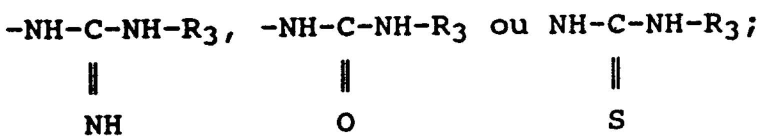

dans lesquelles
$R_3$ représente un atome d'hydrogène, un groupe alkyle en $C_1$-$C_{30}$ à chaîne droite ou ramifiée, un groupe p-phényle-$CH_2$-Y,

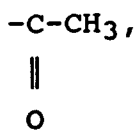

ou encore un groupe fonctionnel non réactif;
Y représente EDTA, DTPA, DOTA, HETA, TRITA ou TETA;
$R_4$ représente H ou

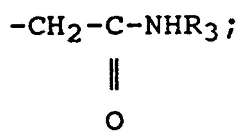

et
M représente un ion métallique,
ou bien un de ses sels pharmaceutiquement acceptables.

**2.** Composé selon la revendication 1, de formule (Ia) :

p-$R_1$-$C_6H_4$-$CH_2$-EDTA-M (Ia)

dans laquelle
$R_1$ représente

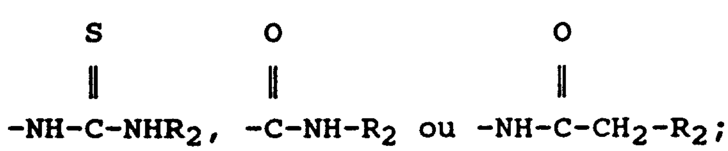

où
$R_2$ représente un atome d'hydrogène; $NH_2$; un groupe alkyle en $C_1$-$C_{30}$ à chaîne droite ou ramifiée non substitué; ou encore un groupe alkyle à chaîne droite ou ramifiée en $C_1$-$C_{30}$, un groupe cycloalkyle, un groupe aryle ou un groupe arylalkyle substitués par un ou plusieurs substituants choisis de manière indépendante parmi le groupe constitué par : -OH; -COOH; =O; =S; -S-; -Cl; -F; -Br; -I; $-NH_2$; $-NO_2$; $-SO_3H$; $-NH-R_3$; $-CONHR_3$; $-COOR_3$; $-SO_4$; $-PO_4$; un groupe phényle et un groupe benzyle;
$R_3$ représente H, un groupe alkyle ou encore un groupe fonctionnel non réactif; et
M représente un ion métallique,

ou un de ses sels pharmaceutiquement acceptables.

**3.** Composé selon la revendication 1, de formule (Ib)

p-$R_1$-$C_6H_4$-$CH_2$-EDTA-M (Ib)

dans laquelle
$R_1$ représente

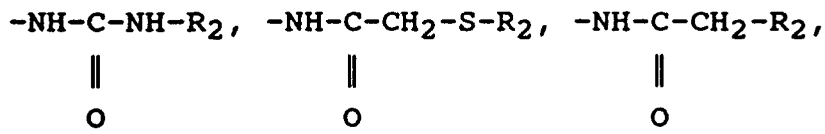

-NH-$R_2$,

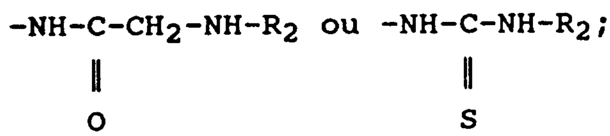

$R_2$ représente un groupe p-phényle-$CH_2$-Y, -OC-(-Y), un groupe alkyle en $C_1$-$C_{30}$ à chaîne droite ou ramifiée, un groupe cycloalkyle, un groupe aryle ou un groupe arylalkyle dans lesquels les substituants sont un ou plusieurs groupes de n'importe quels groupes ci-après :
un groupe hydroxyle,

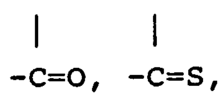

-$SR_4$, -$NHR_3$, -$NHR_4$,
-$N(R_3)_2$, un groupe imidazolo
ou encore un groupe répondant aux formules :

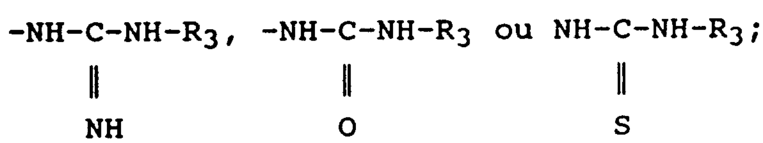

dans lesquelles
$R_3$ représente un groupe p-phényle-$CH_2$-Y ou

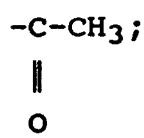

Y représente EDTA, DTPA, DOTA, HETA, TRITA ou TETA;
$R_4$ représente H ou

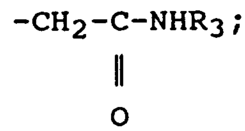

et
M représente un ion métallique,
ou un de ses sels pharmaceutiquement acceptables.

4. Composé selon la revendication 2, dans lequel $R_1$ représente -NH(C = S)NH-$R_2$ où $R_2$ est choisi parmi -H, - $CH_2CH_3$, -$(CH_2)_3CH_3$, -$(CH_2)_7CH_3$, -$CH_2CH_2OH$, -$CH_2CH_2NH_2$, - $C_6H_5$, -$CH_2C_6H_5$, -4-($C_6H_4$)-$CH_2CO_2H$, -$(CH_2)_2CO_2H$, -$(CH_2)_3CO_2H$, -$(CH_2)_4CH(NHCOCH_3)CONH_2$, -$(CH_2)_2NHCO(CH_2)_3CO_2H$, ou un de ses sels pharmaceutiquement acceptables.

5. Composé selon la revendication 4, dans lequel $R_2$ représente -$CH_2CH_2OH$.

6. Composé selon la revendication 3, dans lequel $R_1$ représente : -NH(C = S)NH-$R_2$ où $R_2$ représente
-$C_6H_4$-$CH_2$-DTPA,
- $CH_2CH_2$-NH(C = S)NH-$C_6H_4$-$CH_2$DTPA ou
-$(CH_2)_4$-NH(C = S)NH-$C_6H_4$-$CH_2$DTPA.

7. Composé selon la revendication 6, dans lequel $R_2$ représente -$C_6H_4$-$CH_2$-DTPA.

8. Composé selon la revendication 3, répondant à la formule :

X-$CH_2$-CO-NH-$C_6H_4$-$CH_2$-EDTA-M,

dans laquelle
X représente
-NH-$C_6H_4$-$CH_2$-DTPA,
-$S(CH_2)_4SCH_2$(C = O)NH$C_6H_4CH_2$DTPA,
-$SCH_2$CH(OH)CH(OH)$CH_2SCH_2$(C = O)NH$C_6H_4CH_2$DTPA ou
-NH$CH_2$CH(OH)$CH_2$NH$CH_2$(C = O)NH$C_6H_4$DTPA.

9. Composé selon la revendication 8, dans lequel X représente -NH-$C_6H_4$-$CH_2$-DTPA.

10. Composé selon l'une quelconque des revendications 1 à 9, dans lequel l'ion métallique est l'indium ou l'yttrium.

11. Composé selon la revendication 10, dans lequel l'ion métallique est $^{111}$In ou $^{90}$Y.

12. Formulation diagnostique ou thérapeutique qui comprend un composé des formules (I), (Ia) ou (Ib), ou encore un de ses sels pharmaceutiquement acceptables, tel que défini dans l'une quelconque des revendications 1 à 11, en association avec un support, un diluant ou un excipient pharmaceutiquement acceptable pour le composé.

13. Nécessaire conçu pour l'administration, de manière simultanée, de manière séquentielle ou sous forme d'un complexe. d'un agent immunodiagnostique ou immunothérapeutique qui comprend un composé selon l'une quelconque des revendications 1 à 11 et un anticorps ou un fragment de ce dernier, ledit anticorps ou ledit fragment étant capable de complexer avec ledit composé.

14. Nécessaire selon la revendication 13, dans lequel l'anticorps est un anticorps monoclonal ou un fragment de ce dernier.

15. Nécessaire selon la revendication 13, dans lequel l'anticorps est un anticorps bifonctionnel ou un fragment de ce dernier.

**16.** Nécessaire selon la revendication 13, dans lequel l'anticorps est un anticorps chimère ou un fragment de ce dernier.

**17.** Nécessaire selon la revendication 16, dans lequel l'anticorps est un anticorps humanisé ou un fragment de ce dernier.

**18.** Nécessaire selon la revendication 16 ou 17, dans lequel l'anticorps est un anticorps chimère-bifonctionnel ou un fragment de ce dernier.

**19.** Nécessaire selon l'une quelconque des revendications 15 à 18, dans lequel l'anticorps est un anticorps synthétique croisé ou un fragment de ce dernier.

**20.** Composé selon l'une quelconque des revendications 1 à 11, pour être utilisé dans la thérapie ou dans le diagnostic.

**Revendications pour les Etats contractants suivants : ES, GR**

**1.** Formulation diagnostique ou thérapeutique qui comprend, comme ingrédient actif, un composé de formule (I) :

$\underline{p}$-$R_1$-$C_6H_4$-$CH_2$-EDTA-M (I)

dans laquelle
$R_1$ représente

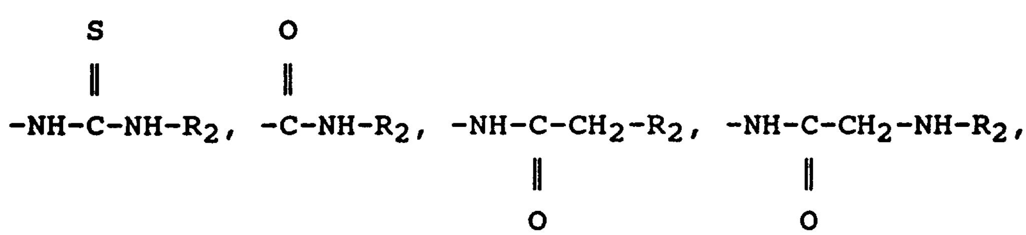

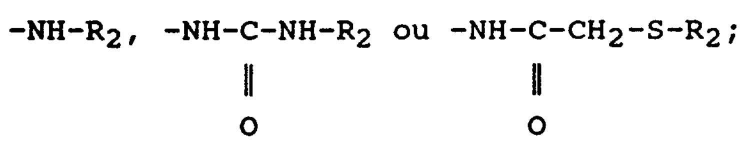
ou

$R_2$ représente un atome d'hydrogène, un groupe amino, -OC-(-Y), un groupe $\underline{p}$-phényle-$CH_2$-Y, un groupe alkyle en $C_1$-$C_{30}$ à chaîne droite ou ramifiée non substitué; un groupe alkyle à chaîne droite ou ramifiée en $C_1$-$C_{30}$, un groupe cycloalkyle, un groupe aryle ou un groupe arylalkyle substitués, dans lesquels les substituants sont un ou plusieurs groupes de n'importe quels groupes ci-après : un groupe hydroxyle, un groupe carboxyle, =O, =S,

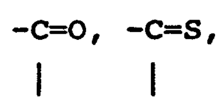

-S-, $-SR_4$, un atome de fluor, un atome de chlore, un atome de brome, un atome d'iode, un groupe amino, un groupe nitro, $-SO_3H$, $-NHR_3$, $-NHR_4$, $-N(R_3)_2$, $-CONHR_3$, $-COOR_3$, - $SO_4$, $-PO_4$, un groupe phényle, un groupe benzyle, un groupe imidazolo ou encore un groupe répondant aux formules :

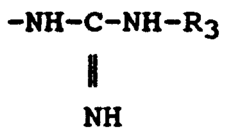, 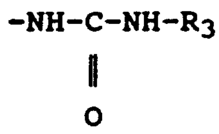 ou 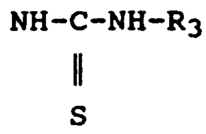;

dans lesquelles

$R_3$ représente un atome d'hydrogène, un groupe alkyle en $C_1$-$C_{30}$ à chaîne droite ou ramifiée, un groupe p-phényle-$CH_2$-Y,

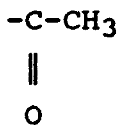,

ou encore un groupe fonctionnel non réactif;

Y représente EDTA, DTPA, DOTA, HETA, TRITA ou TETA;

$R_4$ représente H ou

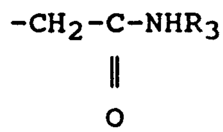;

et

M représente un ion métallique,

ou bien un de ses sels pharmaceutiquement acceptables, en association avec un ou plusieurs supports, diluants ou excipients pharmaceutiquement acceptables pour le composé.

**2.** Formulation diagnostique ou thérapeutique qui comprend, comme ingrédient actif, un composé tel que défini à la revendication 1, de formule (Ia)

p-$R_1$-$C_6H_4$-$CH_2$-EDTA-M (Ia)

dans laquelle

$R_1$ représente

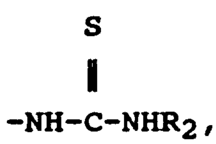 ou 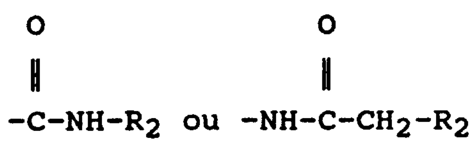;

où

$R_2$ représente un atome d'hydrogène; $NH_2$; un groupe alkyle en $C_1$-$C_{30}$ à chaîne droite ou ramifiée non substitué; ou encore un groupe alkyle à chaîne droite ou ramifiée en $C_1$-$C_{30}$, un groupe cycloalkyle, un groupe aryle ou un groupe arylalkyle substitués par un ou plusieurs substituants choisis de manière indépendante parmi le groupe constitué par : -OH; -COOH; =O; =S; -S-; -Cl; -F; -Br; -I; $-NH_2$; - $NO_2$; $-SO_3H$; -NH-$R_3$; -CONH$R_3$; -COO$R_3$; $-SO_4$; $-PO_4$; un groupe phényle et un groupe benzyle;

$R_3$ représente H, un groupe alkyle ou encore un groupe fonctionnel non réactif; et

M représente un ion métallique,

ou un de ses sels pharmaceutiquement acceptables, en association avec un ou plusieurs supports, diluants ou excipients pharmaceutiquement acceptables pour le composé.

**3.** Formulation diagnostique ou thérapeutique qui comprend, comme ingrédient actif, un composé selon la revendication 1, de formule (Ib) :

p-$R_1$-$C_6H_4$-$CH_2$-EDTA-M (Ib)

dans laquelle
$R_1$ représente

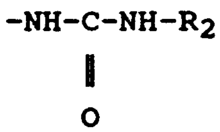, 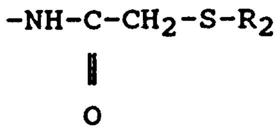, 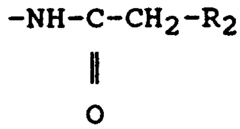,

-NH-$R_2$, 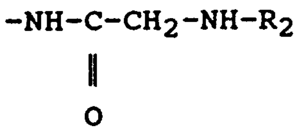 ou 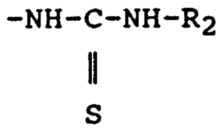;

$R_2$ représente un groupe p-phényle-$CH_2$-Y, -OC-(-Y), un groupe alkyle en $C_1$-$C_{30}$ à chaîne droite ou ramifiée, un groupe cycloalkyle, un groupe aryle ou un groupe arylalkyle substitués dans lesquels les substituants sont un ou plusieurs groupes de n'importe quels groupes ci-après :
un groupe hydroxyle,

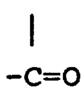, 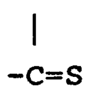,

-$SR_4$, -$NHR_3$, -$NHR_4$,
-$N(R_3)_2$, un groupe imidazolo
ou encore un groupe répondant aux formules :

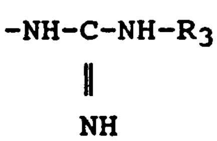, 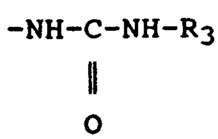 ou 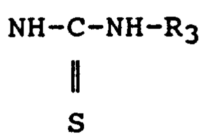;

dans lesquelles
$R_3$ représente un groupe p-phényle-$CH_2$-Y ou

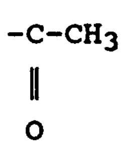;

Y représente EDTA, DTPA, DOTA, HETA, TRITA ou TETA;
$R_4$ représente H ou

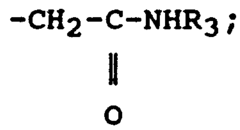

et

M représente un ion métallique,

ou bien un de ses sels pharmaceutiquement acceptables, en association avec un ou plusieurs supports, diluants ou excipients pharmaceutiquement acceptables pour le composé.

4. Formulation selon la revendication 2, dans laquelle $R_1$ de l'ingrédient actif représente $-NH(C=S)NH-R_2$ où $R_2$ est choisi parmi -H, $-CH_2CH_3$, $-(CH_2)_3CH_3$, $-(CH_2)_7CH_3$, $-CH_2CH_2OH$, $-CH_2CH_2NH_2$, $-C_6H_5$, $-CH_2C_6H_5$, $-4-(C_6H_4)CH_2CO_2H$, $-(CH_2)_2CO_2H$, $-(CH_2)_3CO_2H$, $-(CH_2)_4CH(NHCOCH_3)CONH_2$, $-(CH_2)_2NHCO(CH_2)_3CO_2H$, ou un de ses sels pharmaceutiquement acceptables.

5. Formulation selon la revendication 4, dans laquelle $R_2$ de l'ingrédient actif représente $-CH_2CH_2OH$.

6. Formulation selon la revendication 3, dans laquelle $R_1$ de l'ingrédient actif représente : $-NH(C=S)NH-R_2$ où $R_2$ représente
$-C_6H_4-CH_2-DTPA$,
$-CH_2CH_2-NH(C=S)NH-C_6H_4-CH_2DTPA$ ou
$-(CH_2)_4-NH(C=S)NH-C_6H_4-CH_2DTPA$.

7. Formulation selon la revendication 6, dans laquelle $R_2$ de l'ingrédient actif représente $-C_6H_4-CH_2-DTPA$.

8. Formulation selon la revendication 3, dans laquelle l'ingrédient actif est un composé de formule :

$$X-CH_2-CO-NH-C_6H_4-CH_2-EDTA-M,$$

dans laquelle
X représente
$-NH-C_6H_4-CH_2-DTPA$,
$-S(CH_2)_4SCH_2(C=O)NHC_6H_4CH_2DTPA$,
$-SCH_2CH(OH)CH(OH)CH_2SCH_2(C=O)NHC_6H_4CH_2DTPA$ ou
$-NHCH_2CH(OH)CH_2NHCH_2(C=O)NHC_6H_4DTPA$.

9. Formulation selon la revendication 8, dans laquelle X de l'ingrédient actif représente $-NH-C_6H_4-CH_2-DTPA$.

10. Formulation selon l'une quelconque des revendications 1 à 9, dans laquelle l'ion métallique est l'indium ou l'yttrium.

11. Formulation selon la revendication 10, dans laquelle l'ion métallique est $^{111}In$ ou $^{90}Y$.

12. Procédé pour préparer une formulation telle que revendiquée dans l'une quelconque des revendications 1 à 11, qui comprend le fait de mélanger un composé répondant aux formules (I), (Ia) ou (Ib) ou un de ses sels pharmaceutiquement acceptables, tel que défini dans l'une quelconque des revendications 1 à 11, avec un support, un diluant ou un excipient pharmaceutiquement acceptable pour le composé.

13. Nécessaire conçu pour l'administration, de manière simultanée, de manière séquentielle ou sous forme d'un complexe, d'un agent immunodiagnostique ou immunothérapeutique qui comprend un composé selon l'une quelconque des revendications 1 à 11 et un anticorps ou un fragment de ce dernier, ledit anticorps ou ledit fragment étant capable de complexer avec ledit composé.

14. Nécessaire selon la revendication 13, dans lequel l'anticorps est un anticorps monoclonal ou un fragment de ce dernier.

**15.** Nécessaire selon la revendication 13, dans lequel l'anticorps est un anticorps bifonctionnel ou un fragment de ce dernier.

**16.** Nécessaire selon la revendication 13, dans lequel l'anticorps est un anticorps chimère ou un fragment de ce dernier.

**17.** Nécessaire selon la revendication 16, dans lequel l'anticorps est un anticorps humanisé ou un fragment de ce dernier.

**18.** Nécessaire selon la revendication 16 ou 17, dans lequel l'anticorps est un anticorps chimère-bifonctionnel ou un fragment de ce dernier.

**19.** Nécessaire selon l'une quelconque des revendications 15 à 18, dans lequel l'anticorps est un anticorps synthétique croisé ou un fragment de ce dernier.

**20.** Procédé pour préparer un composé de formule (I) :

p-$R_1$-$C_6H_4$-$CH_2$-EDTA-M (I)

dans laquelle
$R_1$ représente

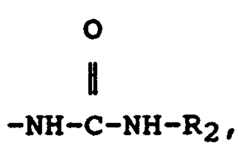, 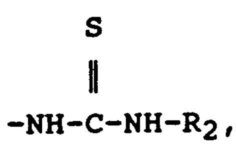, 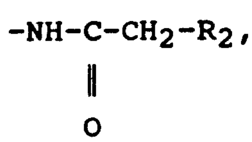, 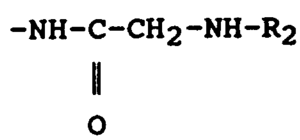,

ou 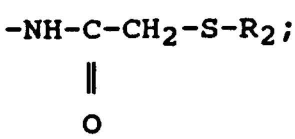;

$R_2$ représente un atome d'hydrogène, un groupe amino, un groupe p-phényle-$CH_2$-Y, un groupe alkyle en $C_1$-$C_{30}$ à chaîne droite ou ramifiée non substitué; un groupe alkyle à chaîne droite ou ramifiée en $C_1$-$C_{30}$, un groupe cycloalkyle, un groupe aryle ou un groupe arylalkyle substitués, dans lesquels les substituants sont un ou plusieurs groupes de n'importe quels groupes ci-après : un groupe hydroxyle, un groupe carboxyle, =O, =S,

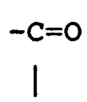, 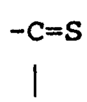,

-S-, -$SR_4$,
un atome de fluor, un atome de chlore, un atome de brome, un atome d'iode, un groupe amino, un groupe nitro, -$SO_3$H, -$NHR_3$, -$NHR_4$, -$N(R_3)_2$, -$CONHR_3$, -$COOR_3$, -$SO_4$, -$PO_4$, un groupe phényle, un groupe benzyle, un groupe imidazolo ou encore un groupe répondant aux formules :

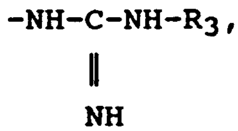 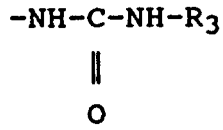 ou 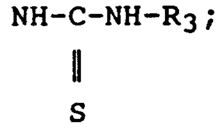

dans lesquelles
$R_3$ représente un atome d'hydrogène, un groupe alkyle en $C_1$-$C_{30}$ à chaîne droite ou ramifiée, un groupe p-phényle-$CH_2$-Y,

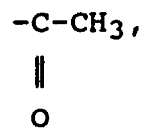

ou encore un groupe fonctionnel non réactif;
Y représente EDTA, DTPA, DOTA, HETA, TRITA ou TETA;
$R_4$ représente H ou

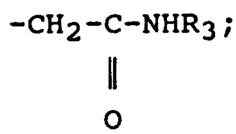

et
M représente un ion métallique,
ou bien un de ses sels pharmaceutiquement acceptables, qui comprend :

(A) dans le cas où $R_1$ représente -NH(C=S)NH-$R_2$ ou -NH(C=O)NH-$R_2$,

(1) la mise en réaction de l'isothiocyanatobenzyl-EDTA, l'isocyanatobenzyl-EDTA, selon le cas, ou encore un de ses chélates métalliques, avec un composé de formule $H_2N$-$R_2$, où $R_2$ est tel que décrit ci-dessus, ou bien

(2) la mise en réaction d'un composé de formule $R_2$-N=C=S, $R_2$-N=C=O, selon le cas, ou encore un de ses chélates métalliques, avec un composé de formule :

EDTA-$CH_2$-$C_6H_4$-$(NH(C=S)NH)_m$-$(X)_n$-$NH_2$ ou
EDTA-$CH_2$-$C_6H_4$-$(NH(C=O)NH)_m$-$(X)_nNH_2$

ou un de ses chélates métalliques, dans laquelle X représente un groupe alkyle en $C_1$-$C_{30}$ à chaîne droite ou ramifiée, un groupe aryle ou un groupe arylalkyle éventuellement substitués avec un ou plusieurs des substituants permis dans la définition de $R_2$ ci-dessus, et n et m, indépendamment l'un de l'autre, représentent 0 ou 1;

(B) si $R_1$ représente -NH(C=O)$CH_2$-S-$R_2$,
la mise en réaction du p-bromoacétamidobenzyl-EDTA ("BABE") ou un de ses chélates métalliques, avec un composé de formule HS-$R_2$ où $R_2$ est tel que défini ci-dessus;

(C) si $R_1$ représente -NH(C=O)$CH_2$-NH-$R_2$,
la mise en réaction du p-bromoacétamidobenzyl-EDTA ("BABE") avec un composé de formule $H_2N$-$R_2$ où $R_2$ est tel que défini ci-dessus;
ou bien si $R_1$ représente -NH(C=O)$CH_2$-NH-$R_2$ où un substituant de $R_2$ représente $NHR_4$, la mise en réaction du p-bromoacétamidobenzyl-Y où Y représente EDTA, DTPA, DOTA, HETA, TRITA ou TETA, ou encore un de ses chélates métalliques, avec un composé de formule

EDTA-$CH_2$-$C_6H_4$-NH(C=O)$CH_2$-NH-X-$NH_2$

ou un de ses chélates métalliques, dans laquelle X représente un groupe alkyle en $C_1$-$C_{30}$ à chaîne droite ou ramifiée, un groupe aryle ou un groupe arylalkyle éventuellement substitués avec un ou plusieurs des substituants permis dans la définition de $R_2$ ci-dessus; et

(D) si on le souhaite, salifier ou préparer le chélate métallique du produit obtenu.

**21.** Procédé selon la revendication 20, pour préparer un composé de formule (I) dans laquelle $R_1$ représente
-NH(C=S)NH-$R_2$ où $R_2$ est choisi parmi -H, $CH_2CH_3$, $-(CH_2)_3CH_3$, $-(CH_2)_7CH_3$, $-CH_2CH_2OH$, $-CH_2CH_2NH_2$, $-C_6H_5$, $-CH_2C_6H_5$, $-4-(C_6H_4)CH_2CO_2H$, $-(CH_2)_2CO_2H$, $-(CH_2)_3CO_2H$, $-(CH_2)_4CH$-($NHCOCH_3$)$CONH_2$, $-(CH_2)_2NHCO(CH_2)_3CO_2H$, ou un de ses sels pharmaceutiquement acceptables.

**22.** Procédé selon la revendication 21, pour préparer un composé dans lequel $R_2$ représente $-CH_2CH_2OH$.

**23.** Procédé selon la revendication 20, pour préparer un composé dans lequel $R_1$ représente : -NH(C=S)-NH-$R_2$ où $R_2$ représente
$-C_6H_4-CH_2$-DTPA,
- $CH_2CH_2$-NH(C=S)NH-$C_6H_4-CH_2$DTPA ou
$-(CH_2)_4$-NH(C=S)NH-$C_6H_4-CH_2$DTPA.

**24.** Procédé selon la revendication 23, pour préparer un composé dans lequel $R_2$ représente $-C_6H_4-CH_2$-DTPA.

**25.** Procédé selon la revendication 20, pour préparer un composé répondant à la formule :

X-$CH_2$-CO-NH-$C_6H_4-CH_2$-EDTA-M,

dans laquelle
X représente
-NH-$C_6H_4-CH_2$-DTPA,
-S$(CH_2)_4SCH_2$(C=O)NH$C_6H_4CH_2$DTPA,
-S$CH_2$CH(OH)CH(OH)$CH_2SCH_2$(C=O)NH$C_6H_4CH_2$DTPA ou
-NH$CH_2$CH(OH)$CH_2NHCH_2$(C=O)NH$C_6H_4CH_2$DTPA.

**26.** Procédé selon la revendication 25, pour préparer un composé dans lequel X représente -NH-$C_6H_4$-$CH_2$-DTPA.

**27.** Procédé selon l'une quelconque des revendications 20 à 25, dans lequel l'ion métallique est l'indium ou l'yttrium.

**28.** Procédé selon la revendication 27, dans lequel l'ion métallique est $^{111}In$ ou $^{90}Y$.

**29.** Procédé selon l'une quelconque des revendications 20 à 28, dans lequel le pH de la réaction se situe entre 6 et 12.

**30.** Procédé selon la revendication 29, dans lequel le pH de la réaction se situe entre 8 et 11.

**31.** Procédé selon la revendication 30, dans lequel la température réactionnelle se situe entre 0 °C et 60 °C.

**32.** Procédé selon la revendication 31, dans lequel la température réactionnelle se situe entre 20 °C et 40 °C.

FIG.1

PRE-MIX PROTOCOL

TUMOR UPTAKE

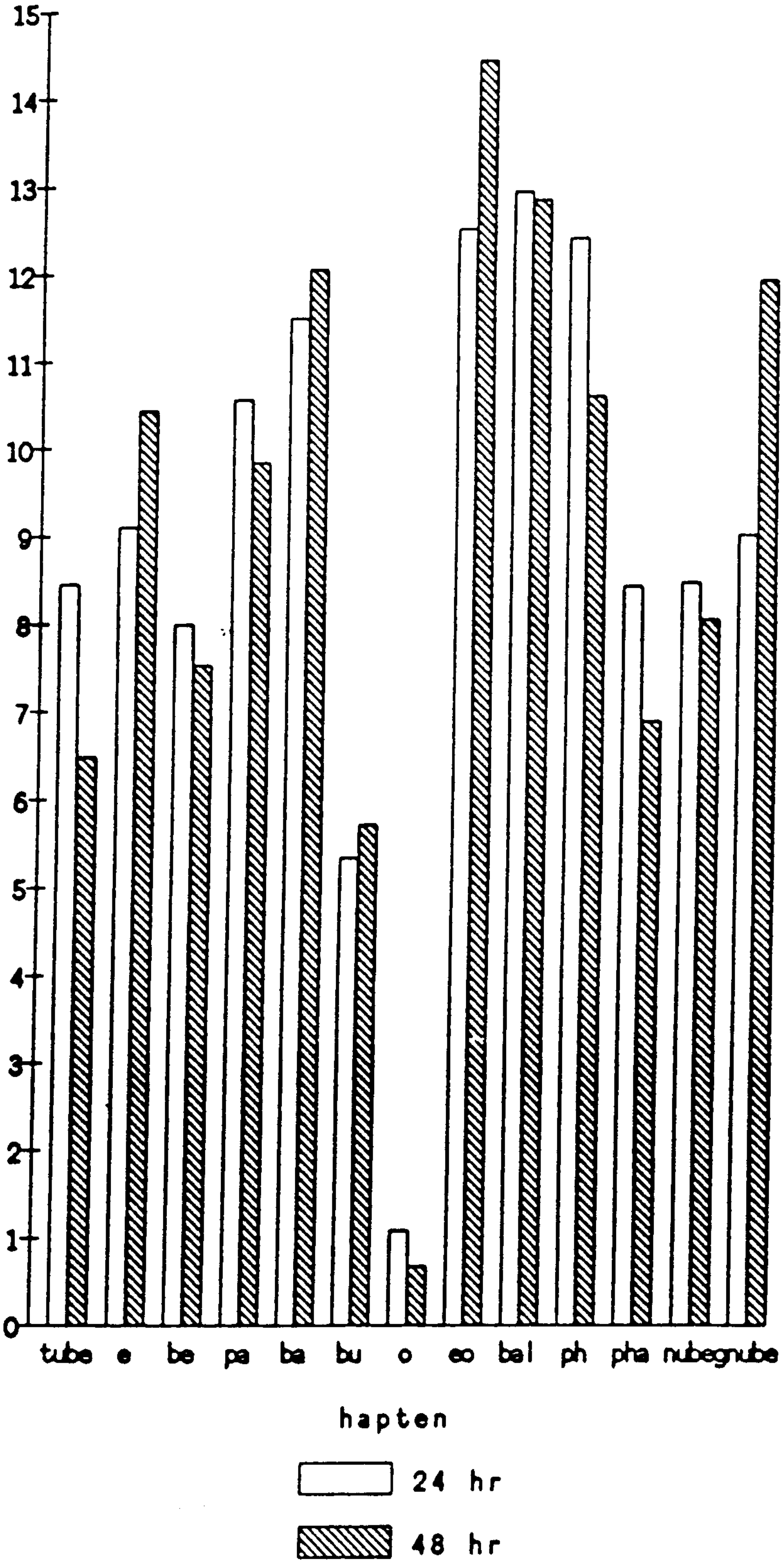

FIG.2

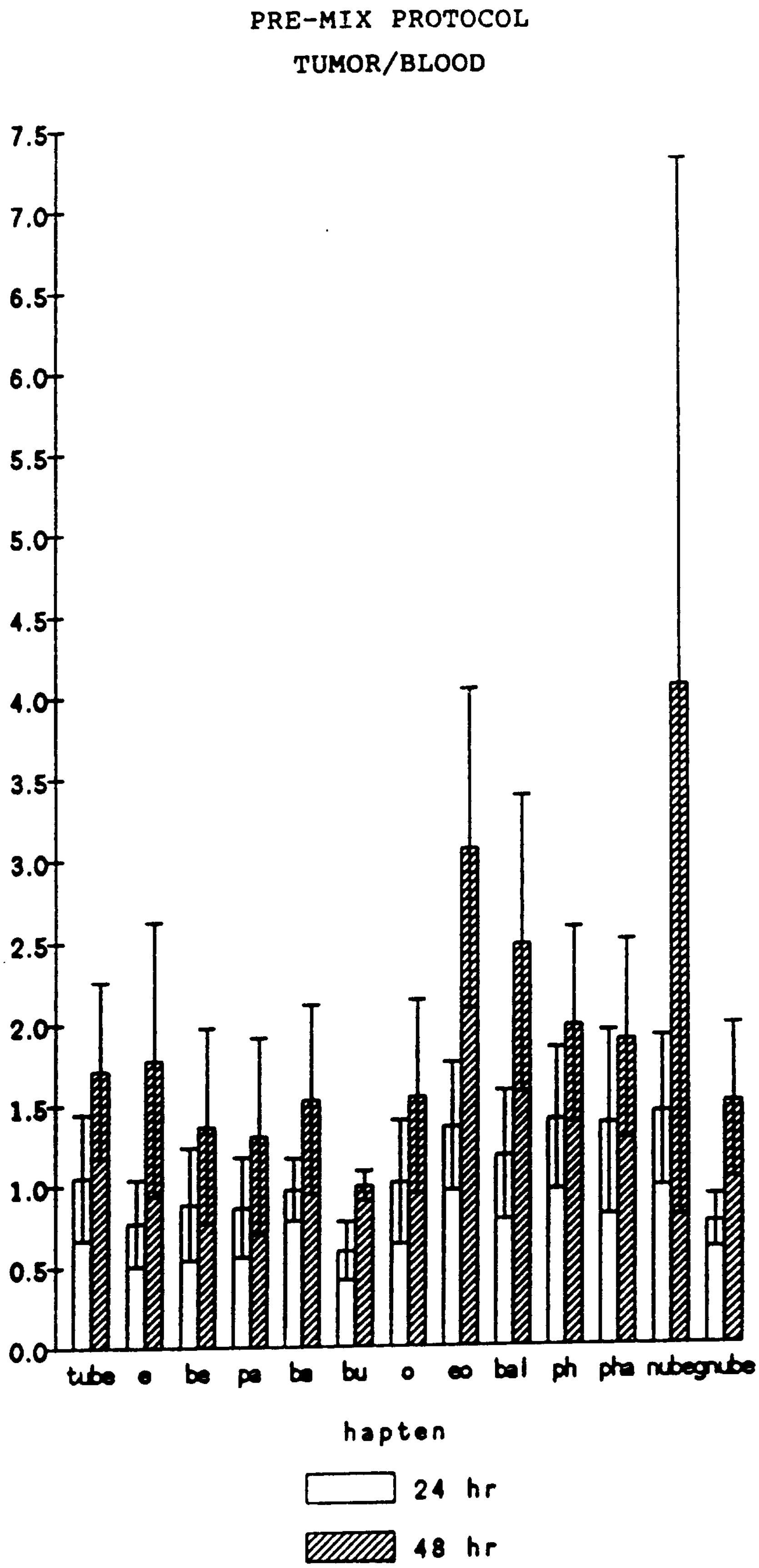
PRE-MIX PROTOCOL
TUMOR/BLOOD
7.5
7.0
6.5
6.0
5.5
5.0
4.5
4.0
3.5
3.0
2.5
2.0
1.5
1.0
0.5
0.0
tube
e
be
pa
ba
bu
o
eo
bal
ph
pha
nube
gnube
hapten
24 hr
48 hr

FIG.3

PRELOCALIZATION OF 5μg of ECH037.2

EOTUBE vs TUBE vs NUBE vs PHATUBE

4 HOUR TIME POINT

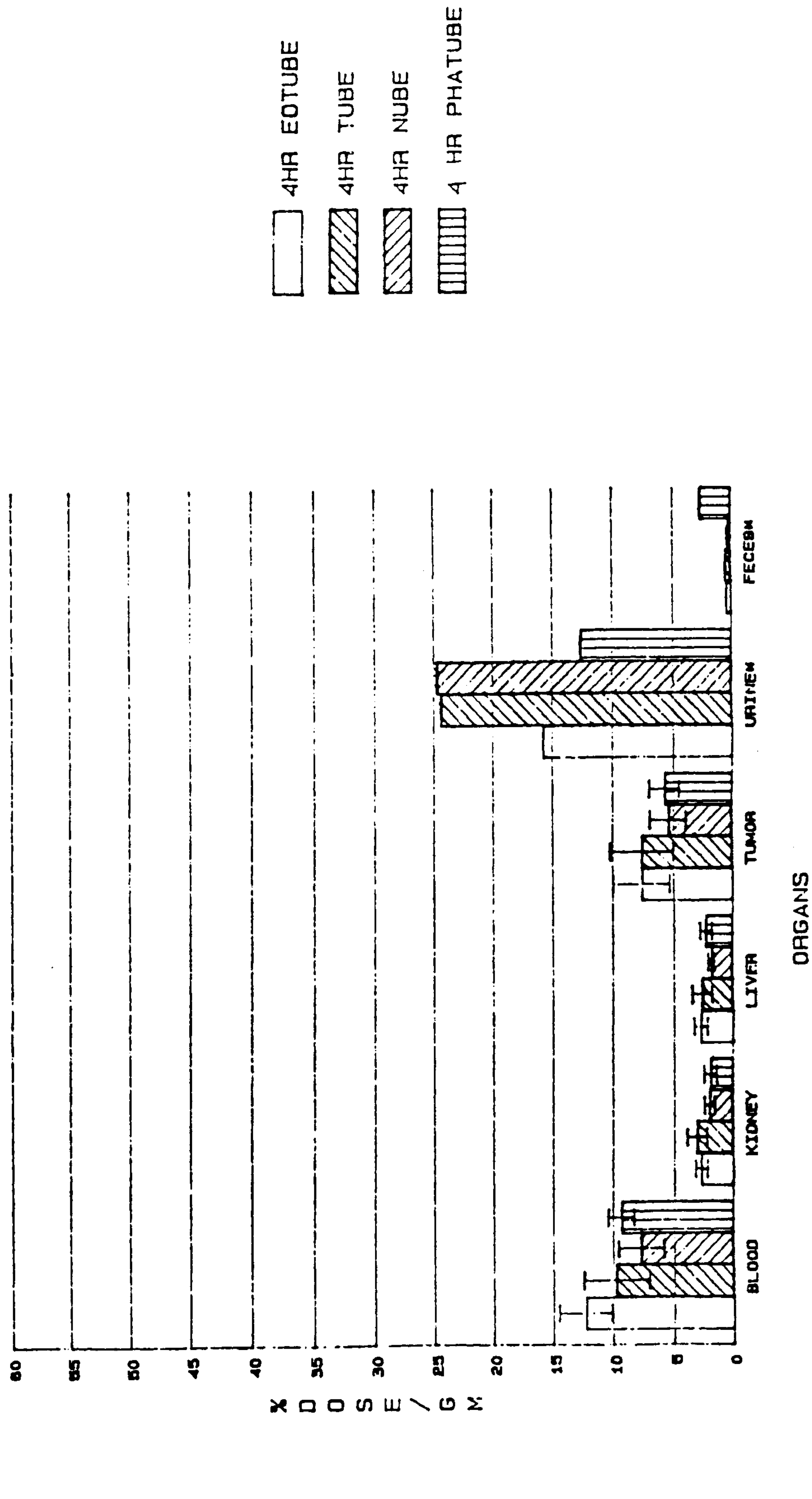

FIG. 4

PRELOCALIZATION OF 5μg ECH037.2
EOTUBE vs TUBE vs NUBE vs PHATUBE.
24HR TIME POINT.

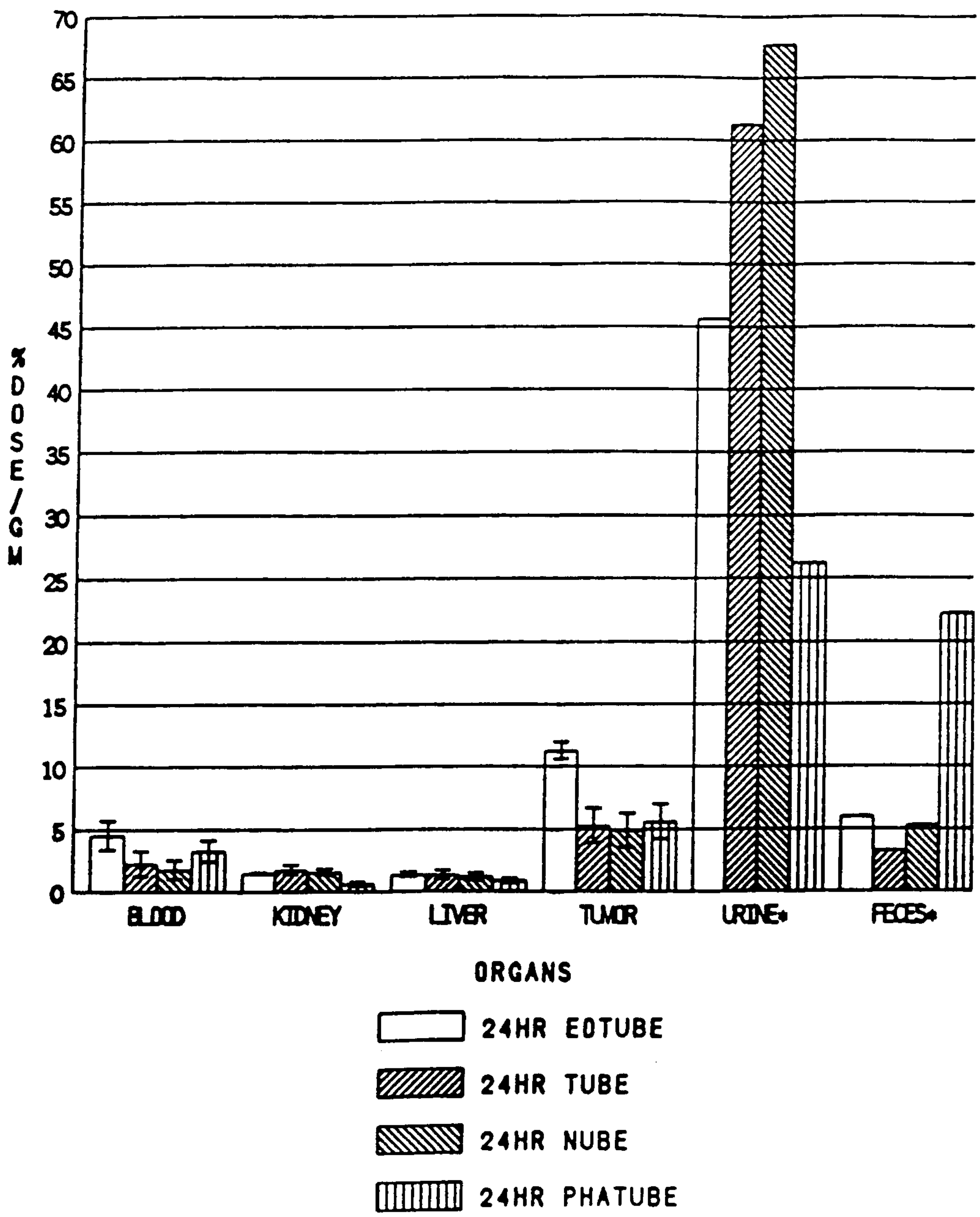

FIG.5

PRELOCALIZATION OF 5μg ECH037.2
EOTUBE vs TUBE vs NUBE vs PHATUBE.
48HR TIME POINT.

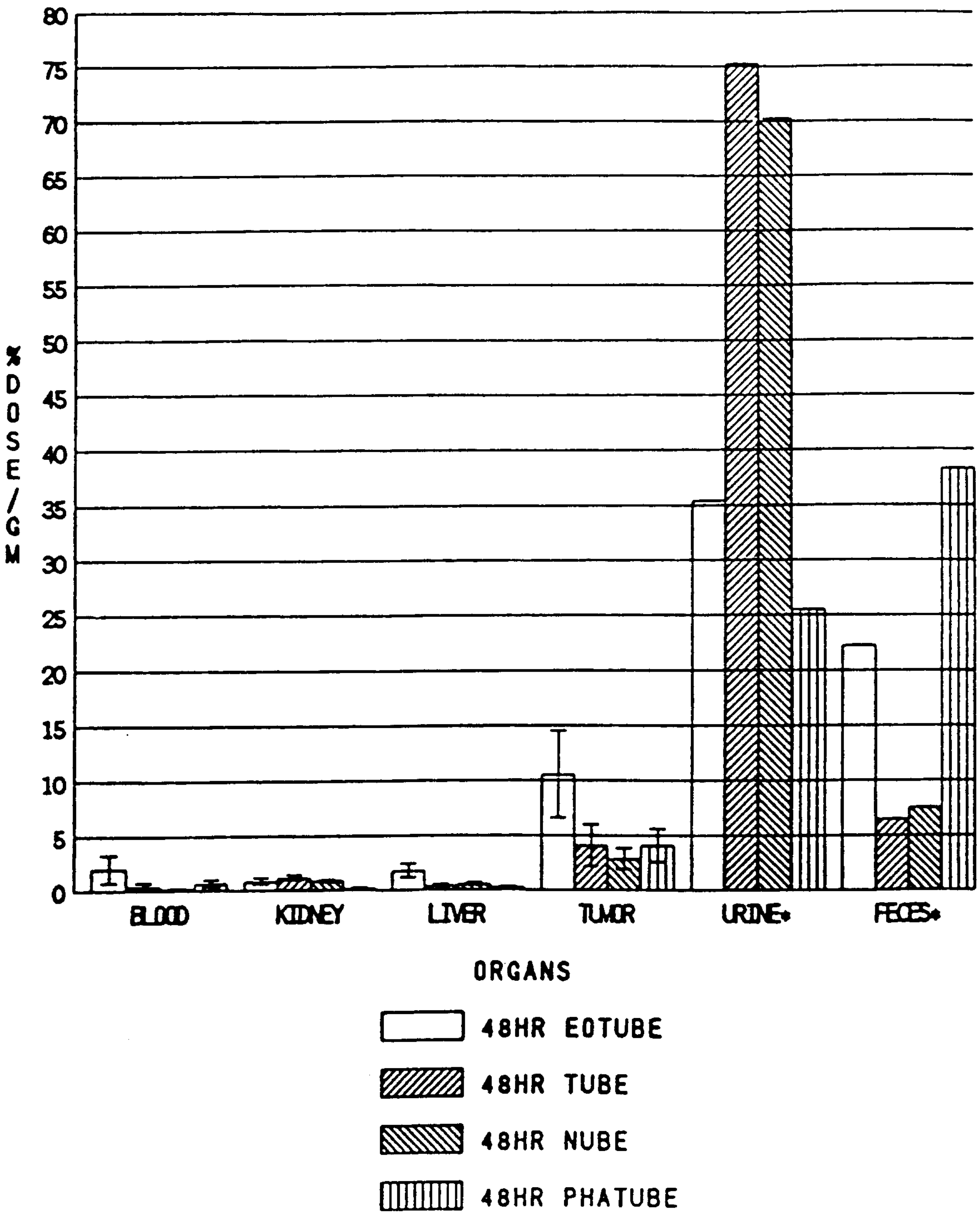

FIG. 6

PRELOCALIZATION OF 5μg ECH037.2
EOTUBE vs TUBE vs NUBE vs PHATUBE.
4HR TUMOR/ORGAN

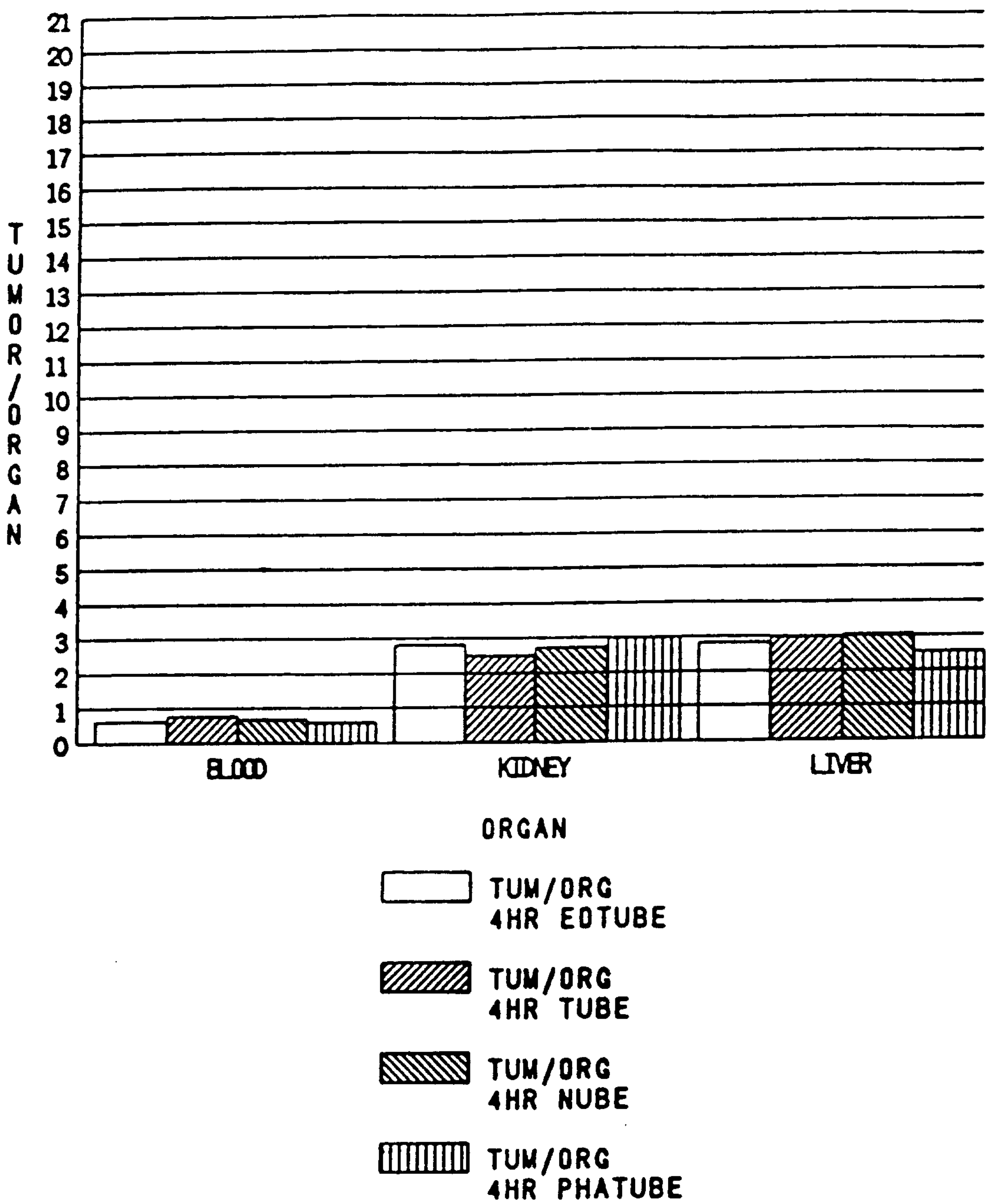

FIG.7

PRELOCALIZATION OF 5μg ECH037.2
EOTUBE vs TUBE vs NUBE vs PHATUBE.
24HR TUMOR/ORGAN.

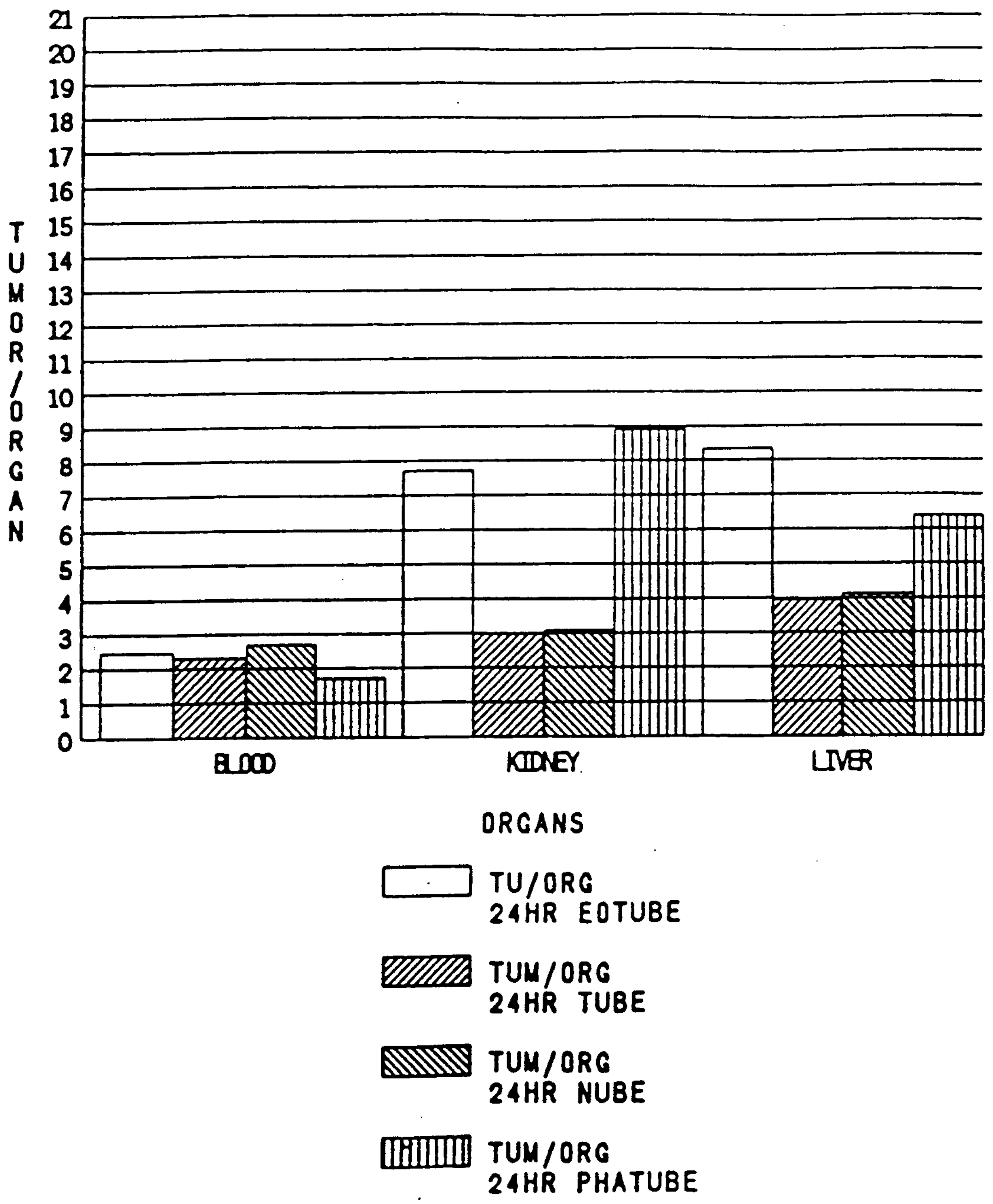

FIG.8

PRELOCALIZATION OF 5µg ECH037.2
EOTUBE vs TUBE vs NUBE vs PHATUBE.
48HR TUMOR/ORGAN